# EUROPÄISCHE PATENTSCHRIFT

(11) **EP 1 017 692 B1**
(45) Veröffentlichungstag und Bekanntmachung des Hinweises auf die Patenterteilung: **03.03.2004**
(21) Anmeldenummer: 98948919.0
(22) Anmeldetag: 07.09.1998
(51) Int. Cl.: C07D 409/04, A61K 31/47, C07D 401/04, C07D 405/04, C07D 215/20, C07F 7/18

(54) **4-HETEROARYL-TETRAHYDROCHINOLINE UND IHRE VERWENDUNG ALS INHIBITOREN DES CHOLESTERIN-ESTER-TRANSFER-PROTEINS (CTEP)**
4-HETEROARYL-TETRAHYDROQUINOLINES AND THEIR USE AS INHIBITORS OF THE CHOLESTERIN-ESTER TRANSFER PROTEIN
4-HETEROARYLE-TETRAHYDROQUINOLEINES ET LEUR UTILISATION COMME INHIBITEURS DE LA PROTEINE DE TRANSFERT CHOLESTERINE-ESTER

(30) Priorität: 18.09.1997 DE 19741051
(43) Veröffentlichungstag der Anmeldung: 12.07.2000
(73) Patentinhaber: Bayer HealthCare AG, 51368 Leverkusen (DE)
(72) Erfinder: STOLTEFUSS, Jürgen, D-42781 Haan (DE); LÖGERS, Michael, D-42327 Wuppertal (DE); SCHMIDT, Gunter, D-42115 Wuppertal (DE); BRANDES, Arndt, D-42115 Wuppertal (DE); SCHMECK, Carsten, D-42119 Wuppertal (DE); BREMM, Klaus-Dieter, D-45661 Recklinghausen (DE); BISCHOFF, Hilmar, D-42113 Wuppertal (DE); SCHMIDT, Delf, D-42113 Wuppertal (DE)
(86) Internationale Anmeldenummer: PCT/EP1998/005656
(87) Internationale Veröffentlichungsnummer: WO 1999/014215

(56) Entgegenhaltungen:
- EP-A- 0 304 063
- EP-A- 0 325 130
- EP-A- 0 818 197
- WO-A-98/39299

## Beschreibung

Die vorliegende Erfindung betrifft Hetero-Tetrahydrochinoline, Verfahren zu ihrer Herstellung und ihre Verwendung in Arzneimitteln.

Aus der Publikation US-5 169 857-A2 sind 7-(polysubstituierte Pyridyl)-6-heptenoate zur Behandlung der Arteriosklerose, Lipoproteinaemia und Hyperproteinaemia bekannt. Außerdem wird die Herstellung von 7-(4-Aryl-3-pyridyl)-3,5-dihydroxy-6-heptenoate in der Publikation EP-325 130-A2 beschrieben. Ferner ist die Verbindung 5(6H)-Chinolone,3-benzyl-7,8-dihydro-2,7,7-trimethyl-4-phenyl, aus der Publikation Khim. Geterotsikl. Soedin. (1967), (6), 1118-1120 bekannt.

Die vorliegende Erfindung betrifft Hetero-Tetrahydrochinoline der allgemeinen Formel (I), 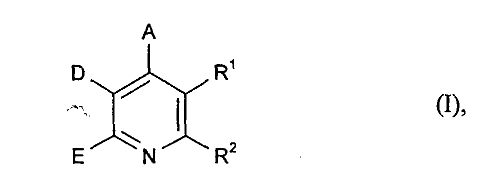 in welcher
- A: für Cycloalkyl mit 3 bis 8 Kohlenstoffatomen oder
für einen 5- bis 7-gliedrigen, gesättigten, partiell ungesättigten oder ungesättigten, gegebenenfalls benzokondensierten Heterocyclus mit bis zu 3 Heteroatomen aus der Reihe S, N und/oder O steht, der im Fall eines gesättigten Heterocyclus mit einer Stickstoffunktion, gegebenenfalls auch über diese gebunden ist, und wobei die oben aufgeführten Ringsysteme gegebenenfalls bis zu 5-fach gleich oder verschieden durch Halogen, Nitro, Hydroxy, Trifluormethyl, Trifluormethoxy oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert sind,
worin
R³ und R⁴ gleich oder verschieden sind und Wasserstoff, Phenyl oder geradkettiges oder verzweigtes Alkyl mit bis zu 6 Kohlenstoffatomen bedeuten,
oder
- A: x für einen Rest der Formel 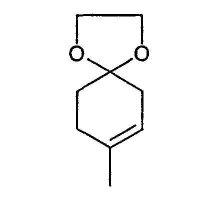 steht,
- D: für Aryl mit 6 bis 10 Kohlenstoffatomen steht, das gegebenenfalls durch Phenyl, Nitro, Halogen, Trifluormethyl oder Trifluormethoxy substituiert ist, oder
für einen Rest der Formel

R⁵―L―

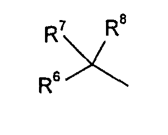

oder

R⁹―T―V―X―

steht,
worin
R⁵, R⁶ und R⁹ unabhängig voneinander Cycloalkyl mit 3 bis 6 Kohlenstoffatomen bedeuten, oder Aryl mit 6 bis 10 Kohlenstoffatomen bedeuten oder einen 5- bis 7-gliedrigen, gegebenenfalls benzokondensierten, gesättigten oder ungesättigten, mono-, bi- oder tricyclischen Heterocyclus mit bis zu 4 Heteroatomen aus der Reihe S, N und/oder O bedeuten,
wobei die Cyclen, gegebenenfalls, im Fall der stickstoffhaltigen Ringe auch über die N-Funktion, bis zu 5-fach gleich oder verschieden durch Halogen, Trifluormethyl, Nitro, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 6 Kohlenstoffatomen, durch Aryl oder Trifluormethyl substituiertes Aryl mit jeweils 6 bis 10 Kohlenstoffatomen oder durch einen, gegebenenfalls benzokondensierten, aromatischen 5- bis 7-gliedrigen Heterocyclus mit bis zu 3 Heteroatomen aus der Reihe S, N und/oder O substituiert sind,
und/oder durch eine Gruppe der Formel -OR¹⁰, -SR¹¹, -SO₂R¹² oder - NR¹³R¹⁴ substituiert sind,
worin
R¹⁰, R¹¹ und R¹² unabhängig voneinander Aryl mit 6 bis 10 Kohlenstoffatomen bedeuten, das seinerseits bis zu 2-fach gleich oder verschieden durch Phenyl, Halogen oder durch geradkettiges oder verzweigtes Alkyl mit bis zu 6 Kohlenstoffatomen substituiert ist,
R¹³ und R¹⁴ gleich oder verschieden sind und die oben angegebene Bedeutung von R³ und R⁴ haben, oder
R⁵ und/oder R⁶ einen Rest der Formel 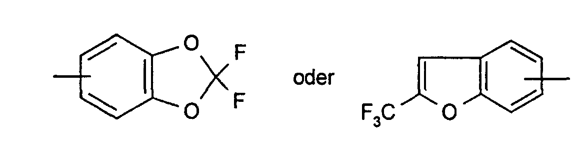 bedeuten,
R⁷ Wasserstoff oder Halogen bedeutet, und
R⁸ Wasserstoff, Halogen, Azido, Trifluormethyl, Hydroxy, Trifluormethoxy, geradkettiges oder verzweigtes Alkoxy oder Alkyl mit jeweils bis zu 6 Kohlenstoffatomen oder einen Rest der Formel -NR¹⁵R¹⁶ bedeutet,
worin
R¹⁵ und R¹⁶ gleich oder verschieden sind und die oben angegebene Bedeutung von R³ und R⁴ haben, oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 6 Kohlenstoffatomen bedeutet,
L eine geradkettige oder verzweigte Alkylen- oder Alkenylen-Kette mit jeweils bis zu 8 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 8 Kohlenstoffatomen bedeuten, oder
T oder X eine Bindung bedeutet,
V für ein Sauerstoff- oder Schwefelatom oder für eine -NR¹⁸-Gruppe steht,
worin
R¹⁸ Wasserstoff oder geradkettiges oder verzweigtes Alkyl mit bis zu 6 Kohlenstoffatomen oder Phenyl bedeutet,
- E: x für Cycloalkyl mit 3 bis 8 Kohlenstoffatomen steht, oder
für geradkettiges oder verzweigtes Alkyl mit bis zu 8 Kohlenstoffatomen steht, das gegebenenfalls durch Cycloalkyl mit 3 bis 8 Kohlenstoffatomen oder Hydroxy substituiert ist, oder für Phenyl steht, das gegebenenfalls durch Halogen oder Trifluormethyl substituiert ist,
- R¹ und R²: gemeinsam eine geradkettige oder verzweigte Alkylenkette mit bis zu 7 Kohlenstoffatomen bilden, die durch eine Carbonylgruppe und/oder durch einen Rest der Formel 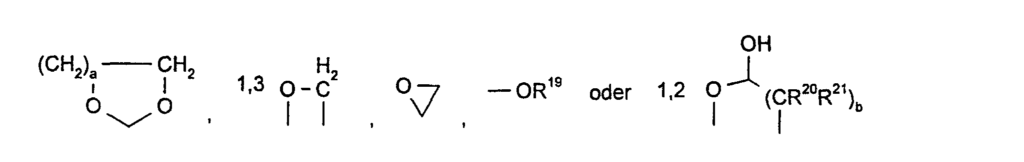 substituiert sein muß,
worin
a und b gleich oder verschieden sind und eine Zahl 1, 2 oder 3 bedeuten,
R¹⁹ Wasserstoff, Cycloalkyl mit 3 bis 7 Kohlenstoffatomen, geradkettiges oder verzweigtes Silylalkyl mit bis zu 8 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes alkoxy mit bis zu 6 Kohlenstoffatomen oder durch Phenyl substituiert ist, das seinerseits durch Halogen, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 4 Kohlenstoffatomen oder Benzyl bedeutet, oder
R¹⁹ geradkettiges oder verzweigtes Acyl mit bis zu 20 Kohlenstoffatomen oder Benzoyl bedeutet, das gegebenenfalls durch Halogen, Trifluormethyl, Nitro oder Trifluormethoxy substituiert ist, oder
geradkettiges oder verzweigtes Fluoracyl mit bis zu 8 Kohlenstoffatomen und 9 Fluoratomen bedeutet,
R²⁰ und R²¹ gleich oder verschieden sind, Wasserstoff, Phenyl oder geradkettiges oder verzweigtes Alkyl mit bis zu 6 Kohlenstoffatomen bedeuten, oder
R²⁰ und R²¹ gemeinsam einen 3 bis 6-gliedrigen Carbocyclus bilden,
und, gegebenenfalls auch geminal, die gebildeten Carbocyclen gegebenenfalls bis zu 6-fach gleich oder verschieden durch Trifluormethyl, Hydroxy, Nitril, Halogen, Carboxyl, Nitro, Azido, Cyano, Cycloalkyl oder Cycloalkyloxy mit jeweils 3 bis 7 Kohlenstoffatomen, durch geradkettiges oder verzweigtes Alkoxycarbonyl, Alkoxy oder Alkylthio mit jeweils bis zu 6 Kohlenstoffatomen oder durch geradkettiges oder verzweigtes Alkyl mit bis zu 6 Kohlenstoffatomen substituiert sind, das seinerseits bis zu 2-fach gleich oder verschieden durch Hydroxyl, Benzyloxy, Trifluormethyl, Benzoyl, geradkettiges oder verzweigtes Alkoxy, Oxyacyl oder Carboxyl mit jeweils bis zu 4 Kohlenstoffatomen und/oder Phenyl substituiert ist, das seinerseits durch Halogen, Trifluormethyl oder Trifluormethoxy substituiert sein kann,
und/oder die gebildeten Carbocyclen, auch geminal, gegebenenfalls bis zu 5-fach gleich oder verschieden durch Phenyl, Benzoyl, Thiophenyl oder Sulfonylbenzyl substituiert sind, die ihrerseits gegebenenfalls durch Halogen, Trifluormethyl, Trifluormethoxy oder Nitro substituiert sind,
und/oder gegebenenfalls durch einen Rest der Formel 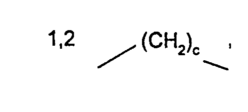 -SO₂-C₆H₅, -(CO)_{d}-NR²³R²⁴ oder =O substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cycloalkyl mit 3 bis 6 Kohlenstoffatomen, geradkettiges oder verzweigtes Alkyl mit bis zu 6 Kohlenstoffatomen, Benzyl oder Phenyl bedeuten, das gegebenenfalls bis zu 2-fach gleich oder verschieden durch Halogen, Trifluormethyl, Cyano, Phenyl oder Nitro substituiert ist,
und/oder die gebildeten Carbocyclen gegebenenfalls durch einen spiro-verknüpften Rest der Formel 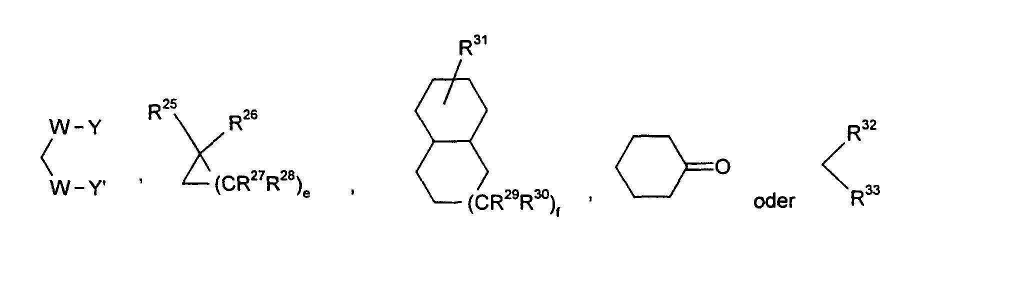 substituiert sind,
worin
W entweder ein Sauerstoff oder ein Schwefelatom bedeutet,
Y und Y' gemeinsam eine 2- bis 6-gliedrige geradkettige oder verzweigte Alkylenkette bilden,
e eine Zahl 1, 2, 3, 4, 5, 6 oder 7 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Halogen oder geradkettiges oder verzweigtes Alkyl oder Alkoxy mit jeweils bis zu 6 Kohlenstoffatomen bedeuten, oder
R²⁵ und R²⁶ oder R²⁷ und R²⁸ jeweils gemeinsam eine geradkettige oder verzweigte Alkylkette mit bis zu 6 Kohlenstoffatomen bilden, oder
R²⁵ und R²⁶ oder R²⁷ und R²⁸ jeweils gemeinsam einen Rest der Formel 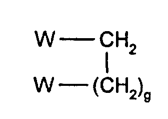 bilden,
worin
W die oben angegebene Bedeutung hat,
g eine Zahl 1, 2, 3, 4, 5, 6 oder 7 bedeutet,
R³² und R³³ gemeinsam einen 3- bis 7-gliedrigen Heterocyclus bilden, der ein Sauerstoff- oder Schwefelatom oder eine Gruppe der Formel SO, SO₂ oder -NR³⁴ enthält,
worin
R³⁴ Wasserstoff, Phenyl, Benzyl oder geradkettiges oder verzweigtes Alkyl mit bis zu 4 Kohlenstoffatomen bedeutet,
und deren Salze und N-Oxide.

Die erfindungsgemäßen Hetero-Tetrahydrochinoline können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren sowie deren jeweiligen Mischungen. Diese Mischungen der Enantiomeren und Diastereomeren lassen sich in bekannter Weise in die stereoisomer einheitlichen Bestandteile trennen.

Ein 3- bis 8-gliedriger gesättigter carbocyclischer Ring steht im Rahmen der Erfindung für einen Cyclopropyl-, Cyclobutyl-, Cyclopentyl-, Cyclohexyl-, Cycloheptyl- oder Cyclooctylring. Bevorzugt sind ein Cyclopropyl-, Cyclobutyl-, Cyclopentyl- oder Cyclohexylring. Besonders bevorzugt sind Cyclobutyl, Cyclopentyl oder Cyclohexyl.

Heterocyclus steht im Rahmen der Erfindung im allgemeinen für einen gesättigten, partiell ungesättigten oder ungesättigten, gegebenenfalls benzokondensierten 5- bis 7-gliedrigen, vorzugsweise 5- bis 6-gliedrigen Heterocyclus, der bis zu 3 Heteroatome aus der Reihe S, N und/oder O enthalten kann. Beispielsweise seien genannt:. Indolyl, Isochinolyl, Chinolyl, Benzo[b]thiophen, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind die erfindungsgemäßen Verbindungen der allgemeinen Formel (I),
in welcher
- A: für Cyclopropyl, Cyclobutyl, Cyclopentyl, Cycloheptyl, Cyclooctyl oder Cyclohexyl steht, oder
für Thienyl, Imidazolyl, Pyrrol, Furryl, Pyridyl, Morpholin, Pyrimidyl oder Pyridazinyl steht, , die gegebenenfalls bis zu 2-fach gleich oder verschieden durch Fluor, Chlor, Brom, Amino, Hydroxy, Trifluormethyl, Trifluormethoxy oder durch geradkettiges oder verzweigtes Alkyl, oder Alkoxy mit jeweils bis zu 6 Kohlenstoffatomen substituiert sind,
oder
- A: für einen Rest der Formel 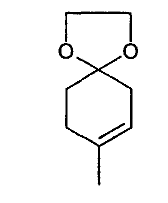 steht,
- D: für Phenyl steht, das gegebenenfalls durch Nitro, Fluor, Chlor, Brom, Phenyl, Trifluormethyl oder Trifluormethoxy substituiert ist, oder
für einen Rest der Formel

R⁵―L― ,

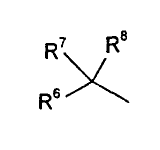

oder

R⁹-T-V―X―

steht,
worin
R⁵, R⁶ und R⁹ unabhängig voneinander Cyclopropyl, Cyclopentyl oder Cyclohexyl bedeuten, oder
Phenyl, Naphthyl, Pyridyl, Tetrazolyl, Pyrimidyl, Pyrazinyl, Pyrrolidinyl, Indolyl, Morpholinyl, Imidazolyl, Benzothiazolyl, Phenoxathiin-2-yl, Benzoxazolyl, Furyl, Chinolyl oder Purin-8-yl bedeuten,
wobei die Cyclen, gegebenenfalls bis zu 3-fach im Fall der stickstoffhaltigen Ringe auch über die N-Funktion, gleich oder verschieden durch Fluor, Chlor, Brom, Trifluormethyl, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 4 Kohlenstoffatomen, Triazolyl, Tetrazolyl, Benzoxathiazolyl, oder Trifluormethyl substituiertes Phenyl oder Phenyl substituiert sind, oder
R⁷ Wasserstoff, Fluor, Chlor oder Brom bedeutet, und
R⁸ Wasserstoff, Fluor, Chlor, Brom, Azido, Trifluormethyl, Hydroxy, Trifluormethoxy, geradkettiges oder verzweigtes Alkoxy oder Alkyl mit bis zu jeweils 5 Kohlenstoffatomen oder einen Rest der Formel - NR¹⁵R¹⁶ bedeutet,
worin
R¹⁵ und R¹⁶ gleich oder verschieden sind und Wasserstoff, Phenyl oder geradkettiges oder verzweigtes Alkyl mit bis zu 4 Kohlenstoffatomen bedeuten, oder
R⁷ und R⁸ gemeinsam einen Rest der Formel =O oder =NR¹⁷ bilden, worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 6 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 6 Kohlenstoffatomen bedeuten, oder
T oder X eine Bindung bedeutet,
V für ein Sauerstoff- oder Schwefelatom oder für eine Gruppe der Formel - NR¹⁸- steht,
worin
R¹⁸ Wasserstoff oder geradkettiges oder verzweigtes Alkyl mit bis zu 4 Kohlenstoffatomen oder Phenyl bedeutet,
- E: für Cyclopropyl, -butyl, -pentyl, -hexyl oder -heptyl steht, oder
für geradkettiges oder verzweigtes Alkyl mit bis zu 6 Kohlenstoffatomen steht, das gegebenenfalls durch Cyclopropyl, -butyl, -hexyl, -pentyl, -heptyl oder durch Hydroxy substituiert ist, oder für Phenyl steht, das gegebenenfalls durch Fluor, Chlor oder Trifluormethyl substituiert ist,
- R¹ und R²: gemeinsam eine geradkettige oder verzweigte Alkylenkette mit bis zu 6 Kohlenstoffatomen bilden, die durch eine Carboxylgruppe und/oder durch einen Rest der Formel 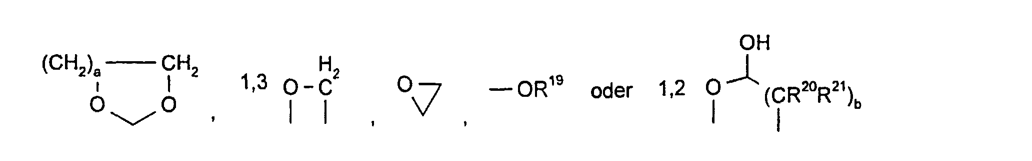 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 7 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy mit bis zu 4 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet, oder
R¹⁹ geradkettiges oder verzweigtes Acyl mit bis zu 18 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 6 Kohlenstoffatomen bedeutet,
R²⁰ und R²¹ gleich oder verschieden sind, Wasserstoff, Phenyl oder geradkettiges oder verzweigtes Alkyl mit bis zu 4 Kohlenstoffatomen bedeuten, oder
R²⁰ und R²¹ gemeinsam einen Cyclpropyl-, Cyclobutyl-, Cyclopentyl-, Cyclohexyl- oder Cycloheptylring bilden,
und die gebildeten Carbocyclen gegebenenfalls bis zu 5-fach gleich oder verschieden, gegebenenfalls auch geminal, durch Trifluormethyl, Hydroxy, Carboxyl, Azido, Fluor, Chlor, Brom, Nitro, Cyano,
Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis ca. 5 Kohlenstoffatomen oder geradkettiges oder verzweigtes Alkyl mit bis zu 5 Kohlenstoffatomen substituiert sind, das seinerseits bis zu 2-fach gleich oder verschieden durch Hydroxyl, Benzyloxy, Benzoyl, geradkettiges oder verzweigtes Alkoxy oder Oxyacyl mit jeweils bis zu 3 Kohlenstoffatomen, Trifluormethyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden durch Phenyl, Benzoyl, Thiophenyl oder Sulfonylbenzyl substituiert sind, die ihrerseits gegebenenfalls durch Fluor, Chlor, Brom, Trifluormethyl, Trifluormethoxy oder Nitro substituiert sind,
und/oder gegebenenfalls durch einen Rest der Formel 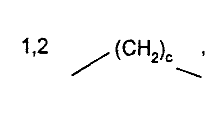 -SO₂-C₆H₅, -(CO)_{d}-NR²³R²⁴ oder =O substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclobutyl, Cyclopentyl, Cyclohexyl, geradkettiges oder verzweigtes Alkyl mit bis zu 5 Kohlenstoffatomen, Benzyl oder Phenyl bedeuten, das gegebenenfalls durch Fluor, Chlor, Brom, Phenyl oder Trifluormethyl substituiert ist, und/oder die gebildeten Carbocyclen gegebenenfalls durch einen spiro-verknüpften Rest der Formel 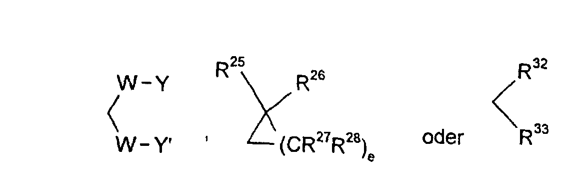 substituiert sind,
worin
W entweder ein Sauerstoff oder ein Schwefelatom bedeutet,
Y und Y' gemeinsam eine 2- bis 5-gliedrige geradkettige oder verzweigte Alkylkette bilden,
e eine Zahl 1, 2, 3, 4, 5 oder 6 bedeutet,
f eine Zahl 1 oder 2 bedeutet,
R²⁵, R²⁶, R²⁷ und R²⁸ gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten, oder
R²⁵ und R²⁶ oder R²⁷ und R²⁸ jeweils gemeinsam eine geradkettige oder verzweigte Alkylkette mit bis zu 5 Kohlenstoffatomen bilden oder oder
R²⁵ und R²⁶ oder R²⁷ und R²⁸ jeweils gemeinsam einen Rest der Formel 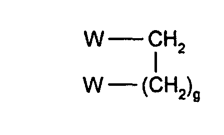 bilden,
worin
W die oben angegebene Bedeutung hat,
g eine Zahl 1, 2, 3, 4, 5 oder 6 bedeutet,
und deren Salze und N-Oxide.

Besonders bevorzugt sind erfindungsgemäße Verbindungen der allgemeinen Formel (I).
in welcher
- A: für Cyclopropyl, Cyclopentyl oder Cyclohexyl steht, oder
für Thienyl oder Pyridyl steht, die gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert sind,
oder
- A: für einen Rest der Formel 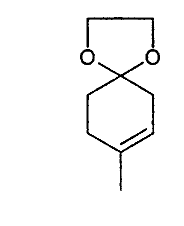 steht,
- D: für Phenyl steht, das gegebenenfalls durch Nitro, Trifluormethyl, Phenyl, Fluor, Chlor oder Brom substituiert ist, oder
für einen Rest der Formel

R⁵―L― ,

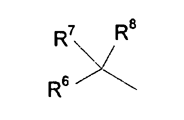

oder

R⁹-T-V―X―

steht,
worin
R⁵, R⁶ und R⁹ unabhängig voneinander Cyclopropyl, Cyclopentyl oder Cyclohexyl bedeuten, oder
Phenyl, Naphthyl oder Pyridyl bedeuten,
wobei die Cyclen, gegebenenfalls bis zu 2-fach, gleich oder verschieden durch Fluor, Chlor, Trifluormethyl, Hydroxy, Cyano, Carboxyl, Trifluormethoxy, geradkettiges oder verzweigtes Alkyl, Alkylthio, Alkylalkoxy, Alkoxy oder Alkoxycarbonyl mit jeweils bis zu 4 Kohlenstoffatomen, oder
R⁷ Wasserstoff oder Fluor bedeutet, und
R⁸ Wasserstoff, Fluor, Chlor, Brom, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten, oder
R⁷ und R⁸ gemeinsam für einen Rest der Formel =O stehen,
L eine geradkettige oder verzweigte Alkylen- oder Alkenylen-Kette mit jeweils bis zu 5 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 3 Kohlenstoffatomen bedeuten, oder
T oder X eine Bindung bedeutet,
V für ein Sauerstoff- oder Schwefelatom oder für eine Gruppe der Formel - NR¹⁸ steht,
worin
R¹⁸ Wasserstoff oder geradkettiges oder verzweigtes Alkyl mit bis zu 3 Kohlenstoffatomen bedeutet,
- E: für Cyclopropyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
- R¹ und R²: gemeinsam eine geradkettige oder verzweigte Alkylenkette mit bis zu 5 Kohlenstoffatomen bilden, die durch eine Carbonylgruppe und/oder einen Rest der Formel -OR¹⁹ substituiert sein muß,
worin
R¹⁹ Wasserstoff, Cyclopropyl, Cyclopentyl oder Cyclohexyl bedeutet, oder
R¹⁹ geradkettiges oder verzweigtes Acyl mit bis zu 15 Kohlenstoffatomen oder Benzoyl bedeutet, das gegebenenfalls durch Fluor, Chlor, Brom, Trifluormethyl, Nitro oder Trifluormethoxy substituiert ist, oder
einen Rest der Formel -Si(CH₃)₂C(CH₃)₃ bedeutet,
und die gebildeten Carbocyclen gegebenenfalls bis zu 4-fach gleich oder verschieden, gegebenenfalls auch geminal, durch Fluor, Hydroxyl, Trifluormethyl, Carboxyl, Azido, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis zu 4 Kohlenstoffatomen oder geradkettiges oder verzweigtes Alkyl mit bis zu 4 Kohlenstoffatomen substituiert sind, das seinerseits bis zu 2-fach gleich oder verschieden durch Hydroxyl, Benzyloxy, Trifluormethyl, Benzoyl, Methoxy, Oxyacetyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden, durch Phenyl, Benzoyl, Thiophenyl oder Sulfonylbenzyl substituiert sind, die ihrerseits gegebenenfalls durch Fluor, Trifluormethyl, Trifluormethoxy oder Nitro substituiert sind,
und/oder gegebenenfalls durch einen Rest der Formel 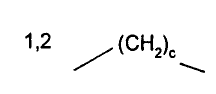 oder =O
substituiert sind,
worin
c eine Zahl 1, 2, 3 oder 4 bedeutet, und/oder die gebildeten Carbocyclen gegebenenfalls durch einen spiro-verknüpften Rest der Formel 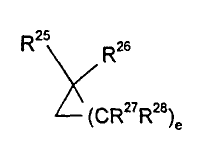 substituiert sind,
worin
e eine Zahl 1, 2, 3, 4 oder 5 bedeutet,
R²⁵, R²⁶, R²⁷ und R²⁸ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten, oder
R²⁵ und R²⁶ oder R²⁷ und R²⁸ gemeinsam eine geradkettige oder verzweigte Alkylkette mit bis zu 4 Kohlenstoffatomen bilden,
und deren Salze und N-Oxide.

Ganz besonders bevorzugt sind erfindungsgemäße Verbindungen der allgemeinen Formel (I),
in welcher
- A: für Cyclopropyl, Cyclopentyl oder Cyclohexyl steht, oder
für Thienyl oder Pyridyl steht, oder A für einen Rest der Formel 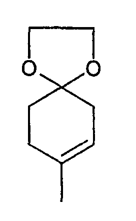 steht,
- D: x für Phenyl steht, das gegebenenfalls durch Trifluormethyl, Fluor, substituiert ist, oder
für einen Rest der Formel 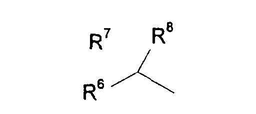 steht,
worin
R⁶ Phenyl bedeutet, das gegebenenfalls durch Fluor, Chlor, Trifluormethyl, Trifluormethoxy, geradkettiges oder verzweigtes Alkyl mit jeweils bis zu 4 Kohlenstoffatomen substituiert ist, oder
R⁷ Wasserstoff oder Fluor bedeutet, und
R⁸ Wasserstoff, Fluor, Chlor, Hydroxy, Methoxy oder
R⁷ und R⁸ gemeinsam für einen Rest der Formel =O stehen,
- E: für Cyclopropyl, Cyclopentyl oder Cyclohexyl
für geradkettiges oder verzweigtes Alkyl mit bis zu 4 Kohlenstoffatomen steht,
- R¹ und R²: gemeinsam eine geradkettige oder verzweigte Alkylenkette mit bis zu 5 Kohlenstoffatomen bilden, die durch eine Carbonylgruppe und/oder einen Rest der Formel -OR¹⁹ substituiert sein muß,
worin
R¹⁹ Wasserstoff bedeutet, oder einen Rest der Formel -Si(CH₃)₂C(CH₃)₃ bedeutet,
und deren Salze und N-Oxide.

Außerdem wurden Verfahren zur Herstellung der erfindungsgemäßen Verbindungen der allgemeinen Formel (I) gefunden, dadurch gekennzeichnet, daß man
[A] im Fall D ≠ Aryl, Verbindungen der allgemeinen Formel (II) 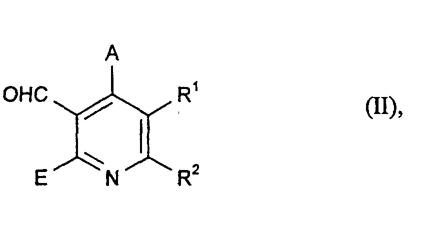 in welcher
   A, E, R¹ und R² die oben angegebene Bedeutung haben,
   mit metallorganischen Reagenzien im Sinne einer Grignard-, Wittig- oder Lithium-organischen-Reaktion den Substituenten D in inerten Lösemitteln synthetisiert,
   oder im Fall, daß D für den Rest der Formel R⁹-T-V-X steht, in welcher V ein Sauerstoffatom bedeutet,
[B] entweder Verbindungen der allgemeinen Formel (III) 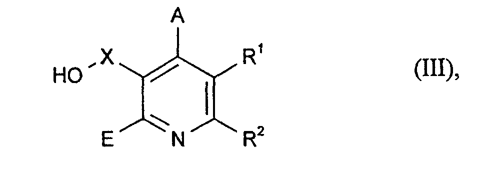 in welcher
   - A, E, X, R¹ und R²: die oben angegebene Bedeutung haben,
   mit Verbindungen der allgemeinen Formel (IV)

   R⁹-T-Z (IV),

   in welcher
   - R⁹ und T: die oben angegebene Bedeutung haben
   und
   - Z: für Halogen, vorzugsweise Chlor oder Brom, steht,
   in inerten Lösemitteln, gegebenenfalls in Anwesenheit einer Base und/oder Hilfsstoffs umsetzt,
   oder
[C] Verbindungen der allgemeinen Formel (III) zunächst durch Umsetzung mit Verbindungen der allgemeinen Formel (V) 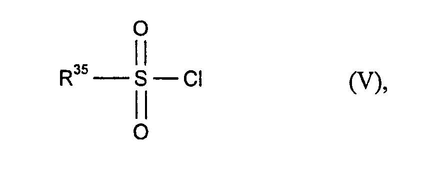 in welcher
   - R³⁵: für geradkettiges Alkyl mit bis zu 4 Kohlenstoffatomen steht,
   in die Verbindungen der allgemeinen Formel (VI) 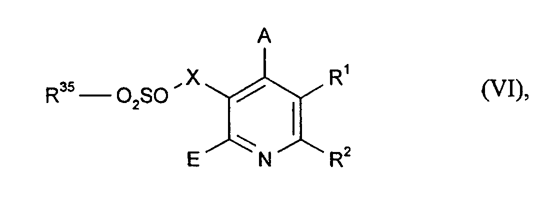 in welcher
   A, E, X, R¹, R² und R³⁵ die oben angegebene Bedeutung haben,
   überführt und anschließend mit Verbindungen der allgemeinen Formel (VII)

   R⁹-T-V-H (VII),

   in welcher
   R⁹, T und V die oben angegebene Bedeutung haben,
   umsetzt und gegebenenfalls Schutzgruppen abspaltet,
   oder
[D] im Fall der Verbindungen der allgemeinen Formel (Ia) 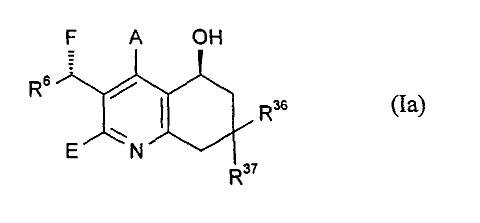 in welcher
   A und R⁶ die oben angegebene Bedeutung haben,
   R³⁶ und R³⁷ gleich oder verschieden sind und
   für Cycloalkyl oder Cycloalkyloxy mit jeweils 3 bis 7 Kohlenstoffatomen stehen, oder
   für geradkettiges oder verzweigtes Alkyl mit bis zu 6 Kohlenstoffatomen stehen, oder
   für Phenyl stehen, das seinerseits gegebenenfalls durch Halogen, Trifluormethyl, Trifluormethoxy oder Nitro substituiert sind, oder
   R³⁶ und R³⁷ für einen der oben aufgeführten spiro-verknüpften Reste der Formel 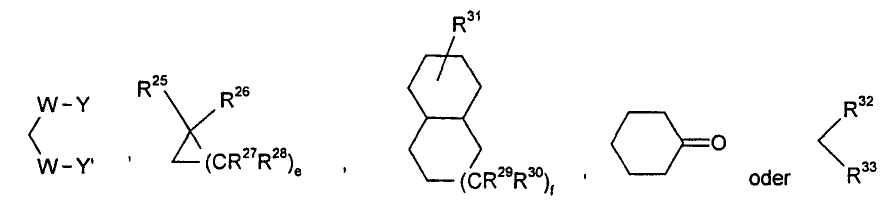 stehen,
   worin
   W, Y, Y', R²⁵, R²⁶, R²⁷, R²⁸, e, R²⁹, R³⁰, R³¹, R³² und R³³ die oben angegebene Bedeutung haben,

Verbindungen der allgemeinen Formel (VIII) 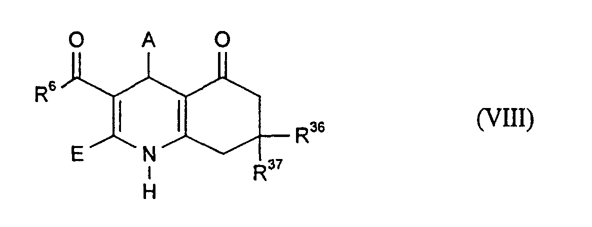 in welcher
R⁶, R³⁶, R³⁷, A und E die oben angegebene Bedeutung haben,
zunächst zu den Verbindungen der allgemeinen Formel (IX) 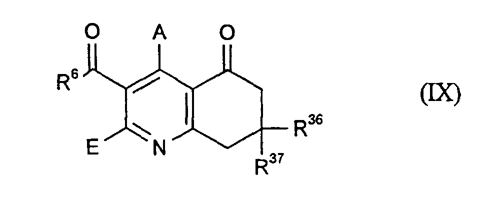 in welcher
R⁶, R³⁶, R³⁷, A und E die oben angegebene Bedeutung haben,
oxidiert,
diese in einem nächsten Schritt durch eine asymmetrische Reduktion zu den Verbindungen der allgemeinen Formel (X) 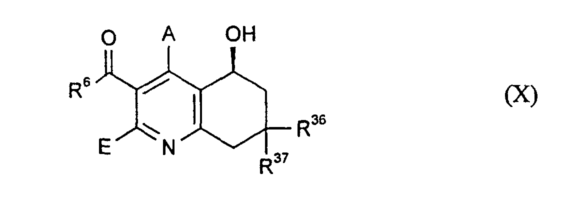 in welcher
R⁶, R³⁶, R³⁷, A und E die oben angegebene Bedeutung haben,
umsetzt,
diese dann durch die Einführung einer Hydroxyschutzgruppe in die Verbindungen der allgemeinen Formel (XI) 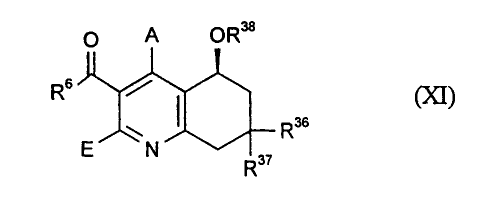 in welcher
- R⁶, R³⁶, R³⁷, A und E: die oben angegebene Bedeutung haben
und
- R³⁸: für eine Hydroxyschutzgruppe, vorzugsweise für einen Rest der Formel - SiR³⁹R⁴⁰R⁴¹ steht,
worin
R³⁹, R⁴⁰ und R⁴¹ gleich oder verschieden sind und C₁-C₄-Alkyl bedeuten,
überführt,
aus diesem in einem Folgeschritt durch diastereoselektive Reduktion die Verbindungen der allgemeinen Formel (XII) 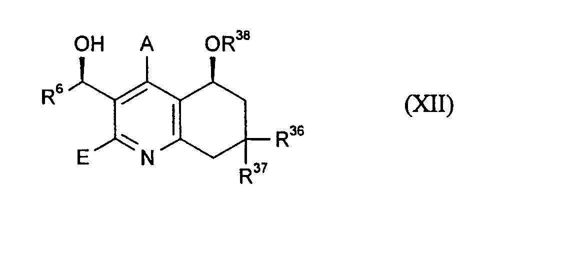 in welcher
R⁶, R³⁶, R³⁷, R³⁸, A und E die oben angegebene Bedeutung haben,
herstellt,
und anschließend durch Einführung des Fluorsubstituenten mit Fluorierungsreagentien, wie z.B. DAST und SF₄-Derivaten die Verbindungen der allgemeinen Formel (XIII) 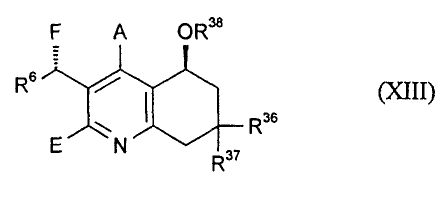 in welcher
R⁶, R³⁶, R³⁷, R³⁸, A und E die oben angegebene Bedeutung haben,
herstellt,
und anschließend die Hydroxyschutzgruppe nach üblichen Methoden abspaltet,
und gegebenenfalls die unter D, E und/oder R¹ und R² aufgeführten Substituenten nach üblichen Methoden variiert oder einführt.

Die erfindungsgemäßen Verfahren können durch folgende Formelschemata beispielhaft erläutert werden:

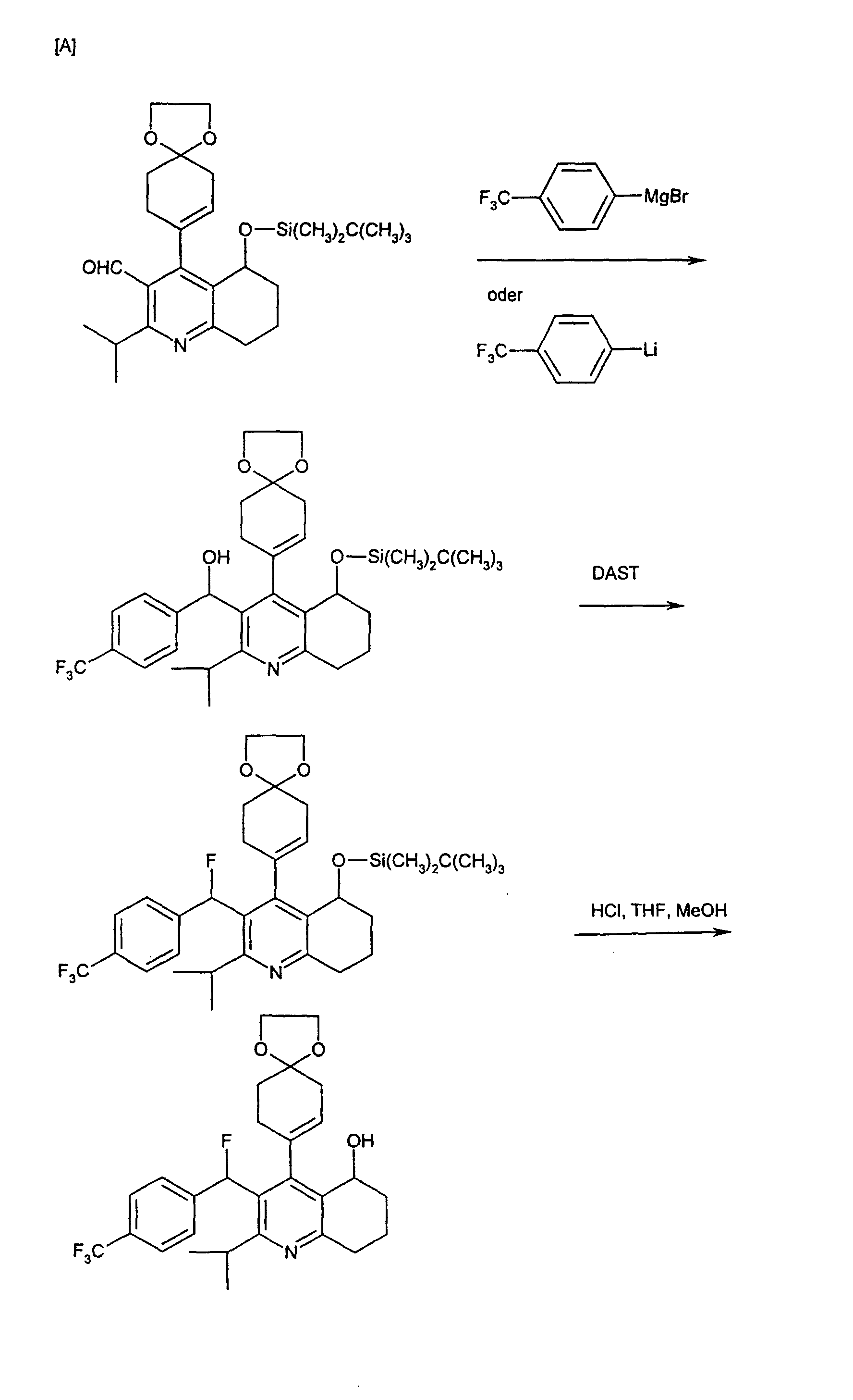

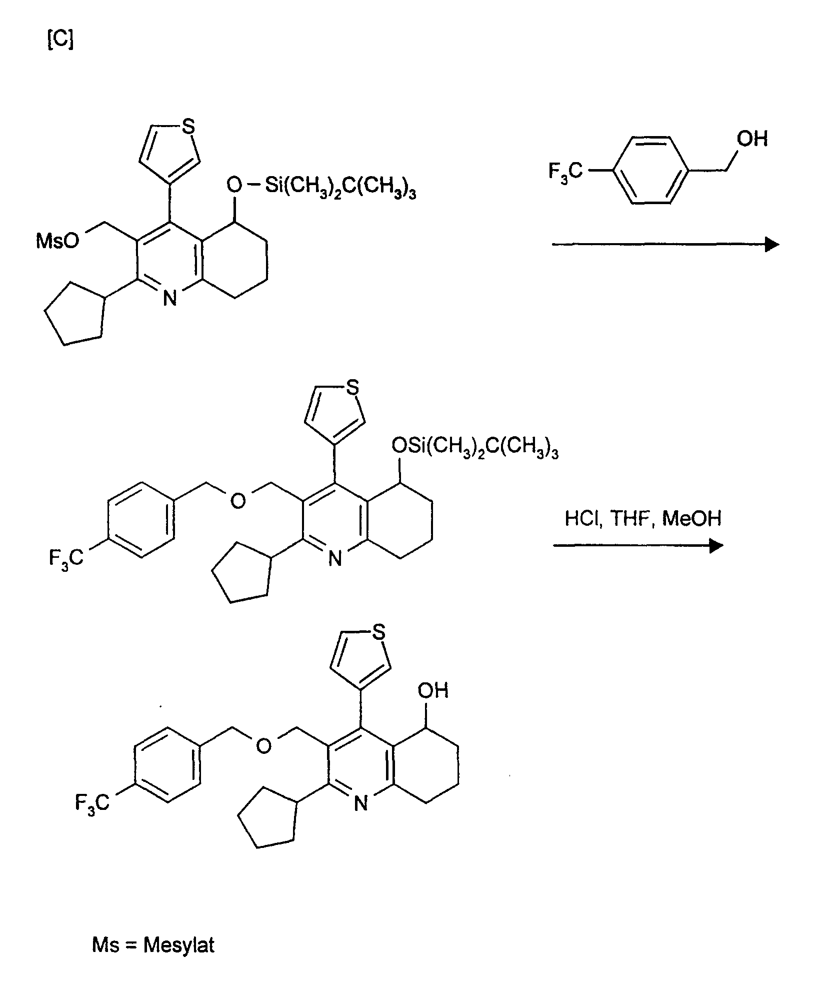

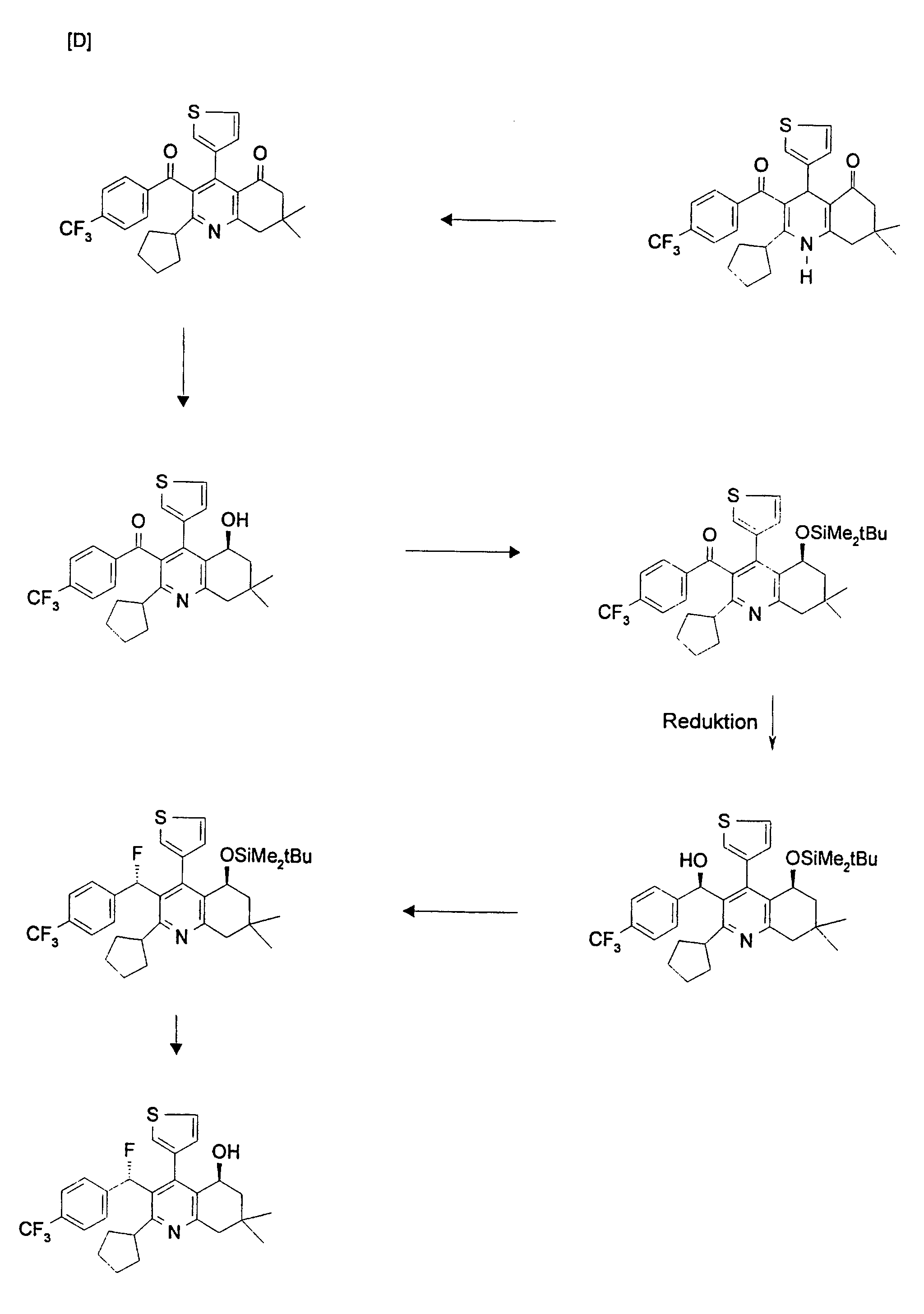

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen kommen für die einzelnen Schritte die üblichen stark basischen Verbindungen in Frage. Hierzu gehören bevorzugt lithiumorganische Verbindungen wie beispielsweise N-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird N-Butyllithium, Natriumhydrid oder Lithiumdiisopropylamid eingesetzt.

Für die Verfahren [B] und [C] eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydrid oder Kaliumhydroxid eingesetzt.

Als metallorganische Reagenzien eignen sich beispielsweise Systeme wie Mg/Brombenzotrifluorid und p-Trifluormethylphenyllithium.

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH, 0°C bis Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Bevorzugt wird das Verfahren im Fall [A] zunächst mit Verbindungen der allgemeinen Formel (II), in welcher der Carbocyclus R¹/R² zunächst nur durch eine Gruppe - OSiR^{I}R^{II}R^{III} substituiert ist, worin R^{I}, R^{II} und R^{III} gleich oder verschieden sind und Phenyl oder geradkettiges oder verzweigtes Alkyl mit bis zu 5 Kohlenstoffatomen bedeuten, durchgeführt und nach Abspaltung der Schutzgruppe der oben unter R¹⁹/R²⁰ angegebene Substituent nach üblichen Methoden einführt.

Die Abspaltung der Schutzgruppe erfolgt im allgemeinen in einem der oben aufgeführten Alkohole und THF, vorzugsweise Methanol / THF in Anwesenheit von Salzsäure in einem Temperaturbereich von 0°C bis 50°C, vorzugsweise bei Raumtemperatur, und Normaldruck. In besonderen Fällen wird die Abspaltung der Schutzgruppe mit Tetrabutylammoniumfluorid (TBAF) in THF bevorzugt.

Hydroxyschutzgruppe im Rahmen der oben angegebenen Definition steht im allgemeinen für eine Schutzgruppe aus der Reihe: Trimethylsilyl, Triisopropylsilyl, tert.-Butyl-dimethylsilyl, Benzyl, Benzyloxycarbonyl, 2-Nitrobenzyl, 4-Nitrobenzyl, tert. - Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Tetrahydropyranyl, tert.Butyldimethylsilyl und Triisopropylsilyl. Besonders bevorzugt ist tert.Butyldimethylsilyl.

Als Lösemittel für die einzelnen Schritte eignen sich Ether wie Diethylether, Dioxan, Tetrahydrofuran, Glykoldimethylether, Diisopropylether oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol. Ebenso ist es möglich, Gemische der genannten Lösemittel zu verwenden.

Als Oxidationsmittel zur Herstellung der Verbindungen der allgemeinen Formel (IX) eignen sich beispielsweise Salpetersäure, Cer(IV)-ammoniumnitrat, 2,3-Dichlor-5,6-dicyan-benzochinon, Pyridiniumchlorochromat (PCC), Pyridiniumchlorochromat auf basischem Aluminiumoxid, Osmiumtetroxid und Mangandioxid. Bevorzugt sind Mangandioxid und Salpetersäure.

Die Oxidation erfolgt in einem der oben aufgeführten chlorierten Kohlenwasserstoffe und Wasser. Bevorzugt sind Dichlormethan und Wasser.

Das Oxidationsmittel wird in einer Menge von 1 mol bis 10 mol, bevorzugt von 2 mol bis 5 mol, bezogen auf 1 mol der Verbindungen der allgemeinen Formel (VIII), eingesetzt.

Die Oxidation verläuft im allgemeinen bei einer Temperatur von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die asymmetrische Reduktion zu den Verbindungen der allgemeinen Formel (X) erfolgt im allgemeinen in einem der oben aufgeführten Ether oder Toluol, vorzugsweise Tetrahydrofuran und Toluol.

Die Reduktion erfolgt im allgemeinen mit enantiomerenreinen 1R,2S-Aminoindanol und Borankomplexen wie BH₃ x THF, BH₃ x DMS und BH₃ x (C₂H₅)₂NC₆H₅. Bevorzugt ist das System Borandiethylanilin / 1R,2S-Aminoindanol.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen bei einer Temperatur von -78°C bis +50°C, bevorzugt von 0°C bis 30°C.

Die Reduktion verläuft im allgemeinen bei Normaldruck, es ist aber auch möglich bei erhöhtem oder erniedrigtem Druck zu arbeiten.

Die Einführung der Hydroxyschutzgruppe erfolgt in einem der oben aufgeführten Kohlenwasserstoffe, Dimethylformamid oder THF, vorzugsweise in Toluol in Anwesenheit von Lutidin in einem Temperaturbereich von -20°C bis +50°C, vorzugsweise von -5°C bis Raumtemperatur und Normaldruck.

Reagenzien zur Einführung der Silylschutzgruppe sind im allgemeinen tert.-Butyldimethylsilylchlorid oder tert.-Butyldimethylsilyltrifluormethansulfonat. Bevorzugt ist tert.-Butyldimethylsilyltrifluormethansulfonat.

Die Reduktion zu den Verbindungen der allgemeinen Formel (XII) verläuft in einem der oben aufgeführten Kohlenwasserstoffe, vorzugsweise Toluol.

Die Reduktion zur Herstellung der Verbindungen der allgemeinen Formel (XII) wird im allgemeinen mit üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in den oben aufgeführten inerten Lösemitteln, wie z.B. Toluol, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid, Natrium-bis-(2-methoxyethoxy)-dihydroaluminat oder Lithiumaluminiumhydrid durchgeführt. Ganz besonders bevorzugt wird die Reduktion mit Natrium-bis-(2-methoxyethoxy)-dihydroaluminat durchgeführt.

Das Reduktionsmittel wird im allgemeinen in einer Menge von 1 mol bis 6 mol, bevorzugt von 1 mol bis 3 mol bezogen auf 1 mol der zu reduzierenden Verbindungen, eingesetzt.

Die Reduktion verläuft im allgemeinen bei einer Temperatur von -20°C bis +110°C, bevorzugt von 0°C bis Raumtemperatur.

Die Reduktion verläuft im allgemeinen bei Normaldruck, es ist aber auch möglich bei erhöhtem oder erniedrigtem Druck zu arbeiten.

Bei der Reduktion zu den Verbindungen der allgemeinen Formel (XII) bleiben in der Mutterlauge geringe Reste des falschen Diastereomeren. Diese Reste können mit gängigen Oxidationsmitteln wie z.B. Pyridiniumchlorochromat (PCC) oder aktiviertem Braunstein, insbesondere mit aktiviertem Braunstein zu geschütztem (XI) reoxidiert werden und somit dem Synthesezyklus ohne Ausbeuteverlust zugeführt werden.

Die Einführung des Fluorsubstituenten erfolgt im allgemeinen in einem der oben aufgeführten Kohlenwasserstoffe oder Methylenchlorid, vorzugsweise in Toluol und unter Schutzgasatmosphäre.

Unter SF₄-Derivaten werden im allgemeinen Diethylaminoschwefeltrifluorid oder 2,2'-Bisfluorsubstituierte Amine wie beispielsweise Diethyl-1,2,3,3,3-hexafluorpropylamin hergestellt.

Die Reaktion verläuft im allgemeinen bei einer Temperatur von -78°C bis 100°C, bevorzugt im Falle des Dimethylaminoschwefeltrifluorid bei -78°C bis RT und im Falle des Diethyl-1,1,2,3,3,3-hexafluorpropylamins bei Raumtemperatur bis 80°C.

Die Abspaltung der Schutzgruppe erfolgt im allgemeinen in einem der oben aufgeführten Alkohole und THF, vorzugsweise Methanol / THF in Anwesenheit von Salzsäure in einem Temperaturbereich von 0°C bis 50°C, vorzugsweise bei Raumtemperatur, und Normaldruck. In besonderen Fällen wird die Abspaltung der Schutzgruppe mit Tetrabutylammoniumfluorid (TBAF) in THF bei Raumtemperatur bevorzugt.

Als Derivatisierungen seien beispielhaft folgende Reaktionstypen genannt:

Oxidationen, Reduktionen, Hydrierungen, Halogenierung, Wittig/Grignard-Reaktionen und Amidierungen/Sulfoamidierungen.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird N-Butyllithium, Natriumhydrid oder Lithiumdiisopropylamid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Als Lösemittel eignen sich für die einzelnen Reaktionsschritte auch Alkohole wie Methanol, Ethanol, Propanol, Butanol oder tert.Butanol. Bevorzugt ist tert.Butanol.

Gegebenenfalls ist es nötig, einige Reaktionsschritte unter Schutzgasatmosphäre durchzuführen.

Die Halogenierungen erfolgen im allgemeinen in einem der oben aufgeführten chlorierten Kohlenwasserstoffen, wobei Methylenchlorid bevorzugt ist.

Als Halogenierungsmittel eignen sich beispielsweise Diethylamino-Schwefeltrifluorid (DAST), Morpholino-Schwefeltrifluorid oder SOCl₂.

Die Halogenierung verläuft im allgemeinen in einem Temperaturbereich von -78°C bis +50°C, bevorzugt von -78°C bis 0°C, jeweils in Abhängigkeit von der Wahl des Halogenierungsmittels sowie Lösemittel.

Die Halogenierung verläuft im allgemeinen bei Normaldurck, es ist aber auch möglich, bei erhöhtem oder erniedrigtem Druck zu arbeiten.

Die Verbindungen der allgemeinen Formeln (II) und (III) sind neu und können hergestellt werden, indem man
durch Umsetzung der Verbindungen der allgemeinen Formel (XIV) 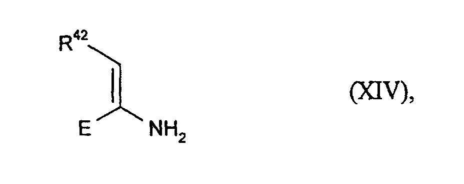 in welcher
- E: die oben angegebene Bedeutung hat
und
- R⁴²: für C₁-C₄-Alkoxycarbonyl oder Aryl (D = Aryl) steht,
mit Aldehyden der allgemeinen Formel (XV)

A-CHO (XV),

in welcher
- A: die oben angegebene Bedeutung hat,
und Verbindungen der allgemeinen Formel (XVI) 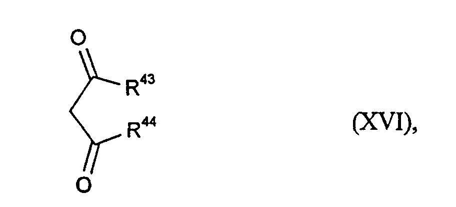 in welcher
- R⁴³ und R⁴⁴: unter Einbezug einer Carbonylgruppe den oben angegebenen Bedeutungsumfang von R¹ und R² umfassen,
die Verbindungen der allgemeinen Formel (XVII) 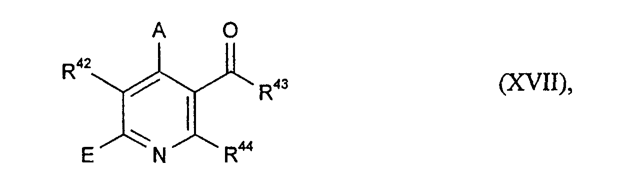 in welcher
- A, E, R⁴², R⁴³ und R⁴⁴: die oben angegebene Bedeutung haben,
herstellt,
und im Fall der Verbindungen der allgemeinen Formel (III) eine Reduktion, wie oben beschrieben, zur Hydroxymethylfunktion anschließt,
und in einem letzten Schritt die Alkoxycarbonylgruppe (R⁴²) durch eine Reduktions-Oxidations-Sequenz in eine Aldehydgruppe überführt.

Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Methylenchlorid.

Als Oxidationsmittel eignen sich beispielsweise Cer(IV)-ammoniumnitrat, 2,3-Dichlor-5,6-dicyan-benzochinon, Pyridiniumchlorochromat (PCC), Pyridiniumchlorochromat auf basischem Aluminiumoxid, Osmiumtetroxid und Mangandioxid. Bevorzugt sind Schwefeltrioxid-Pyridinkomplex in DMSO/Methylenchlorid und Pyridiniumchlorochromat auf basischem Aluminiumoxid.

Das Oxidationsmittel wird in einer Menge von 1 mol bis 10 mol, bevorzugt von 2 mol bis 5 mol, bezogen auf 1 mol der Verbindungen der allgemeinen Formel (XVII), eingesetzt.

Die Oxidation verläuft im allgemeinen in einem Temperaturbereich von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die Verbindungen der allgemeinen Formeln (IV), (V), (VII), (XIV), (XV) und (XVI) sind an sich bekannt oder nach üblichen Methoden herstellbar.

Die Verbindungen der allgemeinen Formeln (VI) und (XV) sind teilweise bekannt oder neu und können dann wie oben beschrieben hergestellt werden.

Die Verbindungen der allgemeinen Formeln (IX) und (X) sind als Species neu und können wie oben beschrieben hergestellt werden.

Die Verbindungen der allgemeinen Formel (VIII) sind neu und können hergestellt werden, indem man

Verbindungen der allgemeinen Formeln (XVa), (XVIII) und (XIX)

A-CHO (XVa),

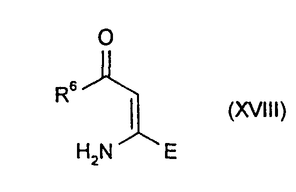

und 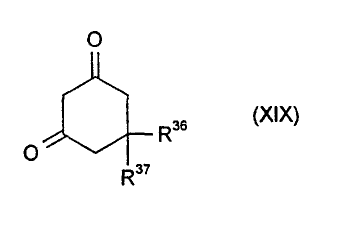 in welcher
- A, E, R⁶, R³⁶ und R³⁷: die oben angegebene Bedeutung haben,
in Gegenwart einer Säure umsetzt.

Als Lösemittel zur Herstellung der Verbindungen der allgemeinen Formel (VIII) eignen sich die oben aufgeführten Ether oder Alkohole. Bevorzugt ist Diisopropylether.

Als Säuren für die Herstellung der Verbindungen der allgemeinen Formel (VIII) eignen sich im allgemeinen organische Carbonsäuren und anorganische Säuren, wie beispielsweise Oxalsäure, Maleinsäure, Phosphorsäure, Fumarsäure und Trifluoressigsäure. Bevorzugt ist Trifluoressigsäure.

Die Säure wird im allgemeinen in einer Menge von 0,1 mol bis 5 mol, bevorzugt 1 mol, bezogen auf 1 mol der Verbindungen der allgemeinen Formel (XIX) eingesetzt.

Die Reaktion wird im allgemeinen bei Normaldruck durchgeführt. Es ist aber auch möglich die Reaktion bei erhöhtem oder erniedrigtem Druck durchzuführen.

Die Reaktion erfolgt im allgemeinen bei der Rückflußtemperatur des jeweiligen Lösemittels.

Die Verbindungen der allgemeinen Formeln (XV) und (XIX) sind an sich bekannt oder nach üblichen Methoden herstellbar.

Die Verbindungen der allgemeinen Formel (XVIII) sind neu und können hergestellt werden, indem man zunächst durch Umsetzung der Verbindungen der allgemeinen Formel (XX)

E-CO₂-R⁴⁵ (XX)

in welcher
- E: die oben angegebene Bedeutung hat
und
- R⁴⁵: für C₁-C₄-Alkyl steht,
mit Verbindungen der allgemeinen Formel (XXI) 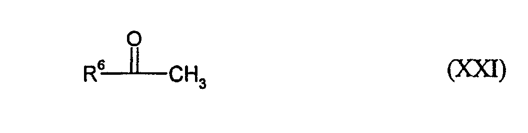 in welcher
- R⁶: die oben angegebene Bedeutung hat,
in einem Lösemittel in Anwesenheit von 18-Krone-6-ether die Verbindungen der allgemeinen Formel (XXII) 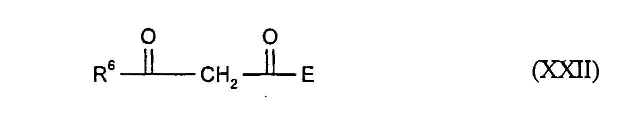 in welcher
- R⁶ und E: die oben angegebene Bedeutung haben,
herstellt
und anschließend mit Ammoniumacetat in inerten Lösemitteln umsetzt.

Als Lösemittel für den ersten Schritt des Verfahrens eignen sich die oben aufgeführten Ether und Kohlenwasserstoffe, wobei Tetrahydrofuran bevorzugt ist.

Als Lösemittel für die Umsetzung mit den Verbindungen der allgemeinen Formel (XXII) eignen sich Alkohole, wie beispielsweise Methanol, Ethanol, Propanol oder Isopropanol. Bevorzugt ist Ethanol.

Alle Schritte des Verfahrens erfolgen bei der jeweiligen Rückflußtemperatur des entsprechenden Lösemittels und bei Normaldruck.

Die Verbindungen der allgemeinen Formeln (XX) und (XXI) sind teilweise bekannt oder können nach bekannten Methoden hergestellt werden.

Die Verbindungen der allgemeinen Formel (XXII) sind als Species partiell neu und können wie oben beschrieben hergestellt werden.

Die erfindungsgemäßen Verbindungen der allgemeinen Formeln (I) und (Ia) haben ein nicht vorhersehbares pharmakologisches Wirkspektrum.

Die erfindungsgemäßen Verbindungen der allgemeinen Formeln (I) und (Ia) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den Reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung und Prävention von Hypolipoproteinämie, Dyslipidämien, Hypertriglyceridämien, Hyperlipidämien oder Arteriosklerose eingesetzt werden.

Die pharmakologische Wirkung der erfindungsgemäßen Stoffe wurde in folgendem Test bestimmt:

### CETP-Inhibitions-Testung

### Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 m NaCl/0,001 m TrisHCl pH 7,4 gewaschen und anschließend mit dest. Wasser eluiert. Die CETP-aktiven Fraktionen werden gepoolt, gegen 50mM NaAcetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### Gewinnung von radioaktiv markiertem HDL

50 ml frisches humanes EDTA-Plasma wird mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl 3H-Cholesterin (Dupont NET-725; 1 -µC/µl gelöst in Ethanol !) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert.

Das Retentat enthält radioaktiv markiertes ³H-CE-HDL, das auf ca. 5x10⁶ cmp pro ml eingestellt zum Test verwendet wird.

### CETP-Test

Zur Testung der CETP-Aktivität wird die Übertragung von ³H-Cholesterolester von humanen HD-Lipoproteinen auf biotinylierte LD-Lipoproteine gemessen.

Die Reaktion wird durch Zugabe von Streptavidin-SPA®beads (Fa. Amersham) beendet und die übertragene Radioaktivität direkt im Liquid Scintillation Counter bestimmt.

Im Testansatz werden 10 µl HDL-³H-Cholesterolester (- 50.000 cpm) mit 10 µl Biotin-LDL (Fa. Amersham) in 50 mM Hepes / 0,15 m NaCl / 0,1% Rinderserumalbumin /0,05% NaN₃ pH 7,4 mit 10 µl CETP (1 mg/ml) und 3 µl Lösung der zu prüfenden Substanz (in 10% DMSO / 1% RSA gelöst), für 18 h bei 37°C inkubiert. Anschließend werden 200 µl der SPA-Streptavidin-Bead-Lösung (TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.

Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle A sind die IC₅₀-Werte (mol/l) für CETP-Inhibitoren angegeben:

**Tabelle A:**

| **Beispiel-Nr.** | **IC**_{**50**}**-Wert (mol/l)** |
|---|---|
| 1 | 1x 10⁻⁸ |

### Ex vivo Aktivität der erfindungsgemäßen Verbindungen

Syrische Goldhamster aus werkseigener Zucht werden nach 24-stündigem Fasten narkotisiert (0,8 mg/kg Atropin, 0,8 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen.

Die Verabreichung der Testsubstanzen kann auch p.o. erfolgen, indem die Substanzen in DMSO gelöst und 0,5% Tylose suspendiert mittels Schlundsonde peroral verabreicht werden. Die Kontrolltiere erhalten identische Volumen Lösemittel ohne Testsubstanz.

Nach verschiedenen Zeitpunkten - bis zu 24 Stunden nach Applikation - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 x g zentrifugiert. Im so erhaltenen Serum wird die CETP-Aktivität durch den modifizierten CETP-Test bestimmt. Es wird wie für den CETP-Test oben beschrieben die Übertragung von ³H-Cholesterolester von HD-Lipoproteinen auf biotinylierte LD-Lipoproteine gemessen.

Die Reaktion wird durch Zugabe von Streptavidin-SPA^{R}beads (Fa. Amersham) beendet und die übertragene Radioaktivität direkt im Liquid Scintlation Counter bestimmt.

Der Testansatz wird wie unter "CETP-Test" beschrieben durchgeführt. Lediglich 10 µl CETP werden für die Testung der Serum durch 10 µl der entsprechenden Serumproben ersetzt. Als Kontrollen dienen entsprechende Inkubationen mit Seren von unbehandelten Tieren.

Die in den Kontrollansätzen mit Kontrollseren übertragene Aktivität wird als 100% Übertragung gewertet. Die Substanzkonzentration, bei der diese Übertragung auf die Hälfte reduziert ist wird als ED₅₀-Wert angegeben.

### In vivo Aktivität der erfindungsgemäßen Verbindungen

Bei Versuchen zur Bestimmung der oralen Wirkung auf Lipoproteine und Triglyceride wird syrischen Goldhamstern aus werkseigener Zucht Testsubstanz in DMSO gelöst und 0,5% Tylose suspendiert mittels Schlundsonde peroral verabreicht. Zur Bestimmung der CETP-Aktivität wird vor Versuchsbeginn durch retro-orbitale Punktion Blut entnommen (ca. 250 µl). Anschließend werden die Testsubstanzen peroral mittels einer Schlundsonde verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen.

Durch Inkubation von 4°C über Nacht wird die Gerinnung abgeschlossen, anschließend wird 10 Minunten bei 6000 x g zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.

100 µl Serum-Verdünnung werden mit 100 µl Testsubstanz in 96-Lochplatten versetzt und 10 Minuten bei Raumtemperatur inkubiert. Anschließend wird die optische Dichte bei einer Wellenlänge von 492 nm mit einem automatischen Platten-Lesegerät bestimmt. Die in den Proben enthaltene Triglycerid- bzw. Cholesterinkonzentration wird mit Hilfe einer parallel gemessenen Standardkurve bestimmt.

Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt.

### In vivo Wirksamkeit an transgenen hCETP-Mäusen

Transgenen Mäusen aus eigener Zucht (Dinchuck, Hart, Gonzalez, Karmann, Schmidt, Wirak; BBA (1995), 1295, 301) wurden die zu prüfenden Substanzen im Futter verabreicht. Vor Versuchsbeginn wurde den Mäusen retroorbital Blut entnommen, um Cholesterin und Triglyceride im Serum zu bestimmen. Das Serum wurde wie oben für Hamster beschrieben durch Inkubation bei 4°C über Nacht und anschließender Zentrifugation bei 6000 x g gewonnen. Nach einer Woche wurde den Mäusen wieder Blut entnommen, um Lipoproteine und Triglyceride zu bestimmen. Die Veränderung der gemessenen Parameter werden als prozentuale Veränderung gegenüber dem Ausgangswert ausgedrückt.

Die Erfindung betrifft außerdem die Kombination von Hetero-Tetrahydrochinolinen der allgemeinen Formel (I) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Weiterhin können die erfindungsgemäßen Verbindungen in Kombination mit Cholesterin senkenden Vastatinen oder ApoB-senkenden Prinzipien kombiniert werden, um Dyslipidemien, kombinierte Hyperlipidemien, Hypercholesterolemien oder Hypertriglyceridemien zu behandeln.

Die genannten Kombinationen sind auch zur primären oder sekundären Prävention koronarer Herzerkrankungen (z.B. Myokardinfarkt) einsetzbar.

Vastatine im Rahmen der Erfindung sind beispielsweise Lovastatin, Simvastatin, Pravastatin, Fluvastatin, Atorvastatin und Cerivastatin. ApoB senkende Mittel sind zum Beispiel MTP-Inhibitoren.

Bevorzugt ist die Kombination von Cerivastatin oder ApoB-Inhibitoren mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise intravenös, oral, parenteral oder perlingual, insbesondere oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkützungen:

- Cy =: Cyclohexan
- EE =: Essigester
- PE =: Petrolether
- THF =: Tetrahydrofuran
- DAST =: Dimethylaminoschwefeltrifluorid
- PTS =: para-Toluolsulfonsäure
- PDC =: Pyridiniumdichromat
- PE/EE =: Petrolether / Essigsäureethylester
- Tol =: Toluol

### Ausgangsverbindungen

### Beispiel I

### 2-Cyclopentyl-7,7-dimethyl-4-(3-thienyl)-3-(4-trifluormethylbenzoyl)-1,4,5,6,7,8-hexahydro-chinolin-5-on

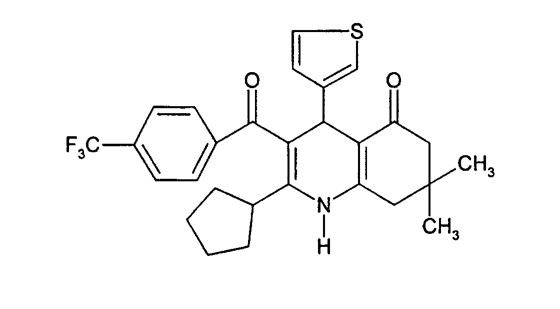

1,425 g (5,03 mol) 3-Amino-3-cyclopentyl-1-(4-trifluormethylphenyl)-propenon werden in 25 ml Diisopropylether suspendiert. Es werden 740 mg (5,28 mol) Dimedon, 0,39 ml (5,03 mol) Trifluoressigsäure und anschließend 592 mg (5,28 mol) Thiophen-3-aldehyd zugegeben. Es wird 2 Stunden zum Rückfluß erhitzt, wobei sofort eine gelbe Lösung entsteht, aus der nach 30 Minuten Produkt ausfällt. Es wird abgekühlt, abgesaugt und mit Diisopropylether gewaschen. Es wird aus Acetonitril umkristallisiert.
Ausbeute: 741 mg, Schmp. 228 - 229°C

Aus der Mutterlauge werden noch 230 mg reines Produkt erhalten.

In Analogie zur Vorschrift des Beispiels I werden die in der Tabelle I aufgeführten Verbindungen hergestellt:

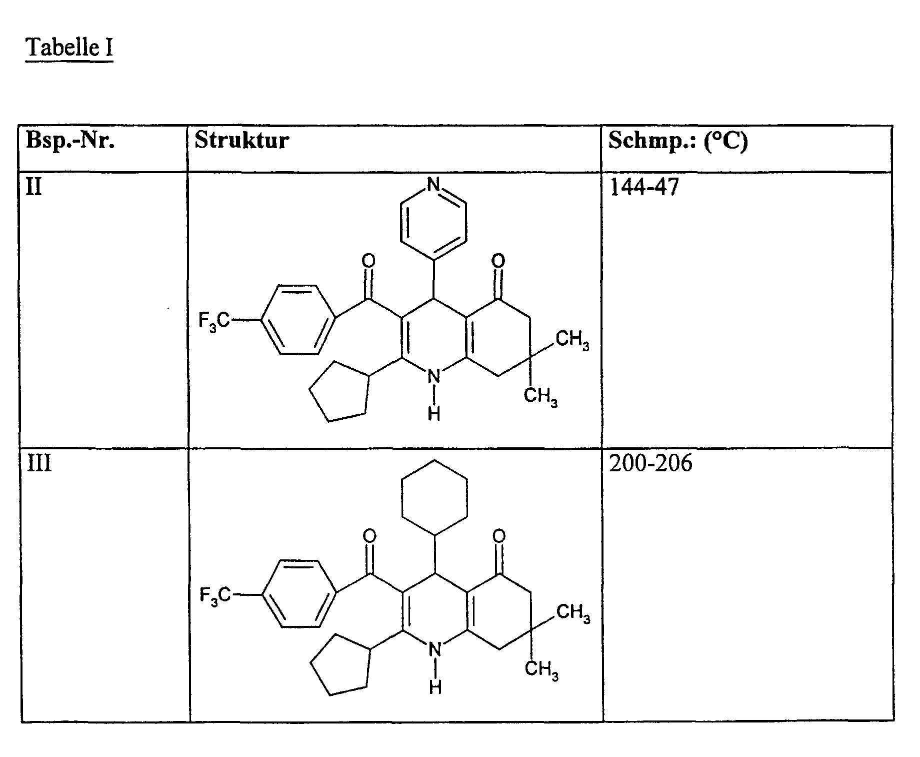

### Herstellungsbeispiele

### Beispiel 1

### 2-Cyclopentyl-7,7-dimethyl-4-(3-thienyl)-3-(4-trifluormethylbenzoyl)-5,6,7,8-tetrahydro-chinolin-5-on

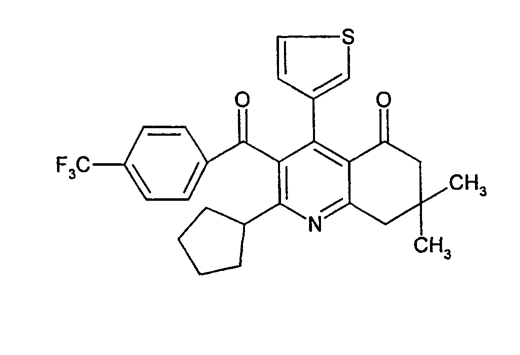

1,21 g (2,42 mmol) der Verbindung aus Beispiel I werden in 35 ml Dichlormethan gelöst und nach Zugabe von 6,8 g Mangandioxid 2 Stunden gerührt. Es wird über Celite als Filtrierhilfsmittel abgesaugt und eingeengt. Der Eindampfrückstand wird mit Acetonitril verrührt, abgesaugt und mit Acetonitril gewaschen. Man erhält 1,045 g Kristalle vom Schmp.: 236-238°C

Analog der Vorschrift des Beispiels 1 werden die in der Tabelle 1 aufgeführten Verbindungen hergestellt:

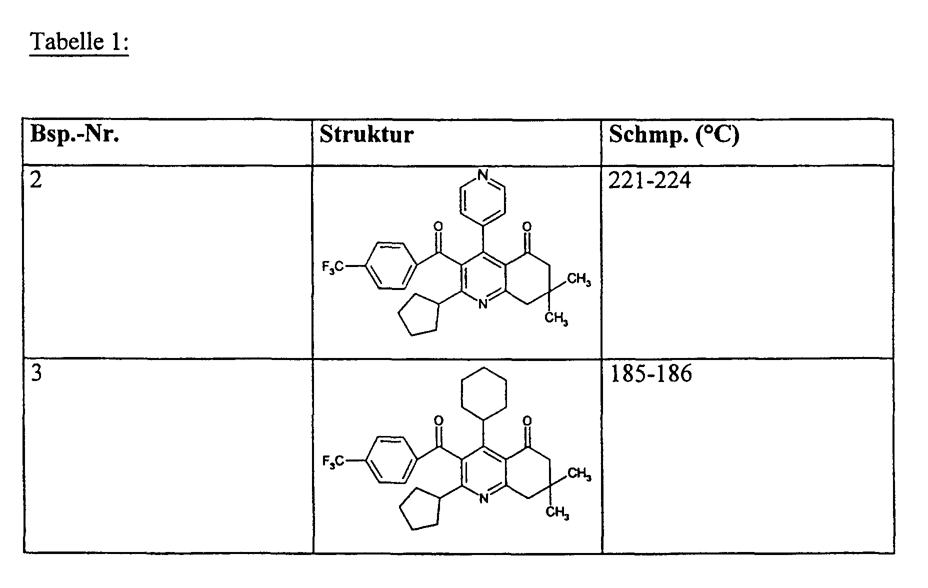

### Beispiel 4

### 2-Cyclopentyl-5-hydroxy-7,7-dimethyl-4-(3-thienyl)-3-(4-trifluormethylbenzoyl)-5,6,7,8-tetrahydrochinolin

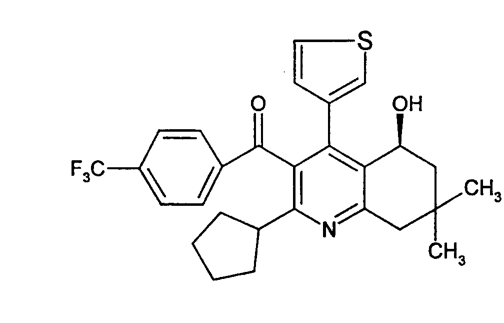

(1R,2S)-Aminoindan-2-ol werden in 0,4 ml THF suspendiert. Bei RT werden N,N-Borandiethylanilincomplex (Aldrich) zugetropft. Es wird alles gelöst und 1 Stunde bei RT gerührt.Dann wird 15 Minuten bei 0°C gerührt. Die Verbindung aus Beispiel 1 wird in 16 ml THF gelöst und wird innerhalb von 10 Minuten bei 0 bis 5°C zugetropft. Dann wird 30 min bei 0°C und 4 Stunden bei RT gerührt. Bei -10°C bis 0°C werden 35 ml 1,2 Ethandiol vorsichtig zugetropft, 30 Minuten nachgerührt, eingeengt, in Essigester gelöst, mit 1 N HCl, dann mit ges. Natriumhydrogencarbonatlösung, dann mit ges. Kochsalzlösung gewaschen, über Natriumsulfat getrocknet, filtriert und eingeengt. Man erhält 1,17 g kristalline Verbindung. Es wird heiß in Cyclohexan gelöst, abfiltriert. Beim Abkühlen kristallisiert die Verbindung aus. Es wird abgesaugt, gewaschen und bei 70°C i.V. getrocknet.
Ausbeute: 0,7 g

Nach Einengen der Mutterlauge (Säule: Toluol:Essigester 20.1) und Lösen in Methylenchlorid und erneutem Einengen werden weitere 0,27 g Kristalle erhalten.
Gesamtausbeute: 970 mg (87,3%) vom Schmp.: 179-182°C

### Beispiel 5

### 5-tert.Butyldimethylsilyloxy-2-cyclopentyl-7,7-dimethyl-4-(3-thienyl)-3-(4-trifluormethylbenzoyl)-5,6,7,8-tetrahydrochinolin

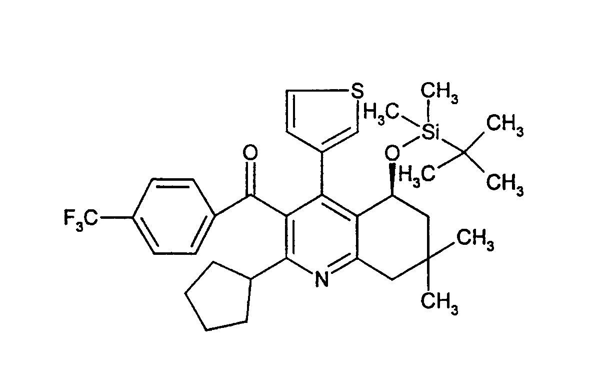

0,8 g (1,6 mmol) der Verbindung aus Beispiel 4 werden in 6,4 ml Toluol unter Argon gelöst und bei -5° bis -10°C tropfenweise mit 0,69 g (6,4 mmol) 2,6-Lutidin versetzt. Es wird 15 Minunten nachgerührt. Bei gleicher Temperatur werden nun 0,86 g (3,2 mmol) Trifluormethansulfonsäure-(tert.butyl-dimethylsilylester) in 1,2 ml Toluol zugetropft. Es wird 15 Minuten bei -5°C bis -10°C, dann 2 Stunden bei Raumtemperatur gerührt.

Es wrid mit Toluol verdünnt, nacheinander mit 2,6 ml 10% Ammoniumchloridlösung, 7 mal mit je 3,5 ml 0,1 N HCl, je einmal mit 1,5 ml ges. Natriumhydrogencarbonatlösung und mit 3,5 ml ges. Natriumchloridlösung gewaschen. Anschließend wird getrocknet, eingeengt und einmal mit Ethanol eingeengt.

Man erhält 1,0 g Es wird aus wenig Ethanol umkristallisiert, abgesaugt, gewaschen und bei 60°C i.V. getrocknet.
Ausbeute: 716 mg, Schmp. 147 - 148°C

Die Mutterlauge wird eingeengt und mit Ethanol abgesaugt. Man erhält nochmals 60 mg.

### Beispiel 6

### 5-(S)-tert.Butyldimethylsilyloxy-2-cyclopentyl-7,7-dimethyl-4-(3-thienyl)-3-[(R)-hydroxy-(4-trifluormethylphenyl)]methyl-5,6,7,8-tetrahydrochinolin

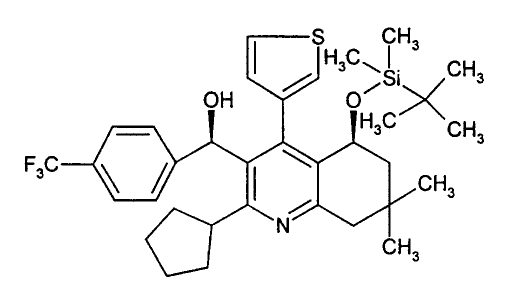

0,69 g (1,124 mmol) der Verbindung aus Beispiel 5 werden in 5 ml Toluol gelöst; bei 0°C werden 1,40 g (4,496 mmol) RED-A1 zugetropft und 30 Minuten bei 0°C und 1 Stunde bei RT gerührt. Bei 0°C werden langsam 0,85 ml Methanol zugetropft und die gelbe Lösung 30 Minuten bei 0°C gerührt. Anschließend werden 0,73 ml einer 20%igen Kalium-Natrium-Tartrat-Lösung zugetropft, abgesaugt, mit Toluol und etws 20%iger Kalium-Natrium-Tartrat-Lösung gewaschen, abgetrennt, einmal mit ges. Natriumhydrogencarbonatlösung und zweimal mit ges. Kochsalzlösung gewaschen, über Natriumsulfat getrocknet und eingeengt. Man erhält 850 mg Öl, bestehend aus den beiden möglichen Diastereomeren, die über eine 400 ml Kieselgelsäule getrennt werden. Es wird mit Petrolether, Petrolether/Essigester 20:1, 10:1 eluiert.

Man erhält 86,2 mg des falschen Diastereomeren und 356,2 mg des richtigen Diastereomeren.

### Beispiel 7

### 5-(S)-tert.Butyldimethylsilyloxy-2-cyclopentyl-7,7-dimethyl-3-[5-fluor-(4-trifluormethylphenyl)-methyl]-4-(3-thienyl)-5,6,7,8-tetrahydrochinolin

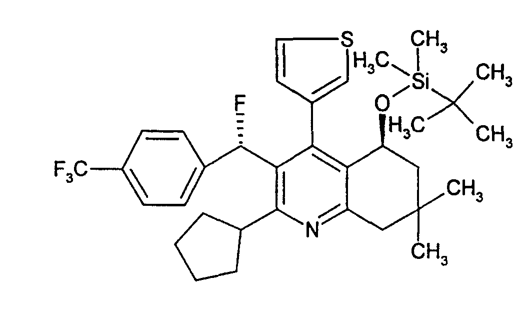

320 mg (0,52 mmol) der Verbindung aus Beispiel 6 werden in 7 ml Dichlormethan gelöst und bei -15°C mit 140 mg (0,86 mmol) DAST versetzt. Nach 30 Minuten wird bei -15°C bis -10°C umgesetzt, mit Methylenchlorid und Wasser versetzt, abgetrennt, einmal mit Methylenchlorid extrahiert, die organischen Phasen einmal mit ges. Kochsalzlösung und mit etwas ges. Natriumhydrogencarbonatlösung gewaschen, getrocknet und eingeengt.

Die Kristallisation erfolgt aus Methanol aus. Es wird abgesaugt und gewaschen.
Ausbeute: 57,8 mg vom Schmp.: 171-172°C

### Beispiel 8

### 2-Cyclopentyl-3-[fluor-(4-trifluormethylphenyl)methyl]-5-hydroxy-7,7-dimethyl-4-(3-thienyl)-5,6,7,8-tetrahydrochinolin

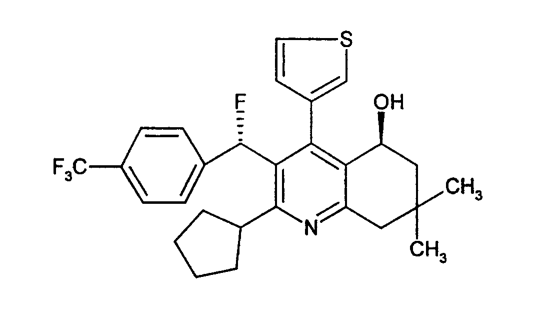

111 mg (0,18 mmol) der Verbindung aus Beispiel 7 werden in 1,4 ml Methanol gelöst und mit 0,9 ml THF und 0,98 ml 5 N Salzsäure versetzt. Es wird 4 Stunden bei 40°C gerührt. Es wird eingeengt, mit Wasser, Ammoniaklösung und Essigester versetzt, abgetrennt und einmal mit Essigester extrahiert. Die organischen Phasen werden einmal mit Kochsalzlösung gewaschen, getrocknet und eingeengt. man erhält 82 mg als Öl.

Es wird in Petrolether und wenig Methylenchlorid gelöst und auf eine Säule gegeben, mit Petrolether : Essigester 30.1, 20.1, 10.1 eluiert und 2 Fraktionen eingeengt.

Der kristalline Feststoff wird mit wenig n-Heptan abgesaugt und i.V. getrocknet.

Man erhält 37,1 mg (41% d.Th.) einer farblosen Substanz vom Schmp.: 157-159°C

### Beispiel 9

### 2-Cyclopentyl-5-hydroxy-7,7-dimethyl-4-(3-thienyl)-3-(trifluormethylbenzyl)-5,6,7,8-tetrahydrochinolin

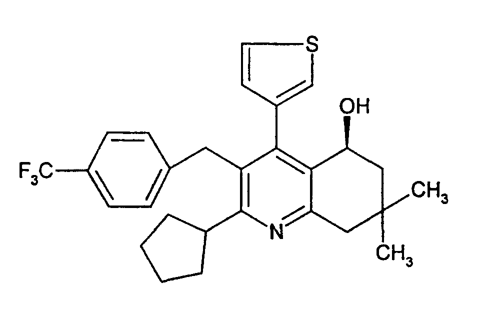

20 mg (0,04 mmol) der Verbindung aus Beispiel 8 werden in 3 ml Toluol gelöst und bei -20°C mit 0,27 ml Dibal-H in Tolol versetzt. Es wird 2 Stunden bei -20°C gerührt. Es wird mit 10 ml 20% Kaliumnatriumtartratlösung und Essigester versetzt, etwas gerührt, abgetrennt, 2 x mit Essigester extrahiert, die organischen Phasen getrocknet und eingeengt.

17 mg der Titelverbindung werden in Methylenchlorid gelöst, über eine Säule gegeben und mit Toluol eluiert.
FR 1-1: 5,5 mg NMR
R_{f}-Wert: DC-Alurollle Kieselgel 60 F₂₅₄, Schichtdicke 0,2 mm = 0,40 (Fließmittel: Petrolether/Essigester 10:1)
R_{f}= 0,45; Fließmittel Toluol / Essigester 10:1

In Analogie zu den oben aufgeführten Vorschriften werden die in der Tabelle 1 aufgeführten Verbindungen hergestellt:

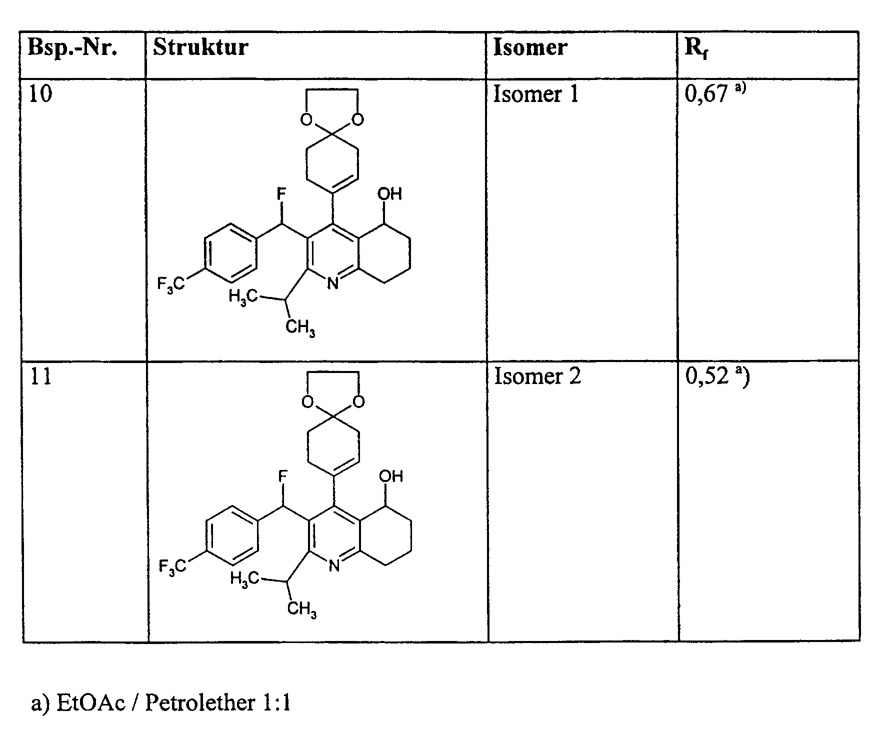

## Patentansprüche

1. Hetero-Tetrahydrochinoline der allgemeinen Formel (I), 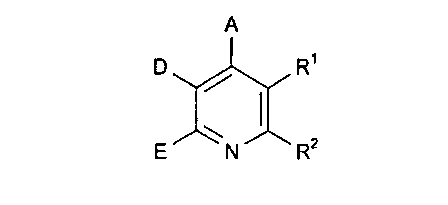 in welcher
A für Cycloalkyl mit 3 bis 8 Kohlenstoffatomen oder
für einen 5- bis 7-gliedrigen, gesättigten, partiell ungesättigten oder ungesättigten, gegebenenfalls benzokondensierten Heterocyclus mit bis zu 3 Heteroatomen aus der Reihe S, N und/oder O steht, der im Fall eines gesättigten Heterocyclus mit einer Stickstoffunktion, gegebenenfalls auch über diese gebunden ist, und wobei die oben aufgeführten Ringsysteme gegebenenfalls bis zu 5-fach gleich oder verschieden durch Halogen, Nitro, Hydroxy, Trifluormethyl, Trifluormethoxy oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert sind,
worin
R³ und R⁴ gleich oder verschieden sind und Wasserstoff, Phenyl oder geradkettiges oder verzweigtes Alkyl mit bis zu 6 Kohlenstoffatomen bedeuten,
oder
A für einen Rest der Formel 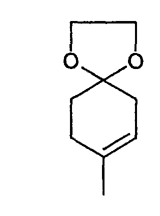 steht,
D für Aryl mit 6 bis 10 Kohlenstoffatomen steht, das gegebenenfalls durch Phenyl, Nitro, Halogen, Trifluormethyl oder Trifluormethoxy substituiert ist, oder
für einen Rest der Formel
R⁵―L― , 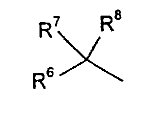
oder
R⁹-T-V―X―
steht,
worin
R⁵, R⁶ und R⁹ unabhängig voneinander Cycloalkyl mit 3 bis 6 Kohlenstoffatomen bedeuten, oder
Aryl mit 6 bis 10 Kohlenstoffatomen bedeuten oder einen 5- bis 7-gliedrigen, gegebenenfalls benzokondensierten, gesättigten oder ungesättigten, mono-, bi- oder tricyclischen Heterocyclus mit bis zu 4 Heteroatomen aus der Reihe S, N und/oder O bedeuten,
wobei die Cyclen, gegebenenfalls, im Fall der stickstoffhaltigen Ringe auch über die N-Funktion, bis zu 5-fach gleich oder verschieden durch Halogen, Trifluormethyl, Nitro, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 6 Kohlenstoffatomen, durch Aryl oder Trifluormethyl substituiertes Aryl mit jeweils 6 bis 10 Kohlenstoffatomen oder durch einen, gegebenenfalls benzokondensierten, aromatischen 5- bis 7-gliedrigen Heterocyclus mit bis zu 3 Heteroatomen aus der Reihe S, N und/oder O substituiert sind,
und/oder durch eine Gruppe der Formel -OR¹⁰, -SR¹¹, -SO₂R¹² oder -NR¹³R¹⁴ substituiert sind,
worin
R¹⁰, R¹¹ und R¹² unabhängig voneinander Aryl mit 6 bis 10 Kohlenstoffatomen bedeuten, das seinerseits bis zu 2-fach gleich oder verschieden durch Phenyl, Halogen oder durch geradkettiges oder verzweigtes Alkyl mit bis zu 6 Kohlenstoffatomen substituiert ist,
R¹³ und R¹⁴ gleich oder verschieden sind und die oben angegebene Bedeutung von R³ und R⁴ haben,
oder
R⁵ und/oder R⁶ einen Rest der Formel 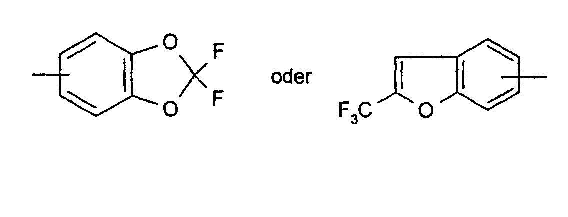 bedeuten,
R⁷ Wasserstoff oder Halogen bedeutet,
und
R⁸ Wasserstoff, Halogen, Azido, Trifluormethyl, Hydroxy, Trifluormethoxy, geradkettiges oder verzweigtes Alkoxy oder Alkyl mit jeweils bis zu 6 Kohlenstoffatomen oder einen Rest der Formel -NR¹⁵R¹⁶ bedeutet,
worin
R¹⁵ und R¹⁶ gleich oder verschieden sind und die oben angegebene Bedeutung von R³ und R⁴ haben,
oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 6 Kohlenstoffatomen bedeutet,
L eine geradkettige oder verzweigte Alkylen- oder Alkenylen-Kette mit jeweils bis zu 8 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 8 Kohlenstoffatomen bedeuten,
oder
T oder X eine Bindung bedeutet,
V für ein Sauerstoff- oder Schwefelatom oder für eine -NR¹⁸-Gruppe steht,
worin
R¹⁸ Wasserstoff oder geradkettiges oder verzweigtes Alkyl mit bis zu 6 Kohlenstoffatomen oder Phenyl bedeutet,
E für Cycloalkyl mit 3 bis 8 Kohlenstoffatomen steht, oder
für geradkettiges oder verzweigtes Alkyl mit bis zu 8 Kohlenstoffatomen steht, das gegebenenfalls durch Cycloalkyl mit 3 bis 8 Kohlenstoffatomen oder Hydroxy substituiert ist, oder für Phenyl steht, das gegebenenfalls durch Halogen oder Trifluormethyl substituiert ist,
R¹ und R² gemeinsam eine geradkettige oder verzweigte Alkylenkette mit bis zu 7 Kohlenstoffatomen bilden, die durch eine Carbonylgruppe und/oder durch einen Rest der Formel 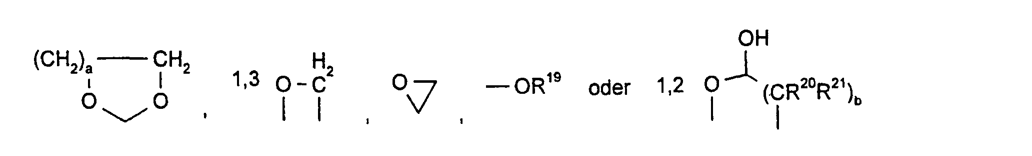 substituiert sein muß,
worin
a und b gleich oder verschieden sind und eine Zahl 1, 2 oder 3 bedeuten,
R¹⁹ Wasserstoff, Cycloalkyl mit 3 bis 7 Kohlenstoffatomen, geradkettiges oder verzweigtes Silylalkyl mit bis zu 8 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes Alkoxy mit bis zu 6 Kohlenstoffatomen oder durch Phenyl substituiert ist, das seinerseits durch Halogen, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 4 Kohlenstoffatomen oder Benzyl bedeutet,
oder
R¹⁹ geradkettiges oder verzweigtes Acyl mit bis zu 20 Kohlenstoffatomen oder Benzoyl bedeutet, das gegebenenfalls durch Halogen, Trifluormethyl, Nitro oder Trifluormethoxy substituiert ist, oder
geradkettiges oder verzweigtes Fluoracyl mit bis zu 8 Kohlenstoffatomen und 9 Fluoratomen bedeutet,
R²⁰ und R²¹ gleich oder verschieden sind, Wasserstoff, Phenyl oder geradkettiges oder verzweigtes Alkyl mit bis zu 6 Kohlenstoffatomen bedeuten,
oder
R²⁰ und R²¹ gemeinsam einen 3 bis 6-gliedrigen Carbocyclus bilden,
und, gegebenenfalls auch geminal, die gebildeten Carbocyclen gegebenenfalls bis zu 6-fach gleich oder verschieden durch Trifluormethyl, Hydroxy, Nitril, Halogen, Carboxyl, Nitro, Azido, Cyano, Cycloalkyl oder Cycloalkyloxy mit jeweils 3 bis 7 Kohlenstoffatomen, durch geradkettiges oder verzweigtes Alkoxycarbonyl, Alkoxy oder Alkylthio mit jeweils bis zu 6 Kohlenstoffatomen oder durch geradkettiges oder verzweigtes Alkyl mit bis zu 6 Kohlenstoffatomen substituiert sind, das seinerseits bis zu 2-fach gleich oder verschieden durch Hydroxyl, Benzyloxy, Trifluormethyl, Benzoyl, geradkettiges oder verzweigtes Alkoxy, Oxyacyl oder Carboxyl mit jeweils bis zu 4 Kohlenstoffatomen und/oder Phenyl substituiert ist, das seinerseits durch Halogen, Trifluormethyl oder Trifluormethoxy substituiert sein kann,
und/oder die gebildeten Carbocyclen, auch geminal, gegebenenfalls bis zu 5-fach gleich oder verschieden durch Phenyl, Benzoyl, Thiophenyl oder Sulfonylbenzyl substituiert sind, die ihrerseits gegebenenfalls durch Halogen, Trifluormethyl, Trifluormethoxy oder Nitro substituiert sind,
und/oder gegebenenfalls durch einen Rest der Formel 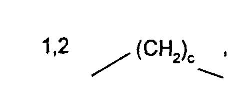 -SO₂-C₆H₅, -(CO)_{d}-NR²³R²⁴ oder =O substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cycloalkyl mit 3 bis 6 Kohlenstoffatomen, geradkettiges oder verzweigtes Alkyl mit bis zu 6 Kohlenstoffatomen, Benzyl oder Phenyl bedeuten, das gegebenenfalls bis zu 2-fach gleich oder verschieden durch Halogen, Trifluormethyl, Cyano, Phenyl oder Nitro substituiert ist,
und/oder die gebildeten Carbocyclen gegebenenfalls durch einen spiro-verknüpften Rest der Formel 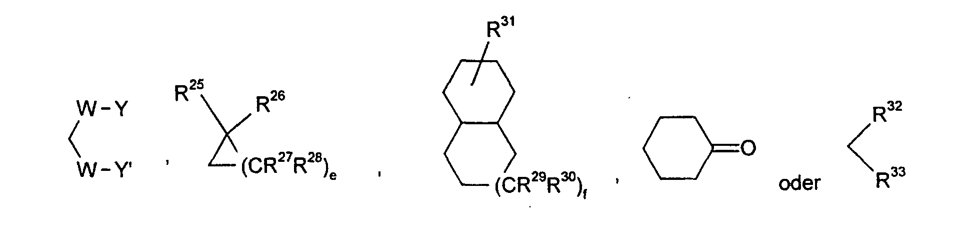 substituiert sind,
worin
W entweder ein Sauerstoff oder ein Schwefelatom bedeutet,
Y und Y' gemeinsam eine 2- bis 6-gliedrige geradkettige oder verzweigte Alkylenkette bilden,
e eine Zahl 1, 2, 3, 4, 5, 6 oder 7 bedeutet,
f eine Zahl 1 oder 2 bedeutet,
R²⁵, R²⁶, R²⁷, R²⁸, R²⁹, R³⁰ und R³¹ gleich oder verschieden sind und Wasserstoff, Trifluormethyl, Phenyl, Halogen oder geradkettiges oder verzweigtes Alkyl oder Alkoxy mit jeweils bis zu 6 Kohlenstoffatomen bedeuten,
oder
R²⁵ und R²⁶ oder R²⁷ und R²⁸ jeweils gemeinsam eine geradkettige oder verzweigte Alkylkette mit bis zu 6 Kohlenstoffatomen bilden,
oder
R²⁵ und R²⁶ oder R²⁷ und R²⁸ jeweils gemeinsam einen Rest der Formel 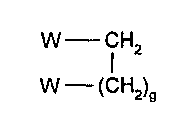 bilden,
worin
W die oben angegebene Bedeutung hat,
g eine Zahl 1, 2, 3, 4, 5, 6 oder 7 bedeutet,
R³² und R³³ gemeinsam einen 3- bis 7-gliedrigen Heterocyclus bilden, der ein Sauerstoff- oder Schwefelatom oder eine Gruppe der Formel SO, SO₂ oder -NR³⁴ enthält,
worin
R³⁴ Wasserstoff, Phenyl, Benzyl oder geradkettiges oder verzweigtes Alkyl mit bis zu 4 Kohlenstoffatomen bedeutet,
und deren Salze und N-Oxide.

2. Hetero-Tetrahydrochinoline der Formel (I) nach Anspruch 1,
in welcher
A für Cyclopropyl, Cyclobutyl, Cyclopentyl, Cycloheptyl, Cyclooctyl oder Cyclohexyl steht, oder
für Thienyl, Imidazolyl, Pyrrol, Furryl, Pyridyl, Morpholin, Pyrimidyl oder Pyridazinyl steht, , die gegebenenfalls bis zu 2-fach gleich oder verschieden durch Fluor, Chlor, Brom, Amino, Hydroxy, Trifluormethyl, Trifluormethoxy oder durch geradkettiges oder verzweigtes Alkyl, oder Alkoxy mit jeweils bis zu 6 Kohlenstoffatomen substituiert sind,
oder
A für einen Rest der Formel 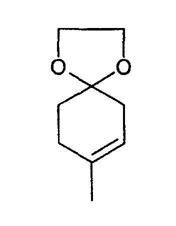 steht,
D für Phenyl steht, das gegebenenfalls durch Nitro, Fluor, Chlor, Brom, Phenyl, Trifluormethyl oder Trifluormethoxy substituiert ist, oder
für einen Rest der Formel
R⁵―L― , 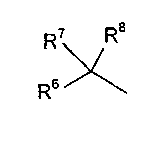
oder
R⁹-T-V―X―
steht,
worin
R⁵, R⁶ und R⁹ unabhängig voneinander Cyclopropyl, Cyclopentyl oder Cyclohexyl bedeuten, oder
Phenyl, Naphthyl, Pyridyl, Tetrazolyl, Pyrimidyl, Pyrazinyl, Pyrrolidinyl, Indolyl, Morpholinyl, Imidazolyl, Benzothiazolyl, Phenoxathiin-2-yl, Benzoxazolyl, Furyl, Chinolyl oder Purin-8-yl bedeuten,
wobei die Cyclen, gegebenenfalls bis zu 3-fach im Fall der stickstoffhaltigen Ringe auch über die N-Funktion, gleich oder verschieden durch Fluor, Chlor, Brom, Trifluormethyl, Hydroxy, Cyano, Carboxyl, Trifluormethoxy, geradkettiges oder verzweigtes Acyl, Alkyl, Alkylthio, Alkylalkoxy, Alkoxy oder Alkoxycarbonyl mit jeweils bis zu 4 Kohlenstoffatomen, Triazolyl, Tetrazolyl, Benzoxathiazolyl, oder Trifluormethyl substituiertes Phenyl oder Phenyl substituiert sind,
oder
R⁷ Wasserstoff, Fluor, Chlor oder Brom bedeutet,
und
R⁸ Wasserstoff, Fluor, Chlor, Brom, Azido, Trifluormethyl, Hydroxy, Trifluormethoxy, geradkettiges oder verzweigtes Alkoxy oder Alkyl mit bis zu jeweils 5 Kohlenstoffatomen oder einen Rest der Formel -NR¹⁵R¹⁶ bedeutet,
worin
R¹⁵ und R¹⁶ gleich oder verschieden sind und Wasserstoff, Phenyl oder geradkettiges oder verzweigtes Alkyl mit bis zu 4 Kohlenstoffatomen bedeuten,
oder
R⁷ und R⁸ gemeinsam einen Rest der Formel =O oder =NR¹⁷ bilden,
worin
R¹⁷ Wasserstoff oder geradkettiges oder verzweigtes Alkyl, Alkoxy oder Acyl mit jeweils bis zu 4 Kohlenstoffatomen bedeutet,
L eine geradkettige oder verzweigte Alkylen- oder Alkenylen-Kette mit jeweils bis zu 6 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 6 Kohlenstoffatomen bedeuten,
oder
T oder X eine Bindung bedeutet,
V für ein Sauerstoff- oder Schwefelatom oder für eine Gruppe der Formel -NR¹⁸- steht,
worin
R¹⁸ Wasserstoff oder geradkettiges oder verzweigtes Alkyl mit bis zu 4 Kohlenstoffatomen oder Phenyl bedeutet,
E für Cyclopropyl, -butyl, -pentyl, -hexyl oder -heptyl steht, oder
für geradkettiges oder verzweigtes Alkyl mit bis zu 6 Kohlenstoffatomen steht, das gegebenenfalls durch Cyclopropyl, -butyl, -hexyl, - pentyl, -heptyl oder durch Hydroxy substituiert ist, oder für Phenyl steht, das gegebenenfalls durch Fluor, Chlor oder Trifluormethyl substituiert ist,
R¹ und R² gemeinsam eine geradkettige oder verzweigte Alkylenkette mit bis zu 6 Kohlenstoffatomen bilden, die durch eine Carboxylgruppe und/oder durch einen Rest der Formel 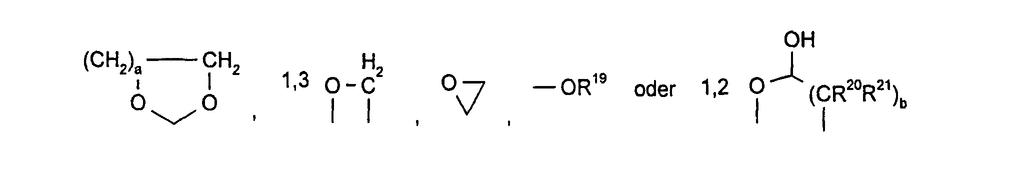 substituiert sein muß,
worin
a und b gleich oder verschieden sind und eine Zahl 1, 2 oder 3 bedeuten,
R¹⁹ Wasserstoff, Cyclopropyl, Cyclopentyl, Cyclohexyl, geradkettiges oder verzweigtes Silylalkyl mit bis zu 7 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy mit bis zu 4 Kohlenstoffatomen oder durch Phenyl substituiert ist, das seinerseits durch Fluor, Chlor, Brom, Nitro, Trifluormethyl, Trifluormethoxy oder durch Phenyl oder Tetrazol substituiertes Phenyl substituiert sein kann,
und Alkyl gegebenenfalls durch eine Gruppe der Formel -OR²² substituiert ist,
worin
R²² geradkettiges oder verzweigtes Acyl mit bis zu 3 Kohlenstoffatomen oder Benzyl bedeutet,
oder
R¹⁹ geradkettiges oder verzweigtes Acyl mit bis zu 18 Kohlenstoffatomen oder Benzoyl bedeutet, das gegebenenfalls durch Fluor, Chlor, Brom, Trifluormethyl, Nitro oder Trifluormethoxy substituiert ist, oder
geradkettiges oder verzweigtes Fluoracyl mit bis zu 6 Kohlenstoffatomen bedeutet,
R²⁰ und R²¹ gleich oder verschieden sind, Wasserstoff, Phenyl oder geradkettiges oder verzweigtes Alkyl mit bis zu 4 Kohlenstoffatomen bedeuten,
oder
R²⁰ und R²¹ gemeinsam einen Cyclpropyl-, Cyclobutyl-, Cyclopentyl-, Cyclohexyl- oder Cycloheptylring bilden,
und die gebildeten Carbocyclen gegebenenfalls bis zu 5-fach gleich oder verschieden, gegebenenfalls auch geminal, durch Trifluormethyl, Hydroxy, Carboxyl, Azido, Fluor, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis ca. 5 Kohlenstoffatomen oder geradkettiges oder verzweigtes Alkyl mit bis zu 5 Kohlenstoffatomen substituiert sind, das seinerseits bis zu 2-fach gleich oder verschieden durch Hydroxyl, Benzyloxy, Benzoyl, geradkettiges oder verzweigtes Alkoxy oder Oxyacyl mit jeweils bis zu 3 Kohlenstoffatomen, Trifluormethyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden durch Phenyl, Benzoyl, Thiophenyl oder Sulfonylbenzyl substituiert sind, die ihrerseits gegebenenfalls durch Fluor, Chlor, Brom, Trifluormethyl, Trifluormethoxy oder Nitro substituiert sind,
und/oder gegebenenfalls durch einen Rest der Formel 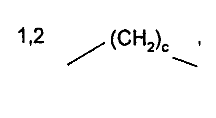 - SO₂-C₆H₅, -(CO)_{d}-NR²³R²⁴ oder =O
substituiert sind,
worin
c eine Zahl 1, 2, 3 oder 4 bedeutet,
d eine Zahl 0 oder 1 bedeutet,
R²³ und R²⁴ gleich oder verschieden sind und Wasserstoff, Cyclopropyl, Cyclobutyl, Cyclopentyl, Cyclohexyl, geradkettiges oder verzweigtes Alkyl mit bis zu 5 Kohlenstoffatomen, Benzyl oder Phenyl bedeuten, das gegebenenfalls durch Fluor, Chlor, Brom, Phenyl oder Trifluormethyl substituiert ist,
und/oder die gebildeten Carbocyclen gegebenenfalls durch einen spiro-verknüpften Rest der Formel 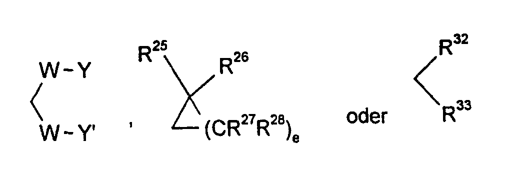 substituiert sind,
worin
W entweder ein Sauerstoff oder ein Schwefelatom bedeutet,
Y und Y' gemeinsam eine 2- bis 5-gliedrige geradkettige oder verzweigte Alkylkette bilden,
e eine Zahl 1, 2, 3, 4, 5 oder 6 bedeutet,
f eine Zahl 1 oder 2 bedeutet,
R²⁵, R²⁶, R²⁷ und R²⁸ gleich oder verschieden sind und Wasserstoff, Trifluormethyl, Phenyl, Fluor, Chlor, Brom oder geradkettiges oder verzweigtes Alkyl oder Alkoxy mit jeweils bis zu 5 Kohlenstoffatomen bedeuten,
oder
R²⁵ und R²⁶ oder R²⁷ und R²⁸ jeweils gemeinsam eine geradkettige oder verzweigte Alkylkette mit bis zu 5 Kohlenstoffatomen bilden oder
oder
R²⁵ und R²⁶ oder R²⁷ und R²⁸ jeweils gemeinsam einen Rest der Formel 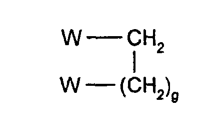 bilden,
worin
W die oben angegebene Bedeutung hat,
g eine Zahl 1, 2, 3, 4, 5 oder 6 bedeutet,
und deren Salze und N-Oxide.

3. Hetero-Tetrahydrochinoline der Formel (I) nach Anspruch 1,
in welcher
A für Cyclopropyl, Cyclopentyl oder Cyclohexyl steht, oder
für Thienyl oder Pyridyl steht, die gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert sind,
oder
A für einen Rest der Formel 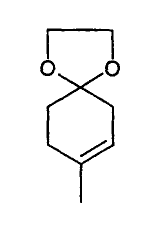 steht,
D für Phenyl steht, das gegebenenfalls durch Nitro, Trifluormethyl, Phenyl, Fluor, Chlor oder Brom substituiert ist, oder
für einen Rest der Formel
R⁵―L― , 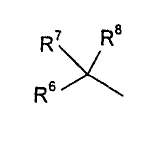
oder
R⁹―T-V―X―
steht,
worin
R⁵, R⁶ und R⁹ unabhängig voneinander Cyclopropyl, Cyclopentyl oder Cyclohexyl bedeuten, oder
Phenyl, Naphthyl oder Pyridyl bedeuten,
wobei die Cyclen, gegebenenfalls bis zu 2-fach, gleich oder verschieden durch Fluor, Chlor, Trifluormethyl, Hydroxy, Cyano, Carboxyl, Trifluormethoxy, geradkettiges oder verzweigtes Alkyl, Alkylthio, Alkylalkoxy, Alkoxy oder Alkoxycarbonyl mit jeweils bis zu 4 Kohlenstoffatomen,
oder
R⁷ Wasserstoff oder Fluor bedeutet,
und
R⁸ Wasserstoff, Fluor, Chlor, Brom, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten,
oder
R⁷ und R⁸ gemeinsam für einen Rest der Formel =O stehen,
L eine geradkettige oder verzweigte Alkylen- oder Alkenylen-Kette mit jeweils bis zu 5 Kohlenstoffatomen bedeutet, die gegebenenfalls bis zu 2-fach durch Hydroxy substituiert sind,
T und X gleich oder verschieden sind und eine geradkettige oder verzweigte Alkylenkette mit bis zu 3 Kohlenstoffatomen bedeuten,
oder
T oder X eine Bindung bedeutet,
V für ein Sauerstoff- oder Schwefelatom oder für eine Gruppe der Formel -NR¹⁸ steht,
worin
R¹⁸ Wasserstoff oder geradkettiges oder verzweigtes Alkyl mit bis zu 3 Kohlenstoffatomen bedeutet,
E für Cyclopropyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
R¹ und R² gemeinsam eine geradkettige oder verzweigte Alkylenkette mit bis zu 5 Kohlenstoffatomen bilden, die durch eine Carbonylgruppe und/oder einen Rest der Formel -OR¹⁹ substituiert sein muß,
worin
R¹⁹ Wasserstoff, Cyclopropyl, Cyclopentyl oder Cyclohexyl bedeutet,
oder
R¹⁹ geradkettiges oder verzweigtes Acyl mit bis zu 15 Kohlenstoffatomen oder Benzoyl bedeutet, das gegebenenfalls durch Fluor, Chlor, Brom, Trifluormethyl, Nitro oder Trifluormethoxy substituiert ist, oder
einen Rest der Formel -Si(CH₃)₂C(CH₃)₃ bedeutet,
und die gebildeten Carbocyclen gegebenenfalls bis zu 4-fach gleich oder verschieden, gegebenenfalls auch geminal, durch Fluor, Hydroxyl, Trifluormethyl, Carboxyl, Azido, Chlor, Brom, Nitro, Cyano, Cyclopropyl, Cyclobutyl, Cyclopentyl, Cyclohexyl, Cyclopropyloxy, Cyclopentyloxy, Cyclohexyloxy, durch geradkettiges oder verzweigtes Alkoxycarbonyl, Alkoxy oder Alkylthio mit jeweils bis zu 4 Kohlenstoffatomen oder geradkettiges oder verzweigtes Alkyl mit bis zu 4 Kohlenstoffatomen substituiert sind, das seinerseits bis zu 2-fach gleich oder verschieden durch Hydroxyl, Benzyloxy, Trifluormethyl, Benzoyl, Methoxy, Oxyacetyl und/oder Phenyl substituiert ist, das seinerseits durch Fluor, Chlor, Brom, Trifluormethyl oder Trifluormethoxy substituiert sein kann,
und/oder die gebildeten Carbocyclen, auch geminal, gegebenenfalls bis zu 4-fach gleich oder verschieden, durch Phenyl, Benzoyl, Thiophenyl oder Sulfonylbenzyl substituiert sind, die ihrerseits gegebenenfalls durch Fluor, Trifluormethyl, Trifluormethoxy oder Nitro substituiert sind,
und/oder gegebenenfalls durch einen Rest der Formel 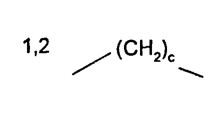 oder =O substituiert sind,
worin
c eine Zahl 1, 2, 3 oder 4 bedeutet,
und/oder die gebildeten Carbocyclen gegebenenfalls durch einen spiro-verknüpften Rest der Formel 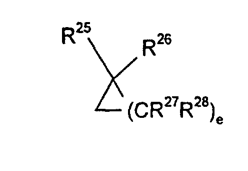 substituiert sind,
worin
e eine Zahl 1, 2, 3, 4 oder 5 bedeutet,
R²⁵, R²⁶, R²⁷ und R²⁸ gleich oder verschieden sind und Wasserstoff, Trifluormethyl, Phenyl, Fluor, Chlor, Brom oder geradkettiges oder verzweigtes Alkyl oder Alkoxy mit jeweils bis zu 4 Kohlenstoffatomen bedeuten,
oder
R²⁵ und R²⁶ oder R²⁷ und R²⁸ gemeinsam eine geradkettige oder verzweigte Alkylkette mit bis zu 4 Kohlenstoffatomen bilden,
und deren Salze und N-Oxide.

4. Hetero-Tetrahydrochinoline der Formel (I) nach Anspruch 1,
in welcher
A für Cyclopropyl, Cyclopentyl oder Cyclohexyl steht, oder
für Thienyl oder Pyridyl steht, oder A für einen Rest der Formel 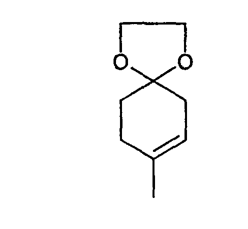 steht,
D für Phenyl steht, das gegebenenfalls durch Trifluormethyl, Fluor, substituiert ist, oder
für einen Rest der Formel 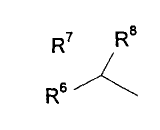 steht,
worin
R⁶ Phenyl bedeutet, das gegebenenfalls durch Fluor, Chlor, Trifluormethyl, Trifluormethoxy, geradkettiges oder verzweigtes Alkyl mit jeweils bis zu 4 Kohlenstoffatomen substituiert ist,
oder
R⁷ Wasserstoff oder Fluor bedeutet,
und
R⁸ Wasserstoff, Fluor, Chlor, Hydroxy, Methoxy oder
R⁷ und R⁸ gemeinsam für einen Rest der Formel =O stehen,
E für Cyclopropyl, Cyclopentyl oder Cyclohexyl
für geradkettiges oder verzweigtes Alkyl mit bis zu 4 Kohlenstoffatomen steht,
R¹ und R² gemeinsam eine geradkettige oder verzweigte Alkylenkette mit bis zu 5 Kohlenstoffatomen bilden, die durch eine Carbonylgruppe und/oder einen Rest der Formel -OR¹⁹ substituiert sein muß,
worin
R¹⁹ Wasserstoff bedeutet,
oder einen Rest der Formel -Si(CH₃)₂C(CH₃)₃ bedeutet,
und deren Salze und N-Oxide.

5. Hetero-Tetrahydrochinoline nach Anspruch 1 bis 4 als Arzneimittel.

6. Verfahren zur Herstellung von Hetero-Tetrahydrochinolinen nach Anspruch 1, **dadurch gekennzeichnet, daß** man
[A] im Fall D ≠ Aryl, Verbindungen der allgemeinen Formel (II) 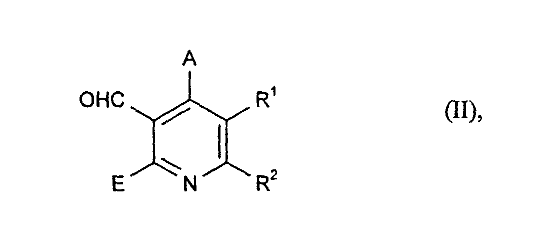 in welcher
A, E, R¹ und R² die oben angegebene Bedeutung haben,
mit metallorganischen Reagenzien im Sinne einer Grignard-, Wittig- oder Lithium-organischen-Reaktion den Substituenten D in inerten Lösemitteln synthetisiert,
oder im Fall, daß D für den Rest der Formel R⁹-T-V-X steht, in welcher V ein Sauerstoffatom bedeutet,
[B] entweder Verbindungen der allgemeinen Formel (III) 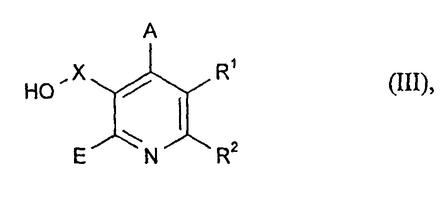 in welcher
A, E, X, R¹ und R² die oben angegebene Bedeutung haben,
mit Verbindungen der allgemeinen Formel (IV)
R⁹-T-Z (IV),
in welcher
R⁹ und T die oben angegebene Bedeutung haben
und
Z für Halogen, vorzugsweise Chlor oder Brom, steht,
in inerten Lösemitteln, gegebenenfalls in Anwesenheit einer Base und/oder Hilfsstoffs umsetzt,
oder
[C] Verbindungen der allgemeinen Formel (III) zunächst durch Umsetzung mit Verbindungen der allgemeinen Formel (V) 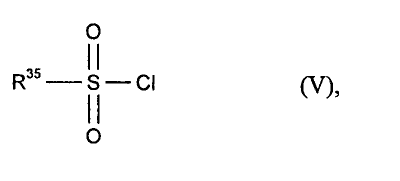 in welcher
R³⁵ für geradkettiges Alkyl mit bis zu 4 Kohlenstoffatomen steht,
in die Verbindungen der allgemeinen Formel (VI) 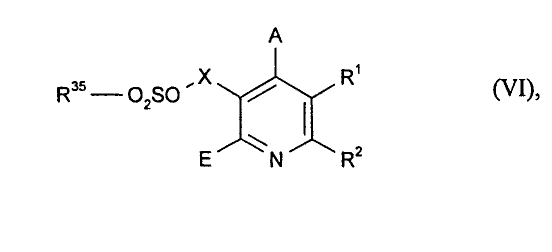 in welcher
A, E, X, R¹, R² und R³⁵ die oben angegebene Bedeutung haben,
überführt und anschließend mit Verbindungen der allgemeinen Formel (VII)
R⁹-T-V-H (VII),
in welcher
R⁹, T und V die oben angegebene Bedeutung haben,
umsetzt und gegebenenfalls Schutzgruppen abspaltet,
oder
[D] im Fall der Verbindungen der allgemeinen Formel (Ia) 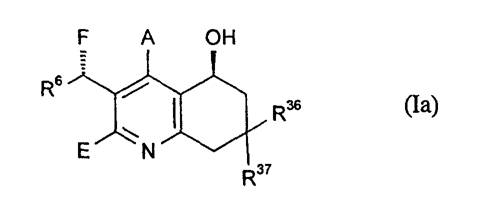 in welcher
A und R⁶ die oben angegebene Bedeutung haben,
R³⁶ und R³⁷ gleich oder verschieden sind und
für Cycloalkyl oder Cycloalkyloxy mit jeweils 3 bis 7 Kohlenstoffatomen stehen, oder
für geradkettiges oder verzweigtes Alkyl mit bis zu 6 Kohlenstoffatomen stehen, oder
für Phenyl stehen, das seinerseits gegebenenfalls durch Halogen, Trifluormethyl, Trifluormethoxy oder Nitro substituiert sind, oder
R³⁶ und R³⁷ für einen der oben aufgeführten spiro-verknüpften Reste der Formel 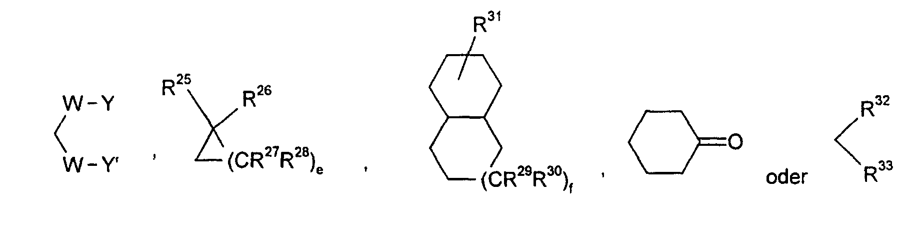 stehen,
worin
W, Y, Y', R²⁵, R²⁶, R²⁷, R²⁸, e, R²⁹, R³⁰, R³¹, R³² und R³³ die oben angegebene Bedeutung haben,
Verbindungen der allgemeinen Formel (VIII) 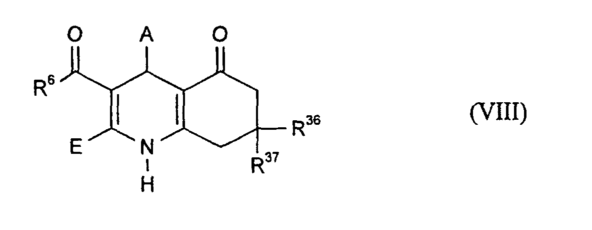 in welcher
R⁶, R³⁶, R³⁷, A und E die oben angegebene Bedeutung haben,
zunächst zu den Verbindungen der allgemeinen Formel (IX) 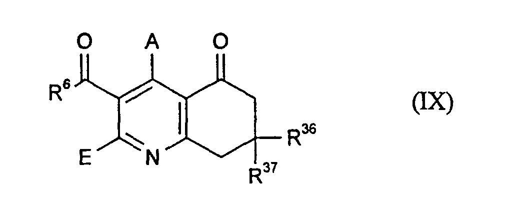 in welcher
R⁶, R³⁶, R³⁷, A und E die oben angegebene Bedeutung haben,
oxidiert,
diese in einem nächsten Schritt durch eine asymmetrische Reduktion zu den Verbindungen der allgemeinen Formel (X) 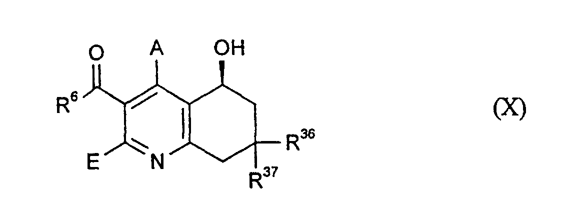 in welcher
R⁶, R³⁶, R³⁷, A und E die oben angegebene Bedeutung haben,
umsetzt,
diese dann durch die Einführung einer Hydroxyschutzgruppe in die Verbindungen der allgemeinen Formel (XI) 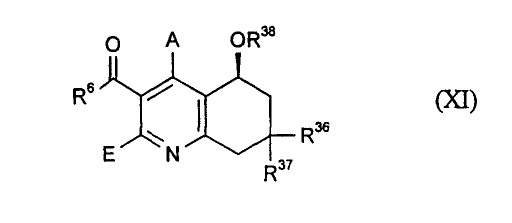 in welcher
R⁶, R³⁶, R³⁷, A und E die oben angegebene Bedeutung haben
und
R³⁸ für eine Hydroxyschutzgruppe, vorzugsweise für einen Rest der Formel -SiR³⁹R⁴⁰R⁴¹ steht,
worin
R³⁹, R⁴⁰ und R⁴¹ gleich oder verschieden sind und C₁-C₄-Alkyl bedeuten,
überführt,
aus diesem in einem Folgeschritt durch diastereoselektive Reduktion die Verbindungen der allgemeinen Formel (XII) 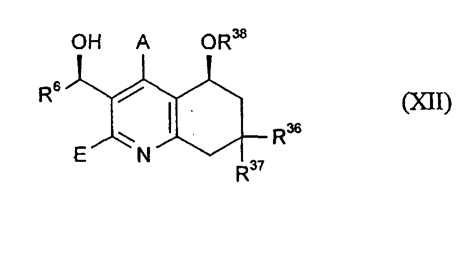 in welcher
R⁶, R³⁶, R³⁷, R³⁸, A und E die oben angegebene Bedeutung haben,
herstellt,
und anschließend durch Einführung des Fluorsubstituenten mit Fluorierungsreagentien, wie z.B. DAST und SF₄-Derivaten die Verbindungen der allgemeinen Formel (XIII) 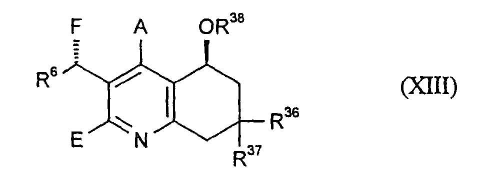 in welcher
R⁶, R³⁶, R³⁷, R³⁸, A und E die oben angegebene Bedeutung haben,
herstellt,
und anschließend die Hydroxyschutzgruppe nach üblichen Methoden abspaltet,
und gegebenenfalls die unter D, E und/oder R¹ und R² aufgeführten Substituenten nach üblichen Methoden variiert oder einführt.

7. Arzneimittel enthaltend mindestens ein Hetero-Tetrahydrochinolin nach Anspruch 1 sowie pharmakologisch verträgliche Formulierungshilfsmittel.

8. Arzneimittel nach Anspruch 7 zur Behandlung von Hyperlipoproteinämie.

9. Arzneimittel nach Anspruch 7 zur Behandlung von Arteriosklerose.

10. Verwendung von Hetero-Tetrahydrochinolinen nach Anspruch 1 zur Herstellung von Arzneimitteln.

11. Verwendung nach Anspruch 10 zur Herstellung von Arzneimitteln zur Behandlung von Arteriosklerose, insbesondere Dyslipidämien.

## Claims

1. Hetero-tetrahydroquinolines of the general formula (I), 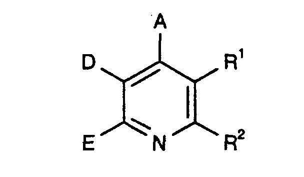 in which
A represents cycloalkyl having 3 to 8 carbon atoms or
represents a 5- to 7-membered saturated, partially unsaturated or unsaturated, optionally benzo-fused heterocycle having up to 3 heteroatoms from the group consisting of S, N and O which, in the case of a saturated heterocycle with a nitrogen function, is optionally also attached via this function, and where the abovementioned ring systems are optionally substituted up to 5 times by identical or different substituents from the group consisting of halogen, nitro, hydroxyl, trifluoromethyl, trifluoromethoxy and straight-chain or branched alkyl, acyl, hydroxyalkyl or alkoxy having in each case up to 7 carbon atoms, or by a group of the formula -NR³R⁴,
in which
R³ and R⁴ are identical or different and represent hydrogen, phenyl or straight-chain or branched alkyl having up to 6 carbon atoms,
or
A represents a radical of the formula 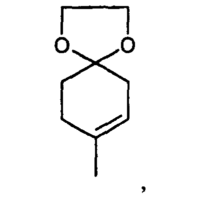
D represents aryl having 6 to 10 carbon atoms which is optionally substituted by phenyl, nitro, halogen, trifluoromethyl or trifluoromethoxy, or
represents a radical of the formula
R⁵―L― , 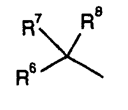
or
R⁹-T-V―X― ,
in which
R⁵, R⁶ and R⁹ independently of one another represent cycloalkyl having 3 to 6 carbon atoms, or
represent aryl having 6 to 10 carbon atoms or represent a 5- to 7-membered optionally benzo-fused saturated or unsaturated mono-, bi- or tricyclic hetreocycle having up to 4 heteroatoms from the group consisting of S, N and O,
where the cycles are optionally substituted, in the case of the nitrogen-containing rings also via the N function, up to 5 times by identical or different substituents from the group consisting of halogen, trifluoromethyl, nitro, hydroxyl, cyano, carboxyl, trifluoromethoxy, and straight-chain or branched acyl, alkyl, alkylthio, alkylalkoxy, alkoxy or alkoxycarbonyl having in each case up to 6 carbon atoms, by aryl or trifluoromethyl-substituted aryl having in each case 6 to 10 carbon atoms or by an optionally benzo-fused aromatic 5- to 7-membered heterocycle having up to 3 heteroatoms from the group consisting of S, N and O,
and/or by a group of the formula -OR¹⁰, -SR¹¹, -SO₂R¹² or -NR¹³R¹⁴,
in which
R¹⁰, R¹¹ and R¹² independently of one another represent aryl having 6 to 10 carbon atoms which for its part is substituted up to 2 times by identical or different substituents from the group consisting of phenyl, halogen and straight-chain or branched alkyl having up to 6 carbon atoms,
R¹³ and R¹⁴ are identical or different and have the meaning of R³ and R⁴ given above,
or
R⁵ and/or R⁶ represent(s) a radical of the formula 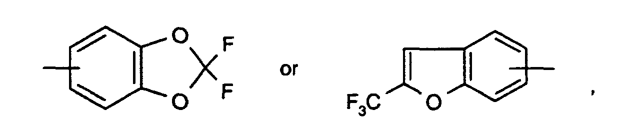
R⁷ represents hydrogen or halogen,
and
R⁸ represents hydrogen, halogen, azido, trifluoromethyl, hydroxyl, trifluoromethoxy, straight-chain or branched alkoxy or alkyl having in each case up to 6 carbon atoms or a radical of the formula -NR¹⁵R¹⁶,
in which
R¹⁵ and R¹⁶ are identical or different and have the meaning of R³ and R⁴ given above,
or
R⁷ and R⁸ together form a radical of the formula =O or =NR¹⁷,
in which
R¹⁷ represents hydrogen or straight-chain or branched alkyl, alkoxy or acyl having in each case up to 6 carbon atoms,
L represents a straight-chain or branched alkylene or alkenylene chain having in each case up to 8 carbon atoms which are optionally substituted up to 2 times by hydroxyl,
T and X are identical or different and represent a straight-chain or branched alkylene chain having up to 8 carbon atoms,
or
T or X represents a bond,
V represents an oxygen or sulphur atom or represents an -NR¹⁸ group,
in which
R¹⁸ represents hydrogen or straight-chain or branched alkyl having up to 6 carbon atoms or phenyl,
E represents cycloalkyl having 3 to 8 carbon atoms, or
represents straight-chain or branched alkyl having up to 8 carbon atoms which is optionally substituted by cycloalkyl having 3 to 8 carbon atoms or hydroxyl, or represents phenyl which is optionally substituted by halogen or trifluoromethyl,
R¹ and R² together form a straight-chain or branched alkylene chain having up to 7 carbon atoms which has to be substituted by a carbonyl group and/or by a radical of the formula 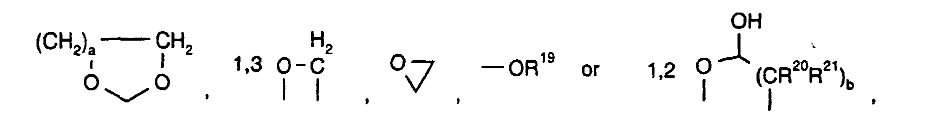 in which
a and b are identical or different and represent a number 1,2 or 3,
R¹⁹ represents hydrogen, cycloalkyl having 3 to 7 carbon atoms, straight-chain or branched silylalkyl having up to 8 carbon atoms or straight-chain or branched alkyl having up to 8 carbon atoms which is optionally substituted by hydroxyl, straight-chain or branched alkoxy having up to 6 carbon atoms or by phenyl which for its part may be substituted by halogen, nitro, trifluoromethyl, trifluoromethoxy or by phenyl or tetrazole-substituted phenyl,
where alkyl is optionally substituted by a group of the formula - OR²²,
in which
R²² represents straight-chain or branched acyl having up to 4 carbon atoms or benzyl,
or
R¹⁹ represents straight-chain or branched acyl having up to 20 carbon atoms or benzoyl which is optionally substituted by halogen, trifluoromethyl, nitro or trifluoromethoxy, or represents straight-chain or branched fluoroacyl having up to 8 carbon atoms and 9 fluorine atoms,
R²⁰ and R²¹ are identical or different, and represent hydrogen, phenyl or straight-chain or branched alkyl having up to 6 carbon atoms,
or
R²⁰ and R²¹ together form a 3- to 6-membered carbocycle
and, if appropriate also geminally, the carbocycles formed are optionally substituted up to 6 times by identical or different substituents from the group consisting of trifluoromethyl, hydroxyl, nitrile, halogen, carboxyl, nitro, azido, cyano, cycloalkyl or cycloalkyloxy having in each case 3 to 7 carbon atoms, straight-chain or branched alkoxycarbonyl, alkoxy or alkylthio having in each case up to 6 carbon atoms and straight-chain or branched alkyl having up to 6 carbon atoms which for its part is substituted up to 2 times by identical or different substituents from the group consisting of hydroxyl, benzyloxy, trifluoromethyl, benzoyl, straight-chain or branched alkoxy, oxyacyl or carboxyl having in each case up to 4 carbon atoms and phenyl which for its part may be substituted by halogen, trifluoromethyl or trifluoromethoxy,
and/or the carbocycles formed are optionally substituted, also geminally, up to 5 times by identical or different substituents from the group consisting of phenyl, benzoyl, thiophenyl and sulphonylbenzyl which for their part are optionally substituted by halogen, trifluoromethyl, trifluoromethoxy or nitro,
and/or are optionally substituted by a radical of the formula 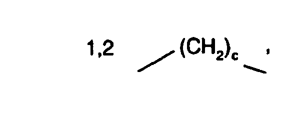 -SO₂-C₆H₅, -(CO)_{d}-NR²³R²⁴ or =O,
in which
c represents a number 1, 2, 3 or 4,
d represents a number 0 or 1,
R²³ and R²⁴ are identical or different and represent hydrogen, cycloalkyl having 3 to 6 carbon atoms, straight-chain or branched alkyl having up to 6 carbon atoms, benzyl or phenyl which is optionally substituted up to 2 times by identical or different substituents from the group consisting of halogen, trifluoromethyl, cyano, phenyl and nitro,
and/or the carbocycles formed are optionally substituted by a spiro-linked radical of the formula 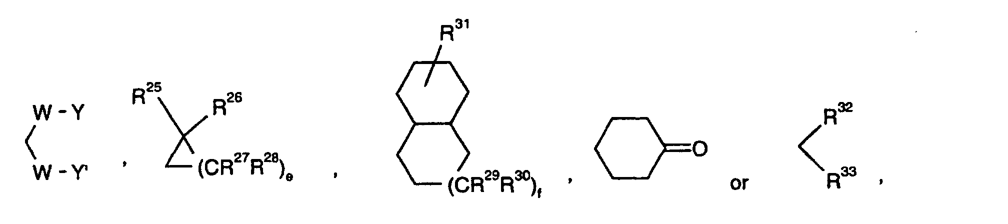 in which
W represents either an oxygen or a sulphur atom,
Y and Y' together form a 2- to 6-membered straight-chain or branched alkylene chain,
e represents a number 1, 2, 3, 4, 5, 6 or 7,
f represents a number 1 or 2,
R²⁵, R²⁶, R²⁷, R²⁸, R²⁹, R³⁰ and R³¹ are identical or different and represent hydrogen, trifluoromethyl, phenyl, halogen or straight-chain or branched alkyl or alkoxy having in each case up to 6 carbon atoms,
or
R²⁵ and R²⁶ or R²⁷ and R²⁸ in each case together form a straight-chain or branched alkyl chain having up to 6 carbon atoms,
or
R²⁵ and R²⁶ or R²⁷ and R²⁸ in each case together form a radical of the formula 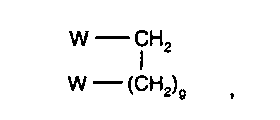 in which
W is as defined above,
g represents a number 1, 2, 3, 4, 5, 6 or 7,
R³² and R³³ together form a 3- to 7-membered heterocycle which contains an oxygen or sulphur atom or a group of the formula SO, SO₂ or -NR³⁴,
in which
R³⁴ represents hydrogen, phenyl, benzyl or straight-chain or branched alkyl having up to 4 carbon atoms,
and their salts and N-oxides.

2. Hetero-tetrahydroquinolines of the formula (I) according to Claim 1, in which
A represents cyclopropyl, cyclobutyl, cyclopentyl, cycloheptyl, cyclooctyl or cyclohexyl, or
represents thienyl, imidazolyl, pyrrole, furryl, pyridyl, morpholine, pyrimidyl or pyridazinyl, which are optionally substituted up to 2 times by identical or different substituents from the group consisting of fluorine, chlorine, bromine, amino, hydroxyl, trifluoromethyl, trifluoromethoxy and straight-chain or branched alkyl, and alkoxy having in each case up to 6 carbon atoms,
or
A represents a radical of the formula 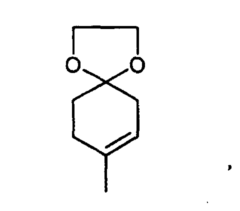
D represents phenyl which is optionally substituted by nitro, fluorine, chlorine, bromine, phenyl, trifluoromethyl or trifluoromethoxy, or
represents a radical of the formula
R⁵―L― , 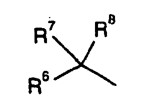
or
R⁹-T-V―X― ,
in which
R⁵, R⁶ and R⁹ independently of one another represent cyclopropyl, cyclopentyl or cyclohexyl, or
represent phenyl, naphthyl, pyridyl, tetrazolyl, pyrimidyl, pyrazinyl, pyrrolidinyl, indolyl, morpholinyl, imidazolyl, benzothiazolyl, phenoxathiin-2-yl, benzoxazolyl, furyl, quinolyl or purin-8-yl,
where the cycles are optionally substituted up to 3 times, in the case of the nitrogen-containing rings also via the N function, by identical or different substituents from the group consisting of fluorine, chlorine, bromine, trifluoromethyl, hydroxyl, cyano, carboxyl, trifluoromethoxy, straight-chain or branched acyl, alkyl, alkylthio, alkylalkoxy, alkoxy or alkoxycarbonyl having in each case up to 4 carbon atoms, triazolyl, tetrazolyl, benzoxathiazolyl, trifluoromethyl-substituted phenyl and phenyl,
or
R⁷ represents hydrogen, fluorine, chlorine or bromine,
and
R⁸ represents hydrogen, fluorine, chlorine, bromine, azido, trifluoromethyl, hydroxyl, trifluoromethoxy, straight-chain or branched alkoxy or alkyl having in each case up to 5 carbon atoms or a radical of the formula -NR¹⁵R¹⁶,
in which
R¹⁵ and R¹⁶ are identical or different and represent hydrogen, phenyl or straight-chain or branched alkyl having up to 4 carbon atoms,
or
R⁷ and R⁸ together form a radical of the formula =O or =NR¹⁷,
in which
R¹⁷ represents hydrogen or straight-chain or branched alkyl, alkoxy or acyl having in each case up to 4 carbon atoms,
L represents a straight-chain or branched alkylene or alkenylene chain having in each case up to 6 carbon atoms which are optionally substituted up to 2 times by hydroxyl,
T and X are identical or different and represent a straight-chain or branched alkylene chain having up to 6 carbon atoms,
or
T or X represents a bond,
V represents an oxygen or sulphur atom or represents a group of the formula -NR¹⁸-,
in which
R¹⁸ represents hydrogen or straight-chain or branched alkyl having up to 4 carbon atoms or phenyl,
E represents cyclopropyl, -butyl, -pentyl, -hexyl or -heptyl, or
represents straight-chain or branched alkyl having up to 6 carbon atoms which is optionally substituted by cyclopropyl, -butyl, -hexyl, -pentyl, -heptyl or by hydroxyl, or represents phenyl which is optionally substituted by fluorine, chlorine or trifluoromethyl,
R¹ and R² together form a straight-chain or branched alkylene chain having up to 6 carbon atoms which has to be substituted by a carboxyl group and/or by a radical of the formula 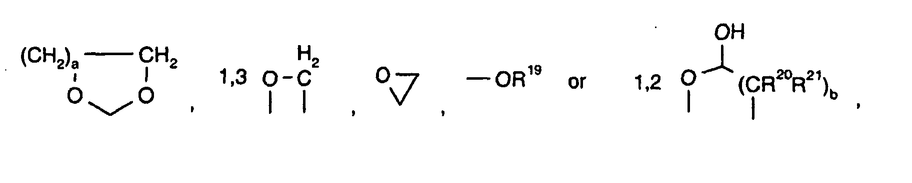 in which
a and b are identical or different and represent a number 1, 2 or 3,
R¹⁹ represents hydrogen, cyclopropyl, cyclopentyl, cyclohexyl, straight-chain or branched silylalkyl having up to 7 carbon atoms or straight-chain or branched alkyl having up to 6 carbon atoms which is optionally substituted by hydroxyl, straight-chain or branched alkoxy having up to 4 carbon atoms or by phenyl which for its part may be substituted by fluorine, chlorine, bromine, nitro, trifluoromethyl, trifluoromethoxy or by phenyl or tetrazole-substituted phenyl,
where alkyl is optionally substituted by a group of the formula -OR²²,
in which
R²² represents straight-chain or branched acyl having up to 3 carbon atoms or benzyl,
or
R¹⁹ represents straight-chain or branched acyl having up to 18 carbon atoms or benzoyl which is optionally substituted by fluorine, chlorine, bromine, trifluoromethyl, nitro or trifluoromethoxy, or
represents straight-chain or branched fluoroacyl having up to 6 carbon atoms,
R²⁰ and R²¹ are identical or different, and represent hydrogen, phenyl or straight-chain or branched alkyl having up to 4 carbon atoms,
or
R²⁰ and R²¹ together form a cyclpropyl, cyclobutyl, cyclopentyl, cyclohexyl or cycloheptyl ring,
and the carbocycles formed are optionally substituted, if appropriate, also geminally, up to 5 times by identical or different substituents from the group consisting of trifluoromethyl, hydroxyl, carboxyl, azido, fluorine, chlorine, bromine, nitro, cyano, cyclopropyl, cyclobutyl, cyclopentyl, cyclohexyl, cyclopropyloxy, cyclopentyloxy, cyclohexyloxy, straight-chain or branched alkoxycarbonyl, alkoxy or alkylthio having in each case up to about 5 carbon atoms and straight-chain or branched alkyl having up to 5 carbon atoms which for its part is substituted up to 2 times by identical or different substituents from the group consisting of hydroxyl, benzyloxy, benzoyl, straight-chain or branched alkoxy or oxyacyl having in each case up to 3 carbon atoms, trifluoromethyl and phenyl which for its part may be substituted by fluorine, chlorine, bromine, trifluoromethyl or trifluoromethoxy,
and/or the carbocycles formed are optionally substituted, also geminally, up to 4 times by identical or different substituents from the group consisting of phenyl, benzoyl, thiophenyl and sulphonylbenzyl which for their part are optionally substituted by fluorine, chlorine, bromine, trifluoromethyl, trifluoromethoxy or nitro,
and/or are optionally substituted by a radical of the formula 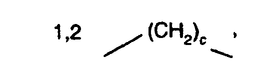 -SO₂-C₆H₅, -(CO)_{d}-NR²³R²⁴ or =O,
in which
c represents a number 1, 2, 3 or 4,
d represents a number 0 or 1,
R²³ and R²⁴ are identical or different and represent hydrogen, cyclopropyl, cyclobutyl, cyclopentyl, cyclohexyl, straight-chain or branched alkyl having up to 5 carbon atoms, benzyl or phenyl which is optionally substituted by fluorine, chlorine, bromine, phenyl or trifluoromethyl,
and/or the carbocycles formed are optionally substituted by a spire-linked radical of the formula 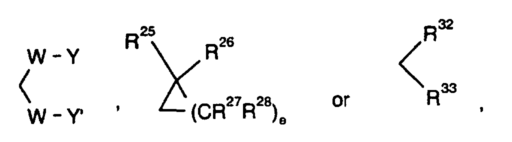 in which
W represents either an oxygen or a sulphur atom,
Y and Y' together form a 2- to 5-membered straight-chain or branched alkyl chain,
e represents a number 1, 2, 3, 4, 5 or 6,
f represents a number 1 or 2,
R²⁵, R²⁶, R²⁷ and R²⁸ are identical or different and represent hydrogen, trifluoromethyl, phenyl, fluorine, chlorine, bromine or straight-chain or branched alkyl or alkoxy having in each case up to 5 carbon atoms,
or
R²⁵ and R²⁶ or R²⁷ and R²⁸ in each case together form a straight-chain or branched alkyl chain having up to 5 carbon atoms
or
R²⁵ and R²⁶ or R²⁷ and R²⁸ in each case together form a radical of the formula 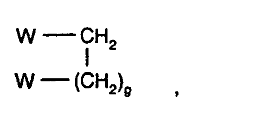 in which
W is as defined above,
g represents a number 1, 2, 3, 4, 5 or 6,
and their salts and N-oxides.

3. Hetero-tetrahydroquinolines of the formula (I) according to Claim 1,
in which
A represents cyclopropyl, cyclopentyl or cyclohexyl, or
represents thienyl or pyridyl which are optionally substituted up to 2 times by identical or different substituents from the group consisting of fluorine, chlorine, bromine, hydroxyl, trifluoromethyl, trifluoromethoxy and straight-chain or branched alkyl or alkoxy having in each case up to 5 carbon atoms,
or
A represents a radical of the formula 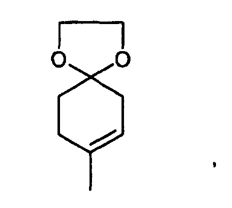
D represents phenyl which is optionally substituted by nitro, trifluoromethyl, phenyl, fluorine, chlorine or bromine, or
represents a radical of the formula
R⁵―L― , 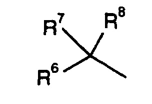
or
R⁹-T-V―X― ,
in which
R⁵, R⁶ and R⁹ independently of one another represent cyclopropyl, cyclopentyl or cyclohexyl, or
represent phenyl, naphthyl or pyridyl,
where the cycles may be substituted optionally up to 2 times, by identical or different substituents from the group consisting of fluorine, chlorine, trifluoromethyl, hydroxyl, cyano, carboxyl, trifluoromethoxy and straight-chain or branched alkyl, alkylthio, alkylalkoxy, alkoxy or alkoxycarbonyl having in each case up to 4 carbon atoms,
or
R⁷ represents hydrogen or fluorine,
and
R⁸ represents hydrogen, fluorine, chlorine, bromine, azido, trifluoromethyl, hydroxyl, trifluoromethoxy, or straight-chain or branched alkoxy or alkyl having in each case up to 4 carbon atoms or a radical of the formula -NR¹⁵R¹⁶,
in which
R¹⁵ and R¹⁶ are identical or different and represent hydrogen or straight-chain or branched alkyl having up to 3 carbon atoms,
or
R⁷ and R⁸ together represent a radical of the formula =O,
L represents a straight-chain or branched alkylene or alkenylene chain having in each case up to 5 carbon atoms which are optionally substituted up to 2 times by hydroxyl,
T and X are identical or different and represent a straight-chain or branched alkylene chain having up to 3 carbon atoms,
or
T or X represents a bond,
V represents an oxygen or sulphur atom or a group of the formula -NR¹⁸,
in which
R¹⁸ represents hydrogen or straight-chain or branched alkyl having up to 3 carbon atoms,
E represents cyclopropyl, cyclopentyl or cyclohexyl or phenyl which is optionally substituted by fluorine or trifluoromethyl, or
represents straight-chain or branched alkyl having up to 4 carbon atoms which is optionally substituted by hydroxyl,
R¹ and R² together form a straight-chain or branched alkylene chain having up to 5 carbon atoms which has to be substituted by a carbonyl group and/or a radical of the formula -OR¹⁹,
in which
R¹⁹ represents hydrogen, cyclopropyl, cyclopentyl or cyclohexyl,
or
R¹⁹ represents straight-chain or branched acyl having up to 15 carbon atoms or benzoyl which is optionally substituted by fluorine, chlorine, bromine, trifluoromethyl, nitro or trifluoromethoxy, or
represents a radical of the formula -Si(CH₃)₂C(CH₃)₃,
and the carbocycles formed are optionally substituted, if appropriate also geminally, up to 4 times by identical or different substituents from the group consisting of fluorine, hydroxyl, trifluoromethyl, carboxyl, azido, chlorine, bromine, nitro, cyano, cyclopropyl, cyclobutyl, cyclopentyl, cyclohexyl, cyclopropyloxy, cyclopentyloxy, cyclohexyloxy, straight-chain or branched alkoxycarbonyl, alkoxy or alkylthio having in each case up to 4 carbon atoms and straight-chain or branched alkyl having up to 4 carbon atoms which for its part is substituted up to 2 times by identical or different substituents from the group consisting of hydroxyl, benzyloxy, trifluoromethyl, benzoyl, methoxy, oxyacetyl and phenyl which for its part may be substituted by fluorine, chlorine, bromine, trifluoromethyl or trifluoromethoxy,
and/or the carbocycles formed are optionally substituted, also geminally, up to 4 times by identical or different substituents from the group consisting of phenyl, benzoyl, thiophenyl and sulphonylbenzyl which for their part are optionally substituted by fluorine, trifluoromethyl, trifluoromethoxy or nitro, and/or are optionally substituted by a radical of the formula 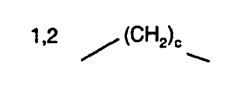 or =O, in which
c represents a number 1, 2, 3 or 4,
and/or the carbocycles formed are optionally substituted by a spire-linked radical of the formula 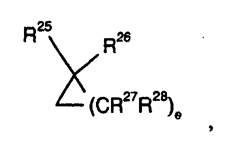 in which
e represents a number 1, 2, 3, 4 or 5,
R²⁵, R²⁶, R²⁷ and R²⁸ are identical or different and represent hydrogen, trifluoromethyl, phenyl, fluorine, chlorine, bromine or straight-chain or branched alkyl or alkoxy having in each case up to 4 carbon atoms,
or
R²⁵ and R²⁶ or R²⁷ and R²⁸ together form a straight-chain or branched alkyl chain having up to 4 carbon atoms,
and their salts and N-oxides.

4. Hetero-tetrahydroquinolines of the formula (I) according to Claim 1,
in which
A represents cyclopropyl, cyclopentyl or cyclohexyl, or represents thienyl or pyridyl, or A represents a radical of the formula 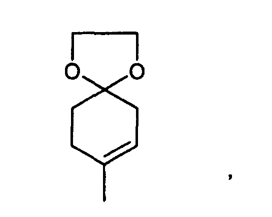
D represents phenyl which is optionally substituted by trifluoromethyl, fluorine, or
represents a radical of the formula 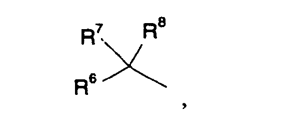 in which
R⁶ represents phenyl which is optionally substituted by fluorine, chlorine, trifluoromethyl, trifluoromethoxy, straight-chain or branched alkyl having in each case up to 4 carbon atoms,
or
R⁷ represents hydrogen or fluorine,
and
R⁸ represents hydrogen, fluorine, chlorine, hydroxyl, methoxy or
R⁷ and R⁸ together represent a radical of the formula =O,
E represents cyclopropyl, cyclopentyl or cyclohexyl,
represents straight-chain or branched alkyl having up to 4 carbon atoms,
R¹ and R² together form a straight-chain or branched alkylene chain having up to 5 carbon atoms which has to be substituted by a carbonyl group and/or a radical of the formula -OR¹⁹,
in which
R¹⁹ represents hydrogen
or represents a radical of the formula -Si(CH₃)₂C(CH₃)₃,
and their salts and N-oxides.

5. Hetero-tetrahydroquinolines according to Claim 1 to 4 as medicaments.

6. Process for preparing hetero-tetrahydroquinolines according to Claim 1, **characterized in that**
[A] in the case where D □ aryl, for compounds of the general formula (II) 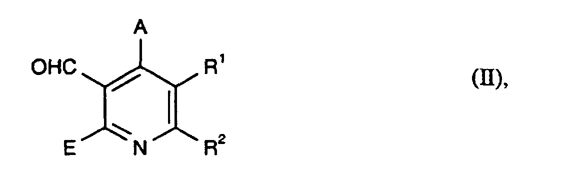 in which
A, E, R¹ and R² are as defined above,
the substituent D is synthesized with organometallic reagents in a Grignard or Wittig reaction or in a reaction with organolithium compounds, in inert solvents,
or in the case where D represents the radical of the formula R⁹-T-V-X in which V is an oxygen atom,
[B] either compounds of the general formula (III) 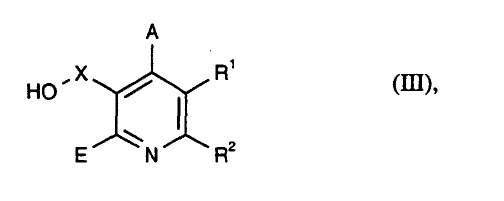 in which
A, E, X, R¹ and R² are as defined above,
are reacted with compounds of the general formula (IV)
R⁹-T- (IV),
in which
R⁹ and T are as defined above
and
Z represents halogen, preferably chlorine or bromine,
in inert solvents, if appropriate in the presence of a base and/or of an auxiliary,
or
[C] compounds of the general formula (III) are initially, by reaction with compounds of the general formula (V) 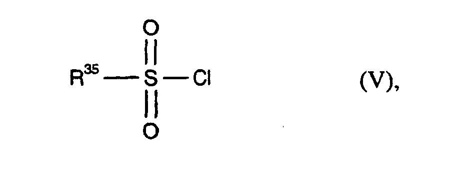 in which
R³⁵ represents straight-chain alkyl having up to 4 carbon atoms,
converted into the compounds of the general formula (VI) 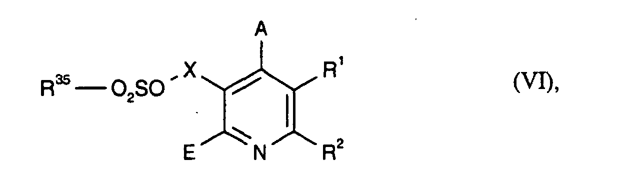 in which
A, E, X, R¹, R² and R³⁵ are as defined above,
and subsequently reacted with compounds of the general formula (VII)
R⁹-T-V-H (VII),
in which
R⁹, T and V are as defined above,
and, if appropriate, protective groups are removed,
or
[D] in the case of the compounds of the general formula (Ia) 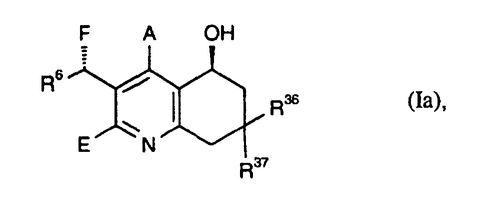 in which
A and R⁶ are as defined above,
R³⁶ and R³⁷ are identical or different and
represent cycloalkyl or cycloalkyloxy having in each case 3 to 7 carbon atoms, or
represent straight-chain or branched alkyl having up to 6 carbon atoms, or
represent phenyl which for its part is optionally substituted by halogen, trifluoromethyl, trifluoromethoxy or nitro, or
R³⁶ and R³⁷ represent one of the abovementioned spiro-linked radicals of the formula 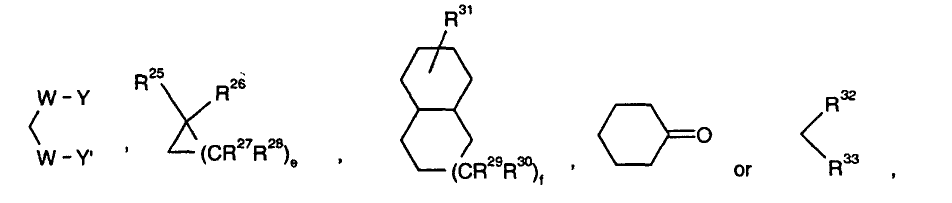
in which
W, Y, Y', R²⁵, R²⁶, R²⁷, R²⁸, e, R²⁹, R³⁰, R³¹, R³² and R³³ are as defined above,
compounds of the general formula (VIII) 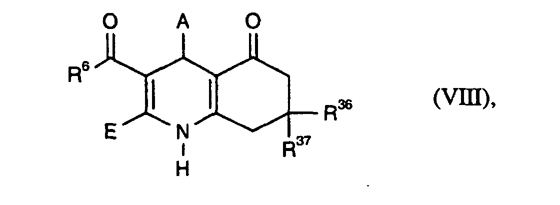 in which
R⁶, R³⁶, R³⁷, A and E are as defined above,
are initially oxidized to the compounds of the general formula (IX) 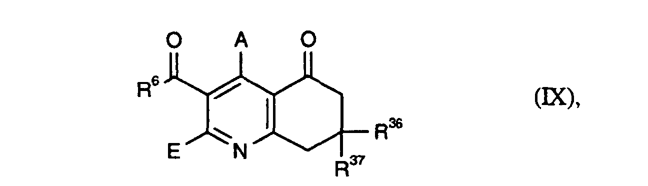 in which
R⁶, R³⁶, R³⁷, A and E are as defined above,
these are, in a subsequent step, converted by asymmetric reduction into the compounds of the general formula (X) 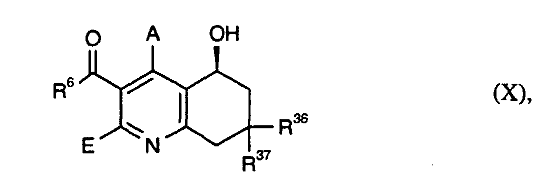 in which
R⁶, R³⁶, R³⁷, A and E are as defined above,
these are then converted, by the introduction of a hydroxyl protective group, into the compounds of the general formula (XI) 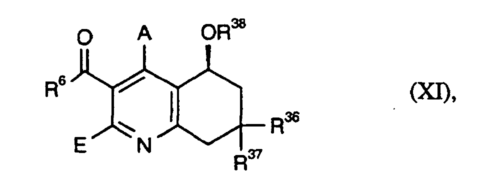 in which
R⁶, R³⁶, R³⁷, A and E are as defined above
and
R³⁸ represents a hydroxyl protective group, preferably a radical of the formula -SiR³⁹R⁴⁰R⁴¹,
in which
R³⁹, R⁴⁰ and R⁴¹ are identical or different and represent C₁-C₄-alkyl,
which is used to prepare in a subsequent step, by diastereoselective reduction, the compounds of the general formula (XII) 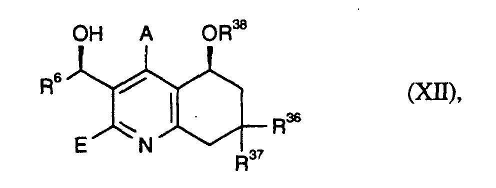 in which
R⁶, R³⁶, R³⁷, R³⁸, A and E are as defined above,
and the compounds of the general formula (XIII) 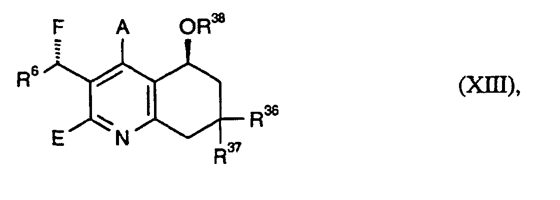 in which
R⁶, R³⁶, R³⁷, R³⁸, A and E are as defined above,
are subsequently prepared by introducing the fluorine substituent with fluorinating agents, such as, for example, DAST and SF₄ derivatives,
and the hydroxyl protective group is then removed by customary methods,
and, if appropriate, the substituents listed under D, E and/or R¹ and R² are varied or introduced by customary methods.

7. Medicament, comprising at least one hetero-tetrahydroquinoline according to Claim 1 and pharmacologically acceptable formulation auxiliaries.

8. Medicament according to Claim 7 for treating hyperlipoproteinanaenua.

9. Medicament according to Claim 7 for treating arteriosclerosis.

10. Use of hetero-tetrahydroquinolines according to Claim 1 for preparing medicaments.

11. Use according to Claim 10 for preparing medicaments for treating arteriosclerosis, in particular dyslipidaemias.

## Revendications

1. Hétérotétrahydroquinoléines de formule générale (I), 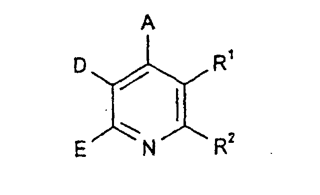 dans laquelle
A est un reste cycloalkyle ayant 3 à 8 atomes de carbone, ou
un hétérocycle pentagonal à heptagonal, saturé, partiellement ou entièrement insaturé, éventuellement condensé au benzène, ayant jusqu'à 3 hétéroatomes de la série S, N et/ou O qui, dans le cas d'un hétérocycle saturé portant une fonction azotée, est aussi le cas échéant lié par l'intermédiaire de cette fonction, et les systèmes cycliques indiqués ci-dessus sont éventuellement substitués jusqu'à 5 fois identiques ou différentes par un halogène, un groupe nitro, un groupe hydroxy, trifluorométhyle, trifluorométhoxy ou par un groupe alkyle, acyle, hydroxyalkyle ou alkoxy, linéaire ou ramifié ayant chacun jusqu'à 7 atomes de carbone, ou par un groupe de formule -NR³R⁴,
où
R³ et R⁴ sont identiques ou différents et représentent l'hydrogène, un reste phényle ou un reste alkyle lié ou ramifié ayant jusqu'à 6 atomes de carbone,
ou bien
A représente un reste de formule 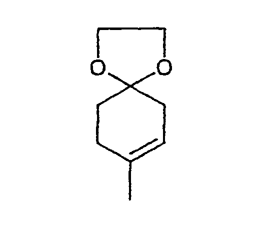
D est un reste aryle ayant 6 à 10 atomes de carbone, qui est éventuellement substitué par un radical phényle, nitro, halogéno, trifluorométhyle ou trifluorométhoxy, ou bien
un reste de formule
R⁵―L― 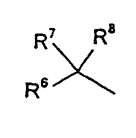
ou
R⁹-T-V―X―
dans laquelle
R⁵, R⁶ et R⁹ représentent indépendamment l'un de l'autre un groupe cycloalkyle ayant 3 à 6 atomes de carbone, ou bien
un groupe aryle ayant 6 à 10 atomes de carbone, ou un hétérocycle pentagonal à heptagonal monocyclique, bicyclique ou tricyclique saturé ou non saturé, éventuellement condensé au benzène, ayant jusqu'à 4 hétéroatomes de la série S, N et/ou O,
les cycles, le cas échéant également par l'intermédiaire de la fonction N dans le cas des noyaux contenant de l'azote, étant substitué jusqu'à 5 fois identiques ou différentes par un halogène, un groupe trifluorométhyle, nitro, hydroxy, cyano, carboxyle, trifluorométhoxy, un groupe acyle, alkyle, alkylthio, alkylalkoxy, alkoxy ou alkoxycarbonyle linéaire ou ramifié, ayant chacun jusqu'à 6 atomes de carbone, par un groupe aryle ou aryle à substituant trifluorométhyle ayant chacun jusqu'à 6 atomes de carbone ou par un hétérocycle aromatique pentagonal à heptagonal éventuellement condensé au benzène, ayant jusqu'à 3 hétéroatomes de la série S, N et/ou O,
et/ou substitués par un groupe de formule -OR¹⁰, -SR¹¹, -SO₂R¹², ou -NR¹³R¹⁴,
où
R¹⁰, R¹¹ et R¹² représentent indépendamment l'un de l'autre un reste aryle ayant 6 à 10 atomes de carbone, qui peut lui-même être substitué jusqu'à deux fois identiques ou différentes par un radical phényle, halogéno ou par un radical alkyle linéaire ou ramifié ayant jusqu'à 6 atomes de carbone,
R¹³ et R¹⁴ sont identiques ou différents et ont la définition indiquée ci-dessus pour R³ et R⁴,
ou bien
R⁵ et/ou R⁶ représentent un reste de formule 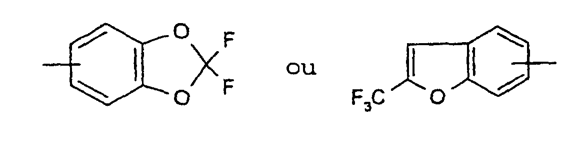
R⁷ est l'hydrogène ou un halogène
et
R⁸ représente un hydrogène, un halogène ou azido, trifluorométhyle, hydroxy, tri-fluorométhoxy, un groupe alkoxy ou alkyle linéaire ou ramifié ayant chacun jusqu'à 6 atomes de carbone ou un reste de formule -NR¹⁵R¹⁶,
dans laquelle
R¹⁵ et R¹⁶ sont identiques ou différents et ont la définition indiquée ci-dessus pour R³ et R⁴,
ou bien
R⁷ et R⁸ forment ensemble un reste de formule=O ou =NR¹⁷,
où
R¹⁷ représente l'hydrogène ou un groupe alkyle, alkoxy ou acyle linéaire ou ramifié ayant chacun jusqu'à 6 atomes de carbone,
L représente une chaîne alkylénique ou acénylénique linéaire ou ramifiée ayant dans chaque cas jusqu'à 8 atomes de carbone et portant éventuellement jusqu'à 2 substituants hydroxy,
T et X sont identiques ou différents et représentent une chaîne alkylénique linéaire ou ramifiée ayant jusqu'à 8 atomes de carbone,
ou bien
T ou X forment une liaison,
V est un atome d'oxygène ou de soufre ou un groupe-NR¹⁸-,
dans lequel
R¹⁸ est l'hydrogène ou un radical alkyle linéaire ou ramifié ayant jusqu'à 6 atomes de carbone ou un radical phényle,
E est un reste cycloalkyle ayant 3 à 8 atomes de carbone, ou bien un reste alkyle linéaire ou ramifié ayant jusqu'à 8 atomes de carbone, qui est éventuellement substitué par un radical cycloalkyle ayant 3 à 8 atomes de carbone ou un radical hydroxy, ou représente un reste phényle qui est éventuellement substitué par un halogène ou un radical trifluorométhyle,
R¹ et R² forment ensemble une chaîne alkylénique linéaire ou ramifiée ayant jusqu'à 7 atomes de carbone, qui doit être substituée par un groupe carbonyle et/ou par un reste de formule 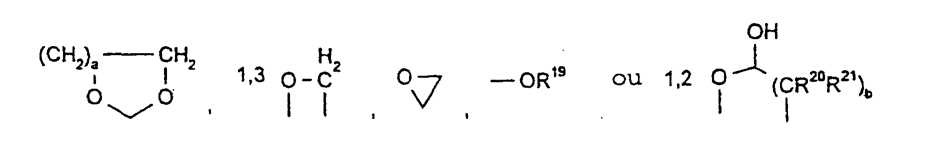 où
a et b sont égaux ou différents et représentent le nombre 1, 2 ou 3,
R¹⁹ représente l'hydrogène, un reste cycloalkyle ayant 3 à 7 atomes de carbone, un reste silylalkyle linéaire ou ramifié ayant jusqu'à 8 atomes de carbone ou un reste alkyle linéaire ou ramifié ayant jusqu'à 8 atomes de carbone, qui est éventuellement substitué par un radical hydroxy, alkoxy linéaire ou ramifié ayant jusqu'à 6 atomes de carbone ou par un radical phényle qui peut lui-même porter un substituant halogéno, nitro, trifluorométhyle, trifluorométhoxy, ou phényle à substituant phényle ou tétrazole,
et un reste alkyle éventuellement substitué par un groupe de formule -OR²²,
dans lequel
R²² est un radical acyle linéaire ou ramifié ayant jusqu'à 4 atomes de carbone ou un radical benzyle,
ou bien
R¹⁹ est un reste acyle linéaire ou ramifié ayant jusqu'à 20 atomes de carbone ou un reste benzoyle qui porte éventuellement un substituant halogéno, trifluorométhyle, nitro ou trifluorométhoxy, ou bien
un reste fluoracyle linéaire ou ramifié ayant jusqu'à 8 atomes de carbone et 9 atomes de fluor,
R²⁰ et R²¹ sont identiques ou différents et représentent l'hydrogène, un reste phényle ou un reste alkyle linéaire ou ramifié ayant jusqu'à.6 atomes de carbone,
ou bien
R²⁰ et R²¹ forment ensemble un cycle carbonide, de 3 à 6 chaînons,
et les cycles carbonés formés portent le cas échéant jusqu'à 6 substituants identiques ou différents, éventuellement géminés, trifluorométhyle, hydroxy, nitrile, halogéno, carboxyle, nitro, azido, cyano, cycloalkyle ou cycloalkyloxy ayant chacun 3 à 7 atomes de carbone, alkoxycarbonyle, alkoxy ou alkylthio linéaire ou ramifié ayant chacun jusqu'à 6 atomes de carbone ou alkyle linéaire ou ramifié ayant jusqu'à 6 atomes de carbone, qui est lui-même substitué jusqu'à 2 fois identiques ou différentes par un radical hydroxy, benzyloxy, trifluorométhyle, benzoyle, alkoxy, oxyacyle ou carboxyle linéaire ou ramifié ayant chacun jusqu'à 4 atomes de carbone, et/ou phényle qui peut lui-même être substitué par un radical halogéno trifluorométhyle ou trifluorométhoxy,
et/ou les cycles carbonés formés portent éventuellement jusqu'à 5 substituants identiques ou différents, également géminés, phényle, benzoyle, thiophényle ou sulfonyl-benzyle, qui sont eux-mêmes éventuellement substitués par un radical halogéno, trifluorométhyle, trifluorométhoxy ou nitro,
et sont éventuellement substitués par un reste de formule 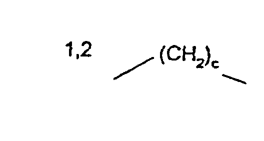 -SO₂-C₆H₅, -(CO)_{d}-NR²³R²⁴ ou =O dans laquelle
c représente le nombre 1, 2, 3 ou 4,
d représente le nombre 0 ou 1,
R²³ et R²⁴ sont identiques ou différents et représentent l'hydrogène, un reste cycloalkyle ayant 3 à 6 atomes de carbone, un reste alkyle linéaire ou ramifié ayant jusqu'à 6 atomes de carbone ou un reste phényle qui est éventuellement substitué jusqu'à 2 fois identiques ou différentes par un radical halogéno, trifluorométhyle, cyano, phényle ou nitro,
et/ou les cycles carbonés formés sont éventuellement substitués par un reste à liaison spiro de formule 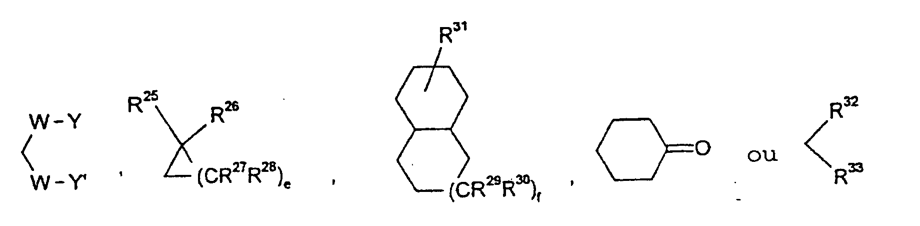
où
W représente un atome d'oxygène ou un atome de soufre,
Y et Y' forment ensemble une chaîne alkylénique linéaire ou ramifiée ayant 2 à 6 chaînons,
e représente le nombre 1, 2, 3, 4, 5, 6 ou 7,
f représente le nombre 1 ou 2,
R²⁵, R²⁶, R²⁷, R²⁸, R²⁹, R³⁰ et R³¹ sont identiques ou différents et représentent l'hydrogène, un reste trifluorométhyle, phényle, un radical halogéno ou un reste alkyle ou alkoxy linéaire ou ramifié ayant chacun jusqu'à 6 atomes de carbone,
ou bien
R²⁵ et R²⁶ ou R²⁷ et R²⁸ forment ensemble dans chaque cas une chaîne alkylique linéaire ou ramifiée ayant jusqu'à 6 atomes de carbone,
ou bien
R²⁵ et R²⁶ ou R²⁷ et R²⁸ forment ensemble dans chaque cas un reste de formule 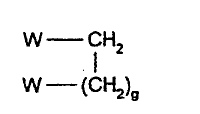 dans lequel
W a la définition indiquée ci-dessus,
g représente le nombre 1, 2, 3, 4, 5, 6 ou 7,
R³² et R³³ forment ensemble un hétérocycle de 3 à 7 chaînons qui contiennent un atome d'oxygène ou de soufre ou un groupe de formule SO, SO₂ ou -NR³⁴,
où
R³⁴ désigne l'hydrogène, un radical phényle, benzyle ou un radical alkyle linéaire ou ramifié ayant jusqu'à 4 atomes de carbone,
et leurs sels et leurs N-oxydes.

2. Hétérotétrahydroquinoléines de formule générale I), suivant la revendication 1,
formule dans laquelle
A est un reste cyclopropyle, cyclobutyle, cyclopentyle, cycloheptyle, cyclooctyle ou cyclohexyle, ou bien
un reste thiényle, imidazolyle, pyrrole, furyle, pyridyle, morpholine, pyrimidyle ou pyridazinyle, ses restes étant éventuellement substitués jusqu'à 2 fois identiques ou différentes par du fluor, du chlore, du brome, un radical amino, hydroxy, trifluorométhyle, trifluorométhoxy ou par un radical alkyle ou alkoxy linéaire ou ramifié ayant chacun jusqu'à 6 atomes de carbone,
ou bien
A est un reste de formule 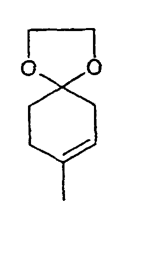
D est un reste phényle qui porte éventuellement un substituant nitro, fluoro, chloro, bromo, phényle, trifluorométhyle ou trifluorométhoxy, ou bien un reste de formule
R⁵―L― , 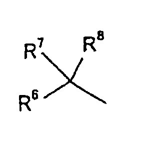
ou
R⁹-T-V―X―
dans laquelle
R⁵, R⁶ et R⁹ représentent indépendamment l'un de l'autre un radical cyclopropyle, cyclopentyle ou cyclohexyle, ou bien
un reste phényle, naphtyle, pyridyle, tétrazolyle, pyrimidyle, pyrazinyle, pyrrolidinyle, indolyle, morpholinyle, imidazolyle, benzothiazolyle, phénoxatiin-2-yle, benzoxazolyle, furyle, quinolyle ou purin-8-yle, les cycles étant substitués, le cas échéant également par l'intermédiaire de la fonction N dans le cas des noyaux contenant de l'azote, jusqu'à 3 fois identiques ou différentes par du fluor, du chlore, du brome, un radical trifluorométhyle, hydroxy, cyano, carboxyle, trifluorométhoxy, un radical acyle, alkyle, alkylthio, alkylalkoxy, alkoxy ou alkoxycarbonyle linéaire ou ramifié ayant chacun jusqu'à 4 atomes de carbone, phényle à substituant triazolyle, tétrazolyle, benzoxathiazolyle ou trifluorométhyle, ou phényle,
ou bien
R⁷ représente l'hydrogène, le fluor, le chlore ou le brome,
et
R⁸ représente l'hydrogène, le fluor, le chlore, le brome, un reste azido, trifluorométhyle, hydroxy, trifluorométhoxy, un reste alkoxy ou alkyle linéaire ou ramifié ayant chacun jusqu'à 5 atomes de carbone ou un reste de formule -NR¹⁵R¹⁶,
où
R¹⁵ et R¹⁶ sont identiques ou différents et représentent l'hydrogène, un radical phényle ou un radical alkyle linéaire ou ramifié ayant jusqu'à 4 atomes de carbone,
ou bien
R⁷ et R⁸ forment ensemble un reste de formule =O ou =NR¹⁷,
où
R¹⁷ représente l'hydrogène ou un radical alkyle, alkoxy ou acyle linéaire ou ramifié ayant chacun jusqu'à 4 atomes de carbone,
L est une chaîne alkylénique ou alcénylétique linéaire ou ramifiée ayant chacune jusqu'à 6 atomes de carbone et chacune étant éventuellement substituée jusqu'à 2 fois par un radical hydroxy,
T et X sont identiques ou différents et représentent une chaîne alkylénique linéaire ou ramifiée ayant jusqu'à 6 atomes de carbone,
ou bien
T ou X représentent une liaison,
V est un atome d'oxygène ou de soufre ou un groupe de formule -NR¹⁸-,
dans laquelle
R¹⁸ représente l'hydrogène ou un radical alkyle linéaire ou ramifié ayant jusqu'à 4 atomes de carbone ou un radical phényle,
E est un reste cyclopropyle, cyclobutyle, cyclopentyle, cyclohexyle ou cycloheptyle, ou bien un reste alkyle linéaire ou ramifié ayant jusqu'à 6 atomes de carbone, qui est éventuellement substitué par un radical cyclopropyle, cyclobutyle, cyclohexyle, cyclopentyle ou cycloheptyle ou un radical hydroxy, ou représente un reste phényle qui est éventuellement substitué par un radical fluoro, chloro ou trifluorométhyle,
R¹ et R² forment ensemble une chaîne alkylénique linéaire ou ramifiée ayant jusqu'à 6 atomes de carbone, qui doit être substituée par un groupe carboxyle et/ou par un reste de formule 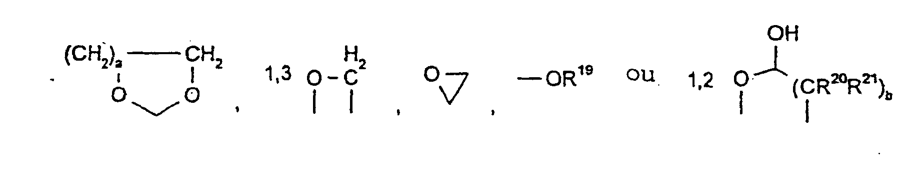 où
a et b sont égaux ou différents et représentent le nombre 1, 2 ou 3,
R¹⁹ représente l'hydrogène, un radical cyclopropyle, cyclopentyle, cyclohexyle, un radical silylakyle linéaire ou ramifié ayant jusqu'à 7 atomes de carbone ou un radical alkyle linéaire ou ramifié ayant jusqu'à 6 atomes de carbone, qui est éventuellement substitué par un radical hydroxy, alkoxy linéaire ou ramifié ayant jusqu'à 6 atomes de carbone ou par un radical phényle qui peut lui-même être substitué par du fluor, du chlore, du brome, un radical nitro, trifluorométhyle, trifluorométhoxy ou par un radical phényle ou phényle à substituant tétrazole, et le reste alkyle est éventuellement substitué par un groupe de formule -OR²²
dans laquelle
R²² désigne un radical acide linéaire ou ramifié ayant jusqu'à 3 atomes de carbone ou un radical benzyle,
ou bien
R¹⁹ est un reste acyle linéaire ou ramifié ayant jusqu'à 18 atomes de carbone ou un reste de benzoyle qui est éventuellement substitué par du fluor, du chlore, du brome, un radical trifluorométhyle, nitro ou trifluorométhoxy,
ou bien
un reste fluoracyle linéaire ou ramifié ayant jusqu'à 6 atomes de carbone,
R²⁰ et R²¹ sont identiques ou différents et représentent l'hydrogène, un reste phényle ou un reste alkyle linéaire ou ramifié ayant jusqu'à 4 atomes de carbone,
ou bien
R²⁰ et R²¹ forment ensemble un noyau cyclopropyle, cyclobutyle, cyclopentyle, cyclohexyle ou cycloheptyle,
et les cycles carbonés formés portent éventuellement jusqu'à 5 substituants identiques ou différents, éventuellement géminés, trifluorométhyle, hydroxy, carboxyle, azido, fluoro, bromo, nitro, cyano, cyclopropyle, cyclobutyle, cyclopentyle, cyclohexyle, cyclopropyloxy, cyclopentyloxy, cyclohexyloxy, alkoxycarbonyle, alkoxy ou alkylthio linéaires ou ramifiés ayant chacun jusqu'à environ 5 atomes de carbone, ou bien alkyle linéaire ou ramifié ayant jusqu'à 5 atomes de carbone, qui peut lui-même être substitué jusqu'à 2 fois identiques ou différentes par un radical hydroxyle, benzyloxy, benzoyle, un radical alkoxy ou oxyacyle linéaire ou ramifié ayant chacun jusqu'à 3 atomes de carbone, un radical trifluorométhyle et/ou un radical phényle qui peut lui-même porter un substituant fluoro, chloro, bromo, trifluorométhyle ou trifluorométhoxy,
et/ou
les cycles carbonés formés portant éventuellement jusqu'à 4 substituants identiques ou différents, également géminés, phényle, benzoyle, thiophényle ou sulfonylbenzyle qui sont eux-mêmes éventuellement substitués par du fluor, du chlore, du brome, un radical trifluorométhyle, trifluorométhoxy ou nitro,
et/ou sont éventuellement substitués par un reste de formule 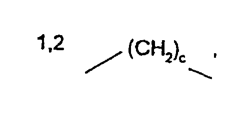 -SO₂-C₆H₅, -(CO)_{d}-NR²³R²⁴ ou =O dans laquelle
c représente le nombre 1, 2, 3 ou 4,
d représente le nombre 0 ou 1,
R²³ et R²⁴ sont identiques ou différents et représentent l'hydrogène, un reste cyclopropyle, cyclobutyle, cyclopentyle, cyclohexyle, un reste alkyle linéaire ou ramifié ayant jusqu'à 5 atomes de carbone, un reste benzyle ou un reste phényle qui est éventuellement substitué par du fluor, du chlore, du brome, un radical phényle ou trifluorométhyle,
et/ou les cycles carbones formés sont éventuellement substitués par un reste à liaison spiro de formule 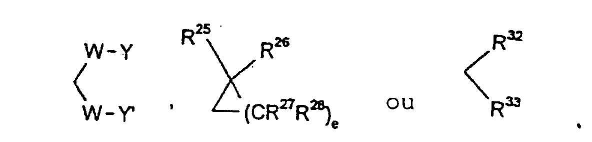 où
W représente un atome d'oxygène ou un atome de souffre,
Y et Y' forment ensemble une chaîne alkylique linéaire ou ramifiée de 2 à 5 chaînons,
e représente le nombre 1, 2, 3, 4, 5 ou 6,
f représente le nombre 1 ou 2,
R²⁵, R²⁶, R²⁷ et R²⁸ sont identiques ou différents et représentent l'hydrogène, un radical trifluorométhyle, phényle, fluoro, chloro, bromo ou un radical alkyle ou alkoxy lié ou ramifié ayant chacun jusqu'à 5 atomes de carbone,
ou bien
R²⁵ et R²⁶ ou R²⁷ et R²⁸ forment ensemble dans chaque cas une chaîne alkylique linéaire
ou ramifiée ayant jusqu'à 5 atomes de carbone,
ou bien
R²⁵ et R²⁶ ou R²⁷ et R²⁸ forment ensemble dans chaque cas un reste de formule 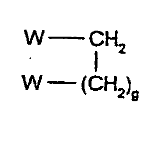 bilden,
dans laquelle
W a la définition indiquée ci-dessus,
g représente le nombre 1, 2, 3, 4, 5 ou 6,
et leurs sels et N-oxydes.

3. Hétérotétrahydroquinoléines de formule (I) suivant la revendication 1,
formule dans laquelle
A est un reste cyclopropyle, cyclopentyle ou cyclohexyle, ou bien
un reste thiényle ou pyridyle, ses restes étant éventuellement substitués jusqu'à 2 fois identiques ou différentes par du fluor, du chlore, du brome, un radical hydroxy, trifluorométhyle, trifluorométhoxy ou par un radical alkyle ou alkoxy linéaire ou ramifié ayant chacun jusqu'à 5 atomes de carbone,
ou bien
A représente un reste de formule 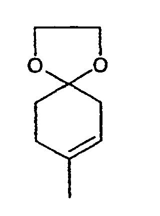
D est un reste phényle qui est éventuellement substitué par un groupe nitro, trifluorométhyle, phényle, du fluor, du chlore ou du brome, ou bien un reste de formule
R⁵―L― , 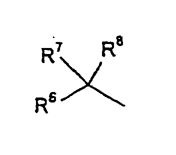
ou
R⁹-T-V―X―
où
R⁵, R⁶ et R⁹ représentent indépendamment l'un de l'autre un radical cyclopropyle, cyclopentyle ou cyclohexyle, ou bien
un radical phényle, naphtyle ou pyridyle,
les cycles étant substitués jusqu'à 2 fois identiques ou différentes par du fluor, du chlore, un groupe trifluorométhyle, hydroxy, cyano, carboxyle, trifluorométhoxy, un groupe alkyle, alkylthio, alkyloxy, alkoxy ou alkoxycarbonyle linéaire ou ramifié ayant chacun jusqu'à 4 atomes de carbone,
ou bien
R⁷ représente l'hydrogène ou le fluor
et
R⁸ représente l'hydrogène, le fluor, le chlore, le brome, un groupe azido, trifluorométhyle, hydroxy, trifluoro-méthoxy ou un groupe alkoxy ou alkyle linéaire ou ramifié ayant chacun jusqu'à 4 atomes de carbone, ou bien un reste de formule -NR¹⁵R¹⁶,
dans laquelle
R¹⁵ et R¹⁶ sont identiques ou différents et ils représentent l'hydrogène ou un radical alkyle linéaire ou ramifié ayant jusqu'à 3 atomes de carbone,
ou bien
R⁷ et R⁸ forment ensemble un reste de formule =O, L est une chaîne alkylénique ou alcénylénique linéaire ou ramifiée ayant dans chaque cas jusqu'à 5 atomes de carbone, ces chaînes étant éventuellement substituées jusqu'à 2 fois par un radical hydroxy,
T et X sont identiques ou différents et représentent une chaîne alkylénique linéaire
ou ramifiée ayant jusqu'à 3 atomes de carbone,
ou bien
T ou X représentent une liaison,
V est un atome d'oxygène ou de soufre ou un groupe de formule -NR¹⁸,
dans laquelle
R¹⁸ représente l'hydrogène ou un radical alkyle linéaire ou ramifié ayant jusqu'à 3 atomes de carbone,
E est un reste cyclopropyle, cyclopentyle ou cyclohexyle, ou un reste phényle qui est éventuellement substitué par du fluor ou un radical trifluorométhyle, ou bien ayant 3 à 8 atomes de carbone, ou bien
un reste alkyle linéaire ou ramifié ayant jusqu'à 4 atomes de carbone, qui est éventuellement substitué par un radical hydroxy,
R¹ et R² forment ensemble une chaîne alkylénique linéaire ou ramifiée ayant jusqu'à 5 atomes de carbone, qui doit être substituée par un groupe carbonyle et/ou par un reste de formule -OR¹⁹,
dans laquelle
R¹⁹ représente l'hydrogène, un radical cyclopropyle, cyclopentyle ou cyclohexyle,
ou bien
R¹⁹ est un radical acyle linéaire ou ramifié ayant jusqu'à 15 atomes de carbone, ou un radical benzoyle qui est éventuellement substitué par du fluor, du chlore, du brome, un radical trifluorométhyle, nitro ou trifluorométhoxy,
ou représente
un reste de formule -Si(CH₃)₂C(CH₃)₃,
et les cycles carbonés formés portent éventuellement jusqu'à 4 substituants identiques ou différents, le cas échéant géminés, fluoro, hydroxyle, trifluorométhyle, carboxyle, azido, chloro, bromo, nitro, cyano, cyclopropyle, cyclobutyle, cyclopentyle, cyclohexyle, cyclopropyloxy, cyclopentyloxy, cyclohexyloxy, alkoxycarbonyle, alkoxy ou alkylthio linéaires ou ramifiés ayant chacun jusqu'à 4 atomes de carbone, ou bien alkyle linéaire ou ramifié ayant jusqu'à 4 atomes de carbone, qui est lui-même substitué jusqu'à 2 fois identiques ou différentes par un radical hydroxyle, benzyloxy, -trifluorométhyle, benzoyle, méthoxy, oxyacétyle et/ou par un radical phényle qui peut lui-même être substitué par un radical fluoro, chloro, bromo, trifluorométhyle ou trifluorométhoxy, et/ou les cycles carbonés formés portent éventuellement jusqu'à 4 substituants identiques ou différents, également géminés, phényle, benzoyle, thiophényle ou sulfonyl-benzyle, qui sont eux-mêmes éventuellement substitués par un radical fluoro, trifluorométhyle, trifluorométhoxy ou nitro, et/ou substitués le cas échéant par un reste de formule 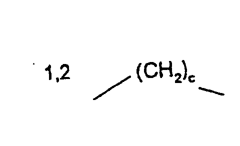 ou =O
dans laquelle
c représente le nombre de 1, 2, 3 ou 4,
et/ou les cycles carbonés formés sont éventuellement substitués par un reste à liaison spiro de formule 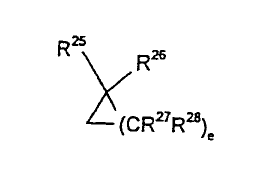
où
e représente le nombre 1, 2, 3, 4 ou 5,
R²⁵, R²⁶, R²⁷ et R²⁸ sont identiques ou différents et représentent l'hydrogène, un radical trifluorométhyle, phényle, fluoro, chloro, bromo, ou un radical alkyle ou alkoxy linéaire ou ramifié ayant chacun jusqu'à 4 atomes de carbone,
ou bien
R²⁵ et R²⁶ ou R²⁷ et R²⁸ forment ensemble une chaîne alkylique linéaire ou ramifiée ayant jusqu'à 4 atomes de carbone,
et leurs sels et N-oxydes.

4. Hétérotétrahydroquinoléines de formule (I) suivant la revendication 1,
formule dans laquelle
A est un reste cyclopropyle, cyclopentyle ou cyclohexyle, ou bien
un reste thiényle ou pyridyle, ou bien A représente un reste de formule 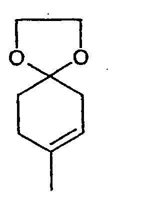
D est un reste phényle qui est éventuellement substitué par un radical trifluorométhyle, fluoro, ou représente
un reste de formule 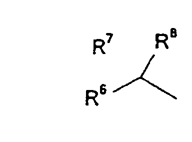 dans laquelle
R⁶ est un radical phényle qui est éventuellement substitué par du fluor, du chlore, un groupe trifluorométhyle, trifluorométhoxy, un groupe alkyle linéaire ou ramifié, ayant jusqu'à 4 atomes de carbone de carbone,
ou bien
R⁷ représente l'hydrogène ou le fluor,
et
R⁸ représente l'hydrogène, le fluor, le chlore, un radical hydroxy, méthoxy, ou bien
R⁷ et R⁸ forment ensemble un reste de formule=O,
E est un reste cyclopropyle, cyclopentyle ou cyclohexyle,
un reste alkyle linéaire ou ramifié ayant jusqu'à 4 atomes de carbone,
R¹ et R² forment ensemble une chaîne alkylénique linéaire ou ramifiée ayant jusqu'à 5 atomes de carbone, qui doit être substituée par un groupe carbonyle et/ou un reste de formule -OR¹⁹
dans laquelle
R¹⁹ représente l'hydrogène,
ou bien représente un reste de formule -Si(CH₃)₂C(CH₃)₃,
et leurs sels et N-oxydes.

5. Hétérotétrahydroquinoléines suivant les revendications 1 à 4, en tant que médicaments.

6. Procédé de production d'hétérotétrahydroquinoléines suivant la revendication 1, **caractérisé en ce que**
[A] au cas où D est ≠ d'un groupe aryle, composés de formule générale (II) 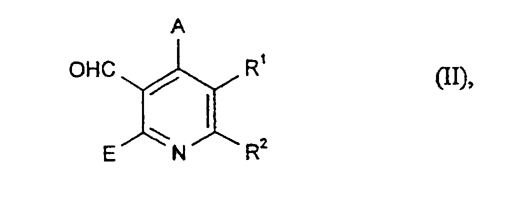 dans laquelle
A, E, R¹ et R² ont la définition indiquée ci-dessus,
on synthétise le substituant D avec des réactifs organométalliques au sens d'une réaction de Grignard, d'une réaction de Wittig ou d'une réaction avec un composé organique de lithium, dans des solvants inertes,
ou bien au cas où D représente le reste de formule R⁹-T-V-X, dans laquelle V est un atome d'oxygène,
[B] on fait réagir des composés de formule générale (III) 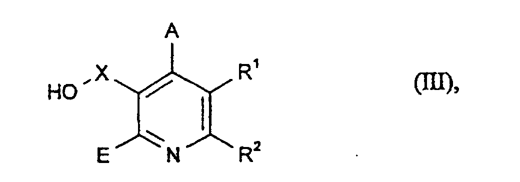 dans laquelle
A, E, X, R¹ et R² ont la définition indiquée ci-dessus,
avec des composés de formule générale (IV)
R⁹-T-Z (IV),
dans laquelle
R⁹ et T ont la définition indiquée ci-dessus
et
Z représente un halogène, de préférence le chlore ou le brome,
dans des solvants inertes, éventuellement en présence d'une base et/ou d'une substance auxiliaire,
ou bien
[C] on transforme tout d'abord des composés de formule générale (III) par réaction avec des composés de formule générale (V) 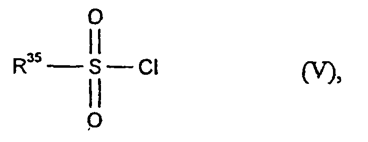 dans laquelle
R³⁵ est un radical alkyle linéaire ayant jusqu'à 4 atomes de carbone,
en composés de formule générale (VI) 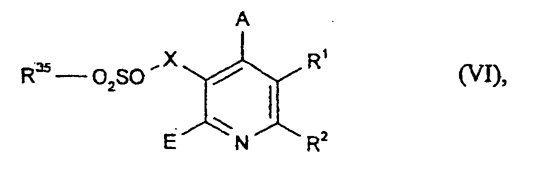 dans laquelle
A, E, X, R¹, R² et R³⁵ ont la définition indiquée ci-dessus
qu'on fait ensuite réagir avec des composés de formule générale (VII)
R⁹-T-V-H (VIII)
dans laquelle
R⁹, T et V ont la définition indiquée ci-dessus
et on élimine éventuellement des groupes protecteurs,
ou bien
[D] Dans le cas des composés de formule générale (Ia) dans laquelle 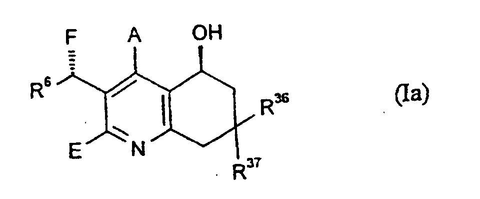 dans laquelle
A et R⁶ ont la définition indiquée ci-dessus,
R³⁶ et R³⁷ sont identiques ou différents et représentent et
un reste cycloalkyle ou cycloalkyloxy ayant dans chaque cas 3 à 7 atomes de carbone, ou représentent
un reste alkyle linéaire ou ramifié ayant jusqu'à 6 atomes de carbone, ou représentent
un reste phényle qui est lui-même éventuellement substitué par un halogène, un radical trifluorométhyle, trifluorométhoxy ou nitro, ou bien
R³⁶ et R³⁷ représentent l'un des restes à liaison spiro indiqués ci-dessus de formule 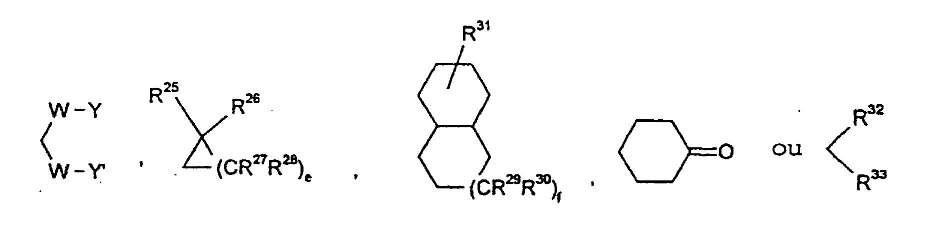 où
W, Y, Y', R²⁵, R²⁶, R²⁷, R²⁸, e, R²⁹, R³⁰, R³¹, R³² et R³³ ont la définition indiquée ci-dessus,
on oxyde tout d'abord des composés de formule générale (VIII) 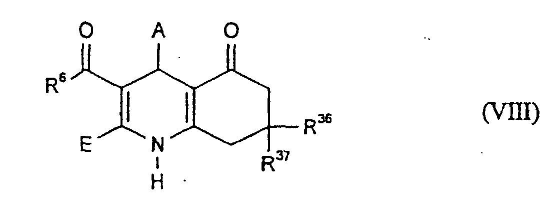 dans laquelle
R⁶, R³⁶, R³⁷, A et E ont la définition indiquée ci-dessus,
tout d'abord en composés de formule générale (IX) 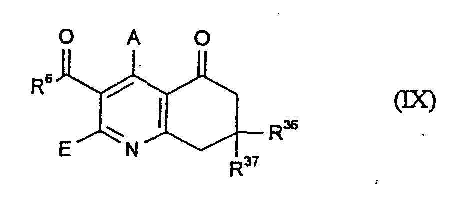 dans laquelle
R⁶, R³⁶, R³⁷, A et E ont la définition indiquée ci-dessus,
on transforme ces composés dans une étape subséquente par une réduction asymétrique en composés de formule générale (X) 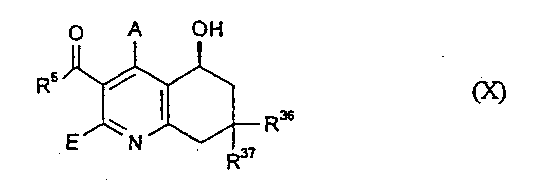
dans laquelle
R⁶, R³⁶, R³⁷, A et E ont la définition indiquée ci-dessus,
on transforme ensuite ces composés par l'introduction d'un groupe ayant la fonction hydroxy en composés de formule générale (XI) 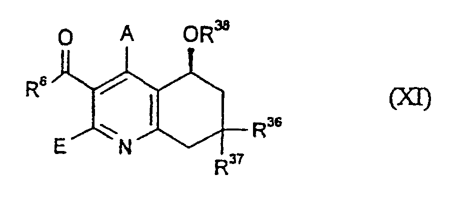
dans laquelle R⁶, R³⁶, R³⁷, A et E ont la définition indiquée ci-dessus,
et
R³⁸ représente un groupe protégeant la fonction hydroxy, de préférence un reste de formule -SiR³⁹R⁴⁰R⁴¹,
dans laquelle
R³⁹, R⁴⁰ et R⁴¹ sont identiques ou différents et représentent des groupes alkyles en C₁-C₄,
on prépare à partir de ces composés dans une étape subséquente, par réduction diastéréosélective, les composés de formule générale (XII) 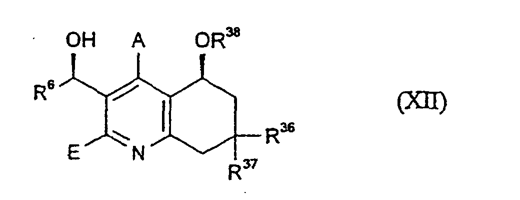 dans laquelle
R⁶, R³⁶, R³⁷, R³⁸, A et E ont la définition indiquée ci-dessus,
puis,
on prépare par introduction du substituant fluoro avec des réactifs de fluoration, tels que, par exemple, DAST et des dérivés de SF₄, les composés de formule générale (XIII) 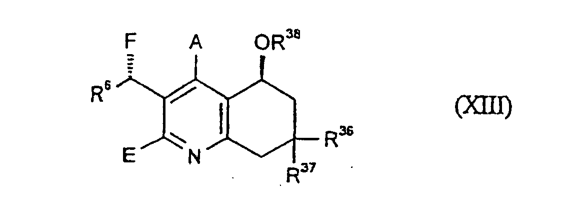 dans laquelle
R⁶, R³⁶, R³⁷, R³⁸, A et E ont la définition indiquée ci-dessus,
et
on élimine ensuite par des moyens classiques le groupe protégeant la fonction hydroxy, et le cas échéant on fait varier ou on introduit par des moyens classiques les substituants indiqués pour D, E et/ou R¹ et R².

7. Médicament contenant au moins une hétérotétraquinoléine suivant la revendication 1, ainsi que des auxiliaires de formulation acceptables du point de vue pharmacologique.

8. Médicament suivant la revendication 7, destiné au traitement de l'hyperlipoprotéinémie.

9. Médicament suivant la revendication 7, destiné au traitement de l'artériosclérose.

10. Utilisation d'hétérotétraquinoléines, suivant la revendication 1, pour la préparation de médicaments.

11. Utilisation suivant la revendication 10, pour la préparation de médicaments destinés au traitement de l'artériosclérose, notamment de dyslipidémies.
